(19) Europäisches Patentamt
European Patent Office
Office européen des brevets

(11) Veröffentlichungsnummer: **0 281 522 B1**

(12) # EUROPÄISCHE PATENTSCHRIFT

(45) Veröffentlichungstag der Patentschrift: **03.02.93**

(51) Int. Cl.[5]: **C07D 493/22**, A01N 43/90, A61K 31/365, //(C07D493/22, 313:00,311:00,311:00,307:00)

(21) Anmeldenummer: **88810126.8**

(22) Anmeldetag: **29.02.88**

Die Akte enthält technische Angaben, die nach dem Eingang der Anmeldung eingereicht wurden und die nicht in dieser Patentschrift enthalten sind.

(54) **Insektizide und Parasitizide.**

(30) Priorität: **06.03.87 CH 847/87**

(43) Veröffentlichungstag der Anmeldung:
**07.09.88 Patentblatt 88/36**

(45) Bekanntmachung des Hinweises auf die Patenterteilung:
**03.02.93 Patentblatt 93/05**

(84) Benannte Vertragsstaaten:
**AT BE CH DE ES FR GB GR IT LI LU NL SE**

(56) Entgegenhaltungen:
**EP-A- 0 147 852**
**EP-A- 0 189 159**
**DE-A- 3 532 794**
**GB-A- 2 187 453**

(73) Patentinhaber: **CIBA-GEIGY AG**
**Klybeckstrasse 141**
**CH-4002 Basel(CH)**

(72) Erfinder: **Gehret, Jean-Claude, Dr.**
**Im Aeschfeld 12**
**CH-4147 Aesch(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft neue C(29)-Oximino-milbemycin-Derivate der Formel I, ihre Herstellung und ihre Verwendung in einem Verfahren zur Bekämpfung von Schädlingen, ferner Schädlingsbekämpfungsmittel, die mindestens eine dieser Verbindungen als Wirkstoff enthalten.

Die neuen Verbindungen haben die allgemeine Formel I

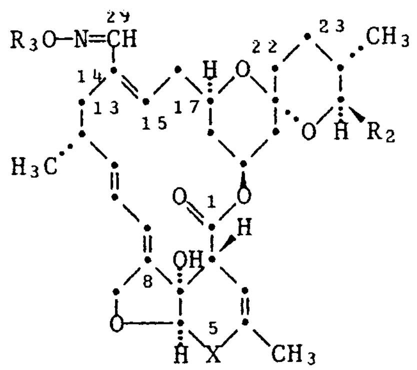

(I)

in welcher

X eine der Gruppen -CH($OR_1$)-, -C(O)- oder -C(=N-OR)- repräsentiert;

$R_1$ Wasserstoff oder eine OH-Schutzgruppe bedeutet;

R für Wasserstoff, eine OH-Schutzgruppe, eine Alkyl-, Cycloalkylgruppe steht; wobei unter OH-Schutzgruppe für die Substituenten R und $R_1$ die Reste $R_4$-C(O)- oder-Si($R_5$)($R_6$)($R_7$) zu verstehen sind, wobei $R_4$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxi, $C_1$-$C_3$-Haloalkoxi, Cyano und/oder Nitro substituierte Gruppe aus der Reihe Phenyl und Benzyl steht; $R_5$, $R_6$, $R_7$ unabhängig voneinander für $C_1$-$C_4$-Alkyl, Benzyl oder Phenyl stehen;

$R_2$ für Methyl, Ethyl, Isopropyl, sek.Butyl oder für die Gruppe

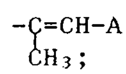

steht, worin A Methyl, Ethyl oder Isopropyl bedeutet, und

$R_3$ für Wasserstoff, $C_1$-$C_6$-Alkyl, $C_2$-$C_6$-Alkenyl, einen unsubstituierten oder einen ein- oder mehrfach durch Halogen, $C_1$-$C_3$-Alkyl, $C_1$-$C_3$-Alkoxy, $C_1$-$C_3$-Alkylthio, $C_1$-$C_3$-Haloalkyl, Nitro oder Cyano substituierten Rest aus der Reihe Phenyl und Benzyl steht oder die Gruppe U

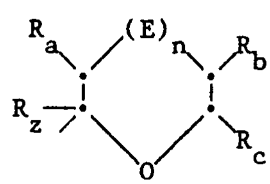

(U)

repräsentiert, worin n für eine der Zahlen 0, 1, 2, 3, 4 oder 5 steht, E für Sauerstoff oder -CH($R_d$)- und $R_z$ für Wasserstoff steht, $R_a$ Wasserstoff, Halogen, $C_1$-$C_3$-Alkyl, $C_1$-$C_3$-Alkoxy oder 2-Tetrahydropyranyl bedeutet und $R_b$, $R_c$ und $R_d$ unabhängig voneinander für Wasserstoff, Halogen, $C_1$-$C_3$-Alkyl oder $C_1$-$C_3$-Alkoxy stehen, wobei die Gruppe U auch in ungesättigter Form vorliegen kann.

Besonders bevorzugt sind die neuen Verbindungen der Formel I

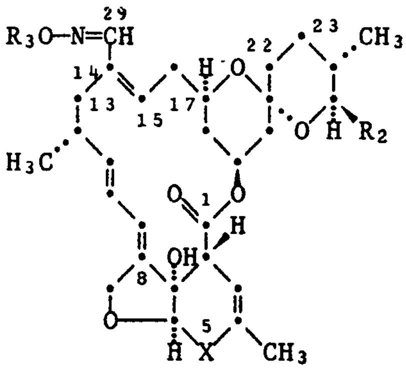

(I)

in welcher

X eine der Gruppen $-CH(OR_1)-$, $-C(O)-$ oder $-C(=N-OR)-$ repräsentiert;

$R_1$ Wasserstoff oder eine OH-Schutzgruppe bedeutet;

R für Wasserstoff, eine OH-Schutzgruppe, eine Alkyl-, Cycloalkyloder Acylgruppe steht;

$R_2$ für Methyl, Ethyl, Isopropyl, sek.Butyl steht; und

$R_3$ für Wasserstoff, $C_1$-$C_6$-Alkyl, $C_2$-$C_6$-Alkenyl, einen unsubstituierten oder einen ein- oder mehrfach durch Halogen, $C_1$-$C_3$-Alkyl, $C_1$-$C_3$-Alkoxy, $C_1$-$C_3$-Alkylthio, $C_1$-$C_3$-Haloalkyl, Nitro oder Cyano substituierten Rest aus der Reihe Phenyl und Benzyl steht oder die Gruppe U

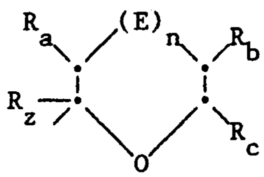

(U)

repräsentiert, worin n für eine der Zahlen 0, 1, 2, 3, 4 oder 5 steht, E für Sauerstoff oder $-CH(R_d)-$ und $R_z$ für Wasserstoff steht, $R_a$ Wasserstoff, Halogen, $C_1$-$C_3$-Alkyl, $C_1$-$C_3$-Alkoxy oder 2-Tetrahydropyranyl bedeutet und $R_b$, $R_c$ und $R_d$ unabhängig voneinander für Wasserstoff, Halogen, $C_1$-$C_3$-Alkyl oder $C_1$-$C_3$-Alkoxy stehen, wobei die Gruppe U auch in ungesättigter Form vorliegen kann.

Ganz besonders bevorzugt sind die neuen Verbindungen der allgemeinen Formel I

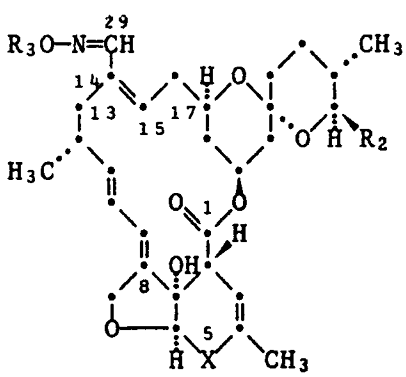

(I)

in welcher

X die Gruppe $-CH(OR_1)-$ repräsentiert;

$R_1$ Wasserstoff oder eine OH-Schutzgruppe bedeutet;

$R_2$ für Methyl, Ethyl, Isopropyl oder sek.Butyl steht; und

$R_3$ für Wasserstoff, $C_1$-$C_6$-Alkyl, $C_2$-$C_6$-Alkenyl, einen unsubstituierten oder einen ein- oder mehrfach durch Halogen, $C_1$-$C_3$-Alkyl, $C_1$-$C_3$-Alkoxy, $C_1$-$C_3$-Alkylthio, $C_1$-$C_3$-Haloalkyl, Nitro oder Cyano substituierten Rest aus der Reihe Phenyl und Benzyl steht oder die Gruppe U

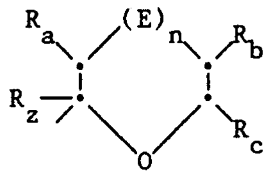

(U)

repräsentiert, worin n für eine der Zahlen 0, 1, 2, 3, 4 oder 5 steht, E für Sauerstoff oder -CH($R_d$)- und $R_z$ für Wasserstoff steht, $R_a$ Wasserstoff, Halogen, $C_1$-$C_3$-Alkyl, $C_1$-$C_3$-Alkoxy oder 2-Tetrahydropyranyl bedeutet und $R_b$, $R_c$ und $R_d$ unabhängig voneinander für Wasserstoff, Halogen, $C_1$-$C_3$-Alkyl oder $C_1$-$C_3$-Alkoxy stehen, wobei die Gruppe U auch in ungesättigter Form vorliegen kann.

Bevorzugt sind Verbindungen worin n für 0 oder 1,
E für Sauerstoff, -CH($R_x$)- oder -CH($R_x$)-CH($R_y$)-,
$R_z$ für Wasserstoff,
$R_a$, $R_b$, $R_c$, $R_x$ und $R_y$ unabhängig voneinander für Wasserstoff, Halogen, $C_1$-$C_3$-Alkyl oder $C_1$-$C_3$-Alkoxy stehen und $R_a$ auch Tetrahydropyranyl bedeuten kann oder die Substituentenpaare $R_z/R_a$, $R_a/R_x$, $R_x/R_y$, $R_y/R_b$ und $R_b/R_c$ unabhängig voneinander sowie, wenn n für 0 steht, auch $R_a/R_b$ und, wenn E für -CH($R_x$)- und n für I steht, auch $R_x/R_b$ jeweils für eine Bindung zwischen den beiden benachbarten Kohlenstoffatomen, an welche sie gebunden sind, stehen können, wobei jeder der Substituenten $R_a$, $R_x$, $R_y$, $R_b$ und $R_c$ in einem einzelnen Vertreter der Gruppe U nur einem der Substituentenpaare angehören kann.

Besonders bevorzugt sind Verbindungen worin
n für 0 oder 1
E für Sauerstoff, -CH($R_x$)- oder -CH($R_x$)-CH($R_y$)- und
$R_a$, $R_b$, $R_c$, $R_x$, $R_y$ und $R_z$ für Wasserstoff stehen und $R_a$ darüberhinaus auch Chlor oder Tetrahydrofuranyl und $R_c$ Brom oder die Gruppe -$OCH_3$ bedeuten kann oder aber die Substituentenpaare $R_z/R_a$ und $R_b/R_c$ unabhängig voneinander für eine Bindung zwischen den beiden benachbarten Kohlenstoffatomen an welche sie gebunden ist, stehen können.

Ganz besonders bevorzugt sind Verbindungen, in denen entweder

i) n für o,
$R_a$, $R_b$ und $R_z$ für Wasserstoff,
$R_c$ für Brom oder aber die Substituentenpaare $R_z/R_a$ und $R_b/R_c$ unabhängig voneinander für eine Bindung zwischen den beiden benachbarten Kohlenstoffatomen, an welche sie gebunden sind, stehen.

ii) n für 1,
E für Sauerstoff und $R_a$, $R_b$, $R_c$ und $R_z$ für Wasserstoff stehen,

iii) n für 1,
E für -CH($R_x$)-,
$R_a$, $R_b$, $R_c$, $R_y$ und $R_z$ für Wasserstoff stehen und $R_a$ darüberhinaus auch Chlor oder Tetrahydrofuranyl und
$R_c$ die Gruppe -$OCH_3$ bedeuten kann

iv) n für 1
E für -CH($R_x$)-CH($R_y$) steht und
$R_a$, $R_b$, $R_c$, $R_x$, $R_y$ und $R_z$ Wasserstoff bedeuten.

Typische Vertreter der Gruppe U sind:

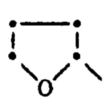 , 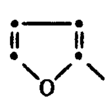 , 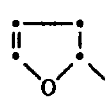 , 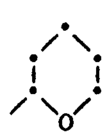 , 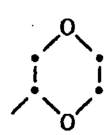 ,

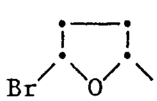 , 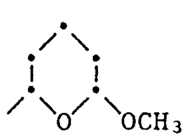 , 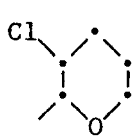 , 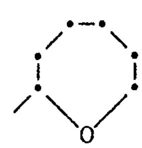 oder 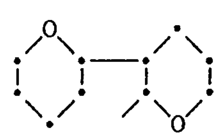

wobei diese Auflistung keinen limitierenden Charakter hat;
Verbindungen der Formel I, worin X die Gruppe -CH($OR_1$)- oder -C(=N-OR)- repräsentiert und $R_1$ und R eine Schutzgruppe darstellen, lassen sich durch einfache, z.B. hydrolytische Abspaltung der Schutzfunktion in die hochaktiven freien 5-Hydroxi-Derivate ($R_1$; R=H) überführen und haben somit auch Zwischenprodukte-Charakter. Im übrigen wird der biologische Wert dieser Verbindungen durch die Schutzgruppe oder den Zuckerrest nicht gemindert.

Als Substituenten der Phenyl-Gruppen sind 1 bis 3 Halogenatome, $C_1$-$C_2$-Alkyl, $C_1$-$C_2$-Alkoxi, $C_1$-$C_2$-Alkylthio, $C_1$-$C_2$-Haloalkyl oder Nitro und Cyano bevorzugt, unter allen Resten, die eine Alkylgruppe enthalten, insbesondere solche mit 1 C-Atom.Diese Substituenten können unabhängig voneinander in untereinander gemischter Anordnung auftreten.

Unter dem Begriff Alkyl selbst oder als Bestandteil eines anderen Substituenten sind je nach Zahl der angegebenen Kohlenstoffatome beispielsweise folgende Gruppen zu verstehen: Methyl, Ethyl, Propyl, Butyl, Pentyl, Hexyl, Heptyl, Octyl, Nonyl, Decyl, usw. sowie die Isomeren, wie z.B. Isopropyl, Isobutyl, tert.-Butyl, Isopentyl usw.. Haloalkyl steht für einen einfach bis perhalogenierten Alkylsubstituenten, wie z.B. $CHCl_2$, $CHF_2$, $CH_2Cl$, $CCl_3$, $CF_3$, $CH_2F$, $CH_2CH_2Cl$, $CHBr_2$ usw., vorzugsweise für $CF_3$. Unter Halogen soll hier und im folgenden Fluor, Chlor, Brom oder Jod, vorzugsweise Fluor, Chlor oder Brom, verstanden werden. Alkenyl steht für einen mindestens durch eine C=C-Doppelbindung charakterisierten aliphatischen Kohlenwasserstoffrest, wie z.B. für Vinyl, Propenyl-(1), Allyl, Butenyl-(1), Butenyl-(2), Butenyl-(3), usw.

Als Cycloalkyl-Gruppen kommen mono- bis tetracyclische Gruppen in Betracht, wie beispielsweise Cyclopropyl, Cyclobutyl, Cyclopentyl, Cyclohexyl, Decalin, Hydrindan, Bicycloheptan, Bicyclooctan, Norbornan, Bornan oder Adamantyl. Diese cycloaliphatischen Gruppen sind vorzugsweise unsubstituiert oder durch Methyl ein- oder mehrfach substituiert.

Die vorgenannten Acyl- und Silylgruppen dienen nicht nur als Schutzgruppen für im Substituenten X vorliegende Hydroxygruppen, sondern auch für alle anderen in den erfindungsgemässen Verbindungen oder Vorstufen dieser Verbindungen vorliegenden Hydroxygruppen.

Verbindungen der Formel I, worin $R_1$ Wasserstoff bedeutet, sind bevorzugt. Acyl- und Silylgruppen als R und $R_1$ sind im allgemeinen als Schutzgruppen aufzufassen.

Aufgrund ihrer ausgezeichneten Wirkung gegen Ektoparasiten am Nutztier bilden die 5-Oxime [X = -C(=N-OR)-] eine wichtige Untergruppe im Umfang der Formel I.

Verbindungen, worin $R_2$ sek.Butyl darstellt, sollen hier und im folgenden gleichfalls zu den Milbemycin-Derivaten gerechnet werden, obwohl sie nach der üblichen Systematik von Avermectin-Derivaten abgeleitet sind. Avermectin-Aglykone (mit einer OH-Gruppe in 13α-Position) lassen sich jedoch gemäss US-PS 4,173,571 in Milbemycin-Homologe überführen.

In natürlich vorkommenden Milbemycinen ($R_1$=H; $R_2$=$CH_3$, $C_2H_5$ oder iso-$C_3H_7$ ist die 13-Position stets lediglich mit Wasserstoff besetzt. Bei Avermectinen dagegen steht in der 13-Position ein α-L-Oleandrosyl-α-L-oleandrose-Rest, der über Sauerstoff in α-Konfiguration mit dem Makrolid-Molekül verknüpft ist. Avermectine unterscheiden sich strukturell ausserdem durch eine 23-OH-Gruppe oder $\Delta^{22,23}$-Doppelbindung und in der Regel durch einen Substituenten $R_2$ = sek.$C_4H_9$ von den Milbemycinen. Durch Hydrolyse des Zucker-Restes der Avermectine gelangt man leicht zu den entsprechenden Avermectin-Aglykonen, die eine allylische 13α-Hydroxi-Gruppe besitzen. Die Avermectin-Aglykone sind wie vorstehend angegeben, in die Milbemycin-Homologen umwandelbar. Bei den Milbemycin-Derivaten der vorliegenden Anmeldung liegt die $\Delta^{22,23}$-Doppelbindung stets in hydrierter Form vor.

Verbindungen der Formel I, worin $R_2$ für die Gruppe

$$-\underset{CH_3}{\underset{|}{C}}=CH-A$$

steht und A Methyl, Ethyl oder Isopropyl bedeutet, repräsentieren solche 23-Deoxy-Abkömmlinge der natürlich vorkommenden Antibiotika S541, die eine C(29) Oximino-Gruppe enthalten und in 5-Position entweder eine freie OH-Gruppe, eine OH-Schutzgruppe, eine Ketogruppe oder eine Oxim-Gruppierung enthalten.

Die Konstitution von natürlichen Antibiotika S541 ist aus der DE 35 32 794 bekannt und sieht wie folgt aus:

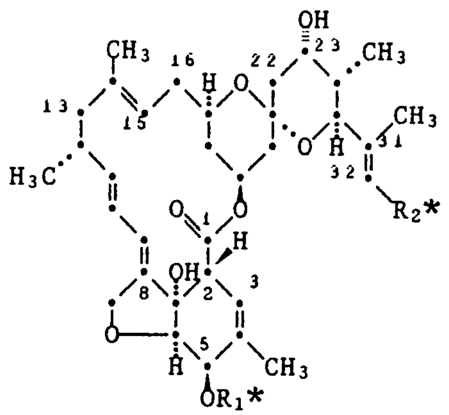

(Antibiotika S541) (S)

| | | |
|---|---|---|
| Faktor A | $R_2{*}=isoC_3H_7$ | $R_1{*}=H$ |
| Faktor B | $R_2{*}=CH_3$ | $R_1{*}=CH_3$ |
| Faktor C | $R_2{*}=CH_3$ | $R_1{*}=H$ |
| Faktor D | $R_2{*}=C_2H_5$ | $R_1{*}=H$ |
| Faktor E | $R_2{*}=C_2H_5$ | $R_1{*}=CH_3$ |
| Faktor F | $R_2{*}=isoC_3H_7$ | $R_1{*}=CH_3$ |

Folgende Untergruppen von Verbindungen der Formel I gemäss vorliegender Erfindung sind auf Grund ihrer ausgeprägten parasitiziden und insektiziden Wirkung besonders bevorzugt:

Gruppe Ia:

Verbindungen der Formel I, worin X eine der Gruppen -CH($OR_1$)-, -C(O)- oder -C(=N-OH)- repräsentiert, wobei $R_1$ Wasserstoff, $R_4$-C(O)- oder -Si($R_5$)($R_6$)($R_7$) bedeutet, wobei $R_4$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxi, $C_1$-$C_3$-Haloalkoxi, Cyano und/oder Nitro substituierte Gruppe aus der Reihe Phenyl und Benzyl steht; $R_5$, $R_6$, $R_7$ unabhängig voneinander für $C_1$-$C_4$-Alkyl, Benzyl oder Phenyl stehen; $R_2$ für Methyl, Ethyl, Isopropyl, sek.-Butyl oder für die Gruppe

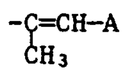

steht, worin A Methyl, Ethyl oder Isopropyl bedeutet; und $R_3$ für Wasserstoff, $C_1$-$C_4$-Alkyl, einen unsubstituierten oder einen ein- bis dreifach durch Halogen, $C_1$-$C_2$-Alkyl, $C_1$-$C_2$-Alkoxy, $C_1$-$C_2$-Alkylthio, $C_1$-$C_2$-Haloalkyl, Nitro oder Cyano substituierten Rest aus der Reihe Phenyl und Benzyl steht oder die Gruppe U

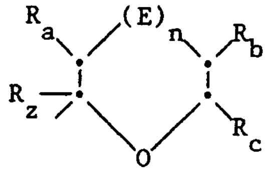

(U)

repräsentiert, worin n für eine der Zahlen 0 oder 1 steht, E für Sauerstoff oder $CH_2$ und $R_z$ für Wasserstoff steht, $R_a$ Wasserstoff, Fluor, Chlor, Brom, Methyl oder 2-Tetrahydropyranyl bedeutet und $R_b$ und $R_c$ unabhängig voneinander für Wasserstoff, Halogen oder Methyl stehen.

Gruppe Ib:

Verbindungen der Formel I, worin X eine der Gruppen $-CH(OR_1)-$, $-C(O)-$ oder $-C(=N-OH)-$ repräsentiert, wobei $R_1$ Wasserstoff, $R_4-C(O)-$ oder $-Si(R_5)(R_6)(R_7)$ bedeutet, wobei $R_4$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxi, $C_1$-$C_3$-Haloalkoxi, Cyano und/oder Nitro substituierte Gruppe aus der Reihe Phenyl und Benzyl steht; $R_5$, $R_6$, $R_7$ unabhängig voneinander für $C_1$-$C_4$-Alkyl, Benzyl oder Phenyl stehen; $R_2$ für Methyl, Ethyl, Isopropyl oder sek.-Butyl steht; und $R_3$ für Wasserstoff, $C_1$-$C_4$-Alkyl, einen unsubstituierten oder einen ein- bis dreifach durch Halogen, $C_1$-$C_2$-Alkyl, $C_1$-$C_2$-Alkoxy, $C_1$-$C_2$-Alkylthio, $C_1$-$C_2$-Haloalkyl, Nitro oder Cyano substituierten Rest aus der Reihe Phenyl und Benzyl steht oder die Gruppe U

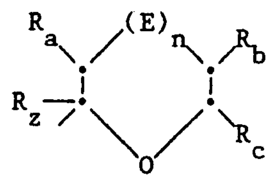 (U)

repräsentiert, worin n für eine der Zahlen 0 oder 1 steht, E für Sauerstoff oder $CH_2$ und $R_z$ für Wasserstoff steht, $R_a$ Wasserstoff, Fluor, Chlor, Brom, Methyl oder 2-Tetrahydropyranyl bedeutet und $R_b$ und $R_c$ unabhängig voneinander für Wasserstoff, Halogen oder Methyl stehen.

Gruppe Ic:

Verbindungen der Formel I, worin X die Gruppe $-CH(OR_1)-$repräsentiert, wobei $R_1$ Wasserstoff, $R_4-C(O)-$ oder $-Si(R_5)(R_6)(R_7)$ bedeutet, wobei $R_4$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxi, $C_1$-$C_3$-Haloalkoxi, Cyano und/oder Nitro substituierte Gruppe aus der Reihe Phenyl und Benzyl steht; $R_5$, $R_6$, $R_7$ unabhängig voneinander für $C_1$-$C_4$-Alkyl, Benzyl oder Phenyl stehen; $R_2$ für Methyl, Ethyl, Isopropyl oder sek.-Butyl steht; und $R_3$ für Wasserstoff, $C_1$-$C_4$-Alkyl, einen unsubstituierten oder einen ein- bis dreifach durch Halogen, $C_1$-$C_2$-Alkyl, $C_1$-$C_2$-Alkoxy, $C_1$-$C_2$-Alkylthio, $C_1$-$C_2$-Haloalkyl, Nitro oder Cyano substituierten Rest aus der Reihe Phenyl und Benzyl steht oder die Gruppe U

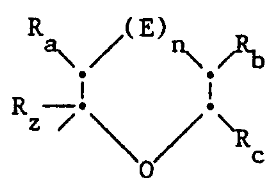 (U)

repräsentiert, worin n für eine der Zahlen 0 oder 1 steht, E für Sauerstoff oder $CH_2$ und $R_z$ für Wasserstoff steht, $R_a$ Wasserstoff, Fluor, Chlor, Brom, Methyl oder 2-Tetrahydropyranyl bedeutet und $R_b$ und $R_c$ unabhängig voneinander für Wasserstoff, Halogen oder Methyl stehen.

Gruppe Id:

Verbindungen der Formel I, worin $R_1$ Wasserstoff bedeutet; $R_2$ für Methyl, Ethyl, Isopropyl oder sek.-Butyl steht; und $R_3$ Wasserstoff, $C_1$-$C_4$-Alkyl, Phenyl, Benzyl, oder eine der Gruppen

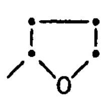 , 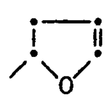 , 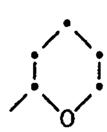 oder 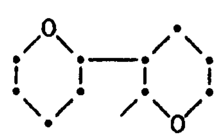

repräsentiert.

Gruppe Ie:

Verbindungen der Formel I, worin X eine der Gruppen $-C(O)$ oder $C(=N-OH)$ bedeutet; $R_2$ für Methyl,

Ethyl, Isopropyl oder sek.-Butyl steht; und $R_3$ Wasserstoff, $C_1$-$C_4$-Alkyl, Phenyl, Benzyl, oder eine der Gruppen

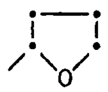 , 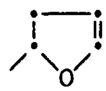 , 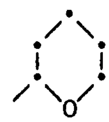 oder 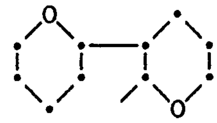

repräsentiert.

Gruppe If:

Verbindungen der Formel I, worin $R_1$ Wasserstoff bedeutet; $R_2$ für Methyl, Ethyl, Isopropyl oder sek.-Butyl steht; und $R_3$ Wasserstoff oder $C_1$-$C_3$-Alkyl bedeutet.

Verbindungen der Formel I, worin $R_2$ für Methyl steht sind bevorzugt. Besonders bevorzugt sind jedoch diejenigen Vertreter der Formel I, worin $R_2$ für Ethyl steht.

Speziell bevorzugte Vertreter der Formel I sind:
5-Acetoxi-29-methoximino-milbemycin D,
5-(Dimethyl-tert.-butyl-silyloxi)-29-hydroximino-milbemycin $A_4$,
29-Hydroximino-milbemycin $A_4$,
29-Methoximino-milbemycin $A_4$,
29-Phenoximino-milbemycin $A_4$,
29-(Tetrahydropyran-2-yl)-oximino-milbemycin $A_4$
5,29-bis-Hydroximino-milbemycin $A_4$

Die Verbindungen der Formel I werden erfindungsgemäss dadurch hergestellt, dass man ein 29-Oxo-Milbemycin der Formel II

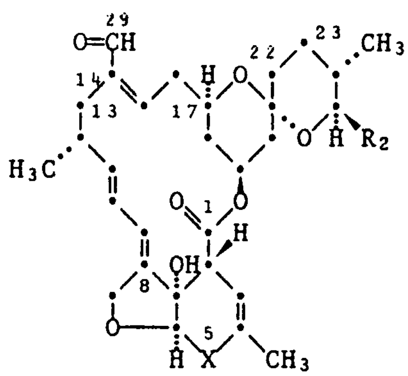

(II)

mit einem primären Oxamin der Formel III

$H_2N$-$OR_3$ (III)

oder einem seiner Salze umsetzt, und sofern gewünscht, die resultierende Verbindung der Formel I, sofern $R_3$ für Wasserstoff steht mit einem Halogenid der Formel IV

$R_3'$ -Hal (IV)

weiterreagieren lässt, wobei die Substituenten $R_2$ und $R_3$ die unter Formel I angegebenen Bedeutungen haben, X für -$C(OR_1)$- steht, wobei $R_1$ Wasserstoff oder eine OH-Schutzgruppe bedeutet und Hal für Halogen steht, und $R_3'$ alle Bedeutungen von $R_3$, mit Ausnahme von Wasserstoff hat, wobei man in der Regel von Verbindungen der Formel II ausgeht, die entweder eine freie 5 Hydroxigruppe enthalten und, sofern gewünscht, die resultierenden Produkte am 5-O-Atom nachacyliert oder nachsilyliert oder bereits von einer in 5-Position silylierten oder acylierten Verbindung der Formel II ausgeht und die Schutzgruppe, sofern gewünscht, abspaltet; oder sofern Verbindungen der Formel I, worin X für die Gruppe -C(O)-steht, das Herstellungsziel sind, von Verbindungen der Formel II ausgeht, die eine freie 5-Hydroxygruppe

enthalten und diese mit einem zur Oxidation geeigneten Reagenz behandelt; oder aber, sofern Verbindungen der Formel I, worin X für die Gruppe -C(=N-OR)- steht, wobei R die unter Formel I angegebene Bedeutung hat, gewünscht sind, von einer Verbindung der Formel I ausgeht, in der X für -C(O)- steht und diese mit Hydroxylamin oder einem seiner Salze umsetzt und gewünschtenfalls anschliessend den Substituenten R einführt oder aber die Umsetzung mit einer Verbindung der Formel $NH_2$-OR oder einem Salz davon vornimmt. Hal in der Formel IV steht vorzugsweise für Chlor oder Brom.

Die Herstellung der C(29)-Oxime der Formel I erfolgt durch Reaktion einer C(29)-Oxo-Verbindung der Formel II mit einem primären Oxamin der Formel III oder einem seiner Salze, vorzugsweise einem seiner Mineralsäuresalze, insbesondere seinem Hydrochlorid. Die Reaktion wird zweckmässigerweise in einem geeigneten Lösungsmittel, z.B. einem Niederalkanol, wie z.B. Methanol, Ethanol, Propanol; einer etherartigen Verbindung, wie z.B. Tetrahydrofuran oder Dioxan; einer aliphatischen Carbonsäure, wie z.B. Essigsäure oder Propionsäure; Wasser oder in Gemischen dieser Lösungsmittel untereinander oder mit anderen üblichen reaktionsinerten Lösungsmitteln durchgeführt. Die Reaktionstemperaturen können in weiten Bereichen variieren. Man arbeitet zweckmässigerweise etwa im Bereich von +10° bis +100°C. Wird das Oxamin in Form eines Salzes, z.B. als Hydrochlorid eingesetzt, so ist es vorteilhaft, wenn man zum Abfangen der Säure (z.B. HCl) eine der für solche Zwecke üblichen Basen zusetzt und zusätzlich in Gegenwart eines Wasserbinders, z.B. eines Molekularsiebes, arbeitet. Als geeignete Basen kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide, Hydride und Hydroxide, Carbonate und Hydrogencarbonate von Alkali- und Erdalkalimetallen (CaO, BaO, NaOH, KOH, NaH, $Ca(OH)_2$, $KHCO_3$, $NaHCO_3$, $Ca(HCO_3)_2$, $K_2CO_3$, $Na_2CO_3$), sowie Alkaliacetate wie $CH_3COONa$ oder $CH_3COOK$. Darüberhinaus eignen sich auch Alkalialkoholate wie $C_2H_5ONa$, $C_3H_7$-nONa usw. Bevorzugt werden Trialkylamine, insbesondere Triethylamin.

Die Reaktion eines Oxims der Formel I, bei dem $R_3$ für Wasserstoff steht, mit einem Halogenid der Formel IV wird im allgemeinen in einem reaktions-inerten Lösungsmittel oder in einem der beteiligten Reaktanden, sofern diese flüssig sind, durchgeführt. Geeignete Lösungsmittel sind z.B.: Ether und etherartige Verbindungen wie Dialkylether (Diethylether, Diisopropylether, tert.-Butylmethylether, Dimethoxiethan, Dioxan, Tetrahydrofuran, Anisol, usw.); halogenierte Kohlenwasserstoffe wie Chlorbenzol, Methylenchlorid, Ethylenchlorid, Chloroform, Tetrachlorkohlenstoff, Tetrachlorethylen, usw.; oder Sulfoxide wie Dimethylsulfoxid, wobei auch aromatische oder aliphatische Kohlenwasserstoffe, wie Benzol, Toluol, Xylole, Petrolether, Ligroin, Cyclohexan, usw. anwesend sein können. In manchen Fällen kann es von Vorteil sein, wenn die Reaktionen unter Schutzgasatmosphäre (z.B. Argon, Helium, Stickstoff, etc.) und/oder in absoluten Lösungsmitteln durchgeführt werden. Gewünschtenfalls können die Endprodukte auf übliche Art und Weise gereinigt werden, z.B. durch Waschen, Dispergieren, Extraktion, Umkristallisation, Chromatographie usw..

Die Reaktion von Verbindungen der Formel I, worin $R_3$ für H steht, mit Halogeniden der Formel IV wird normalerweise in den obengenannten reaktionsinerten Lösungsmitteln im allgemeinen bei Temperaturen von 0° bis 100°C, vorzugsweise von 20° bis 60°C, durchgeführt. Zum Abfangen der dabei als Nebenprodukt gebildeten Säuren findet die Reaktion zweckmässigerweise in Gegenwart eines Neutralisationsmittels statt. Als solche kommen organische Basen in Betracht, z.B. tertiäre Amine wie Trialkylamine (Trimethylamin, Triethylamin, Diisopropylethylamin, Tripropylamin, usw.), Pyridin und Pyridinbasen (4-Dimethylaminopyridin, 4-Pyrrolidylaminopyridin usw.), bevorzugt ist Pyridin. Das Neutralisationsmittel wird üblicherweise in mindestens äquimolarer Menge in bezug auf die Ausgangsstoffe eingesetzt. Die organischen Basen können auch als Lösungsmittel eingesetzt werden. Ferner kann auch ein Katalysator wie p-Dimethylaminopyridin zugesetzt werden.

Die Oximderivate der Formel III und Halogenide der Formel IV sind allgemein bekannt oder können analog zu den bekannten Vertretern hergestellt werden.

Zur Herstellung der 5-Oxime [X = -C(=N-OR)-] im Umfang der Formel I können die am 5-C-Atom vorliegenden Substituenten abgespalten und gewünschtenfalls durch andere Substituenten ersetzt werden, soweit diese den erfindungsgemässen Definitionen entsprechen. Die Abspaltungen und Einführungen definitionsgemässer Substituenten können nach an sich bekannten Methoden erfolgen. Zur Einführung von Acyl-, und Silylgruppen geht man zweckmässigerweise von Verbindungen der Formeln I, in denen X für -CH(OH)- oder -C(=N-OH)- steht, von Verbindungen der Formel S, in denen $R_1$ für Wasserstoff steht, oder von Verbindungen der Formel VIII aus. Die Herstellung von Verbindungen der Formel I, in denen X für -C(=N-OR)- steht, kann beispielsweise erfolgen, indem man Verbindungen der Formeln I, in denen X für -C(O)- steht, mit Hydroxylamin oder einem seiner Salze umsetzt und gewünschtenfalls anschliessend den Substituenten R, wobei R die für Formel I angegebenen Bedeutungen mit Ausnahme von Wasserstoff hat, einführt, oder indem man die Umsetzung mit einer Verbindung der Formel $NH_2$-OR, worin R die für Formel I angegebenen Bedeutungen mit Ausnahme von Wasserstoff hat, oder einem Salz davon vornimmt. Als

Salze kommen beispielsweise solche der vorgenannten Aminoverbindungen mit Schwefelsäure, Salpetersäure und vor allem Salzsäure in Betracht. Die Reaktion wird zweckmässigerweise in einem geeigneten Lösungsmittel ausgeführt, z.B. einem Niederalkanol, wie Methanol, Ethanol, Propanol; einer etherartigen Verbindung, wie Tetrahydrofuran oder Dioxan; einer aliphatischen Carbonsäure, wie Essigsäure oder Propionsäure; Wasser oder in Gemischen dieser Lösungsmittel untereinander oder mit anderen üblichen reaktionsinerten Lösungsmitteln. Die Reaktionstemperaturen können in weiten Bereichen variieren. Man arbeitet zweckmässigerweise etwa im Bereich von +10° bis +100°C. Wird Hydroxylamin in Form eines seiner Salze, z.B. als Hydrochlorid eingesetzt, so ist es vorteilhaft, wenn man zum Abfangen der Säure eine der für solche Zwecke üblichen Basen zusetzt und gegebenenfalls in Gegenwart eines Wasserbinders, z.B. eines Molekularsiebes, arbeitet. Als geeignete Basen kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide, Hydride und Hydroxide, Carbonate und Hydrogencarbonate von Alkali-und Erdalkalimetallen (CaO, BaO, NaOH, KOH, NaH, $Ca(OH)_2$, $KHCO_3$, $NaHCO_3$, $Ca(HCO_3)_2$, $K_2CO_3$, $Na_2CO_3$), sowie Alkaliacetate wie $CH_3COONa$ oder $CH_3COOK$. Darüberhinaus eignen sich auch Alkalialkoholate wie $C_2H_5ONa$, n-$C_3H_7ONa$ usw. Bevorzugt wird Triethylamin.

Die unter den Umfang der Formel I fallenden 5-Keto-Milbemycine, in denen X für -C(O)- steht, lassen sich beispielsweise gewinnen, indem man Verbindungen der Formeln I, in denen X für -CH(OH)- steht, mit einem zur Oxidation geeigneten Reagenz behandelt. Geeignete Oxydationsmittel sind beispielsweise aktiviertes Mangandioxyd, Oxalylchlorid/Dimethylsulfoxyd/Triethylamin oder Chromtrioxyd/Pyridin. Ein geeignetes Verfahren stellt auch die Oppenauer-Oxydation dar, bei welcher Verbindungen der Formeln I, in denen X für -CH(OH)-steht, mit einem Keton, vorzugsweise Cyclohexanon oder Aceton, in Gegenwart eines Aluminiumalkoholats, vorzugsweise des Aluminiumisopropylats oder Aluminium-tert.-butylats, umsetzt.

Die Oxydation wird zweckmässigerweise in einem inerten Lösungsmittel durchgeführt. Als geeignete Lösungsmittel kommen Alkane, wie beispielsweise Hexan, Heptan oder Oktan, aromatische Kohlenwasserstoffe, wie beispielsweise Benzol, Toluol oder Xylole, oder bevorzugt chlorierte Kohlenwasserstoffe, insbesondere Methylenchlorid, in Frage. Die Oxydation wird zweckmässigerweise bei Temperaturen von -80°C bis +60°C, vorzugsweise -60°C bis +30°C, durchgeführt.

Aus Verbindungen der Formeln I, in denen X für die Gruppe -C(O)-steht, lassen sich durch Reduktion auf an sich bekannte Weise wieder diejenigen Verbindungen gewinnen, in denen X für die Gruppe -CHOHsteht. Die Reduktion kann beispielsweise durch katalytische Hydrierung mit Platin- oder Raney-Nickel-Katalysator oder gemäss der Meerwein-Ponndorf-Verley-Reduktion mit Aluminiumisopropylat in Isopropanol erfolgen.

Die Verbindungen der Formel II können durch eine oxidative allylische Umlagerung aus 15-Hydroxi-Milbemycinderivaten der Formel V

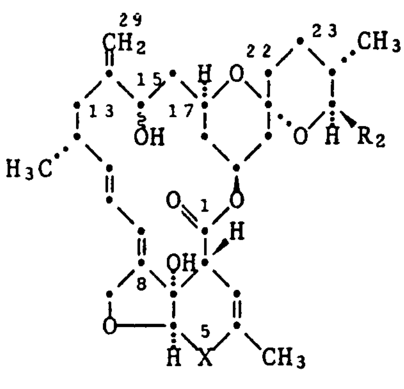

(V),

worin X und $R_2$ die unter Formel I angegebenen Bedeutungen haben, erhalten werden.

Die Reaktion kann wie folgt skizziert werden:

Oxidative allylische Umlagerung

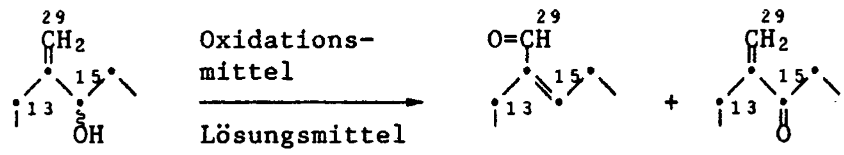


(V) (II) (VI)

des Allylalkohols (V) zu einer 29-Oxo-Verbindung (II). Hierbei wird der Allylalkohol (V) mit einem geeigneten Oxidationsmittel in einem reaktionsinerten Lösungsmittel oxidativ in den entsprechenden Aldehyd ( = 29-Oxo-Verbindung) (II) umgelagert. Dabei entsteht im allgemeinen als Nebenprodukt das entsprechende ungesättigte Keton (VI), das seinerseits Zwischenproduktcharakter aufweist, da es auf Grund seiner Reaktivität zur Synthese weiterer Milbemycin-Derivate verwendet werden kann. Normalerweise entstehen nebeneinander trans- und cis-Form des Aldehyds (II), wobei die trans-Form im allgemeinen überwiegt. Die Verbindungen der Formel II und VI lassen sich säulenchromatographisch, z.B. an Kieselgel, leicht voneinander trennen.

Die 29-Oxo-Verbindungen der Formel II haben folgende chemische Struktur

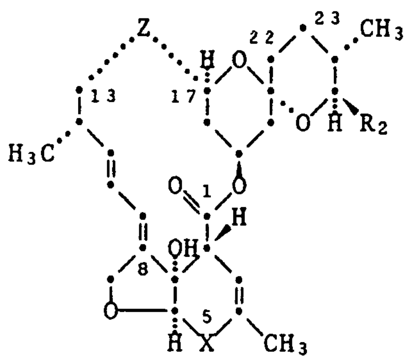

(X),

worin Z für eine der Gruppen

α) 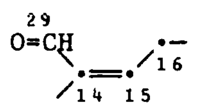

[ = $\Delta^{14,15\text{-trans}}$-29-Oxo = Verbindungen der Formel X ]

oder

β) 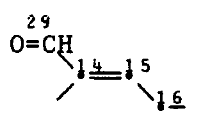

[ = $\Delta^{14,15\text{-cis}}$-29-Oxo = Verbindungen der Formel X ]

steht und X und $R_2$ die unter Formel I angegebenen Bedeutungen haben. Die Ueberführung der $\Delta^{14,15\text{-trans}}$-29-oxo-Verbindungen der Formel X in die $\Delta^{14,15\text{-cis}}$-29-oxo-Verbindungen der Formel X kann analog zu an sich bekannten Verfahren durchgeführt werden. Verbindungen der Formel X sind auf Grund ihrer spezifischen Struktur für die Herstellung der wertvollen Endprodukte der Formel I prädestiniert, da sie die unmittelbare Vorstufe zu deren Herstellung darstellen.

Als (oxidative) Umlagerungsreagenzien kommen insbesondere Cr-VI-Verbindungen, wie z.B. Pyridiniumdichromat, Pyridiniumchlorochromat, usw., in Frage. Man arbeitet zweckmässigerweise in einem reakionsinerten Lösungsmittel. Geeignete Lösungsmittel sind z.B. Ether und etherartige Verbindungen wie Dialkylether (Diethylether, Diisopropylether, tert.Butylmethylether, Dimethoxiethan), Dioxan, Tetrahydrofuran, Anisol, usw.; halogenierte Kohlenwasserstoffe wie Chlorbenzol, Methylenchlorid, Ethylenchlorid, Chloroform, Tetrachlorkohlenstoff, Tetrachlorethylen, usw.; Sulfoxide wie Dimethylsulfoxid; Amide wie N,N-Dimethylformamid; Ester wie Ethylacetat, Propylacetat, Butylacetat, usw.; sowie Mischungen dieser Lösungsmittel untereinander oder mit Wasser und/oder anderen üblichen inerten Lösungsmitteln wie Benzol, Xylol, Petrolether, Ligroin, Cyclohexan, usw. In manchen Fällen kann es von Vorteil sein, wenn die Reaktion oder Teilschritte davon unter Schutzgasatmosphäre (z.B. Argon, Helium, Stickstoff, etc.) und/oder in absoluten Lösungsmitteln durchgeführt werden. Gewünschtenfalls können Zwischenprodukte aus dem Reaktionsmedium isoliert und falls erwünscht, vor der Weiterreaktion auf übliche Art und Weise gereinigt werden, z.B. durch Waschen, Dispergieren, Extraktion, Umkristallisation, Chromatographie usw. Man kann jedoch auch auf derartige Reinigungsschritte verzichten und diese erst mit den entsprechenden Endprodukten durchführen. Bei der oxidativen allylischen Umlagerung beträgt die Reaktionstemperatur normalerweise -50° bis +50°C, vorzugsweise -10° bis +30°C. Die Reaktionszeit hängt im wesentlichen von den Reaktionstemperaturen ab und variiert im allgemeinen zwischen 10 Minuten und ca. 12 Stunden.

Die Ketone der Formel VI

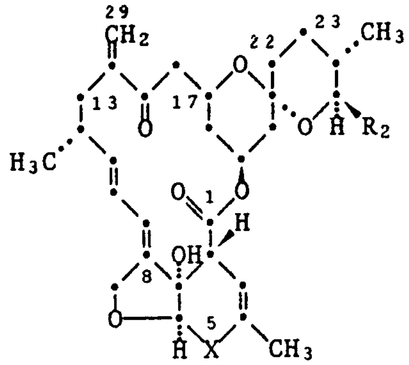

(VI),

worin

X und $R_2$ die unter Formel I angegebenen Bedeutungen haben, sind auf Grund ihres Zwischenprodukte-Charakters, zur Herstellung weiterer Milbemycin-derivate geeignet.

Verbindungen der Formel II können auch erhalten werden durch eine allylische Umlagerung einer Verbindung der Formel A,

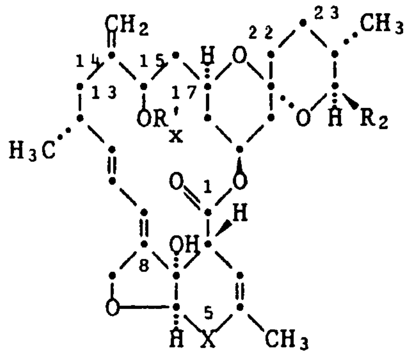

(A),

worin X und $R_2$ eine der unter Formel I angegebenen Bedeutungen haben und $R_x'$ für Wasserstoff oder eine leicht abspaltbare Gruppe steht, nach allgemein bekannten Methoden, wie z.B. durch Behandlung mit einer wässrigen Säure (z.B. einer Mineralsäure, wie HCl, HBr, $H_2SO_4$ etc., einer organischen Säure, wie p-Toluolsulfonsäure, Camphersulfonsäure etc.) mit oder ohne Zusatz eines üblichen, reaktionsinerten Lösungsmittels wie z.B. Tetrahydrofuran, Diethylether, Dioxan etc.,

zu einer Verbindung der Formel B,

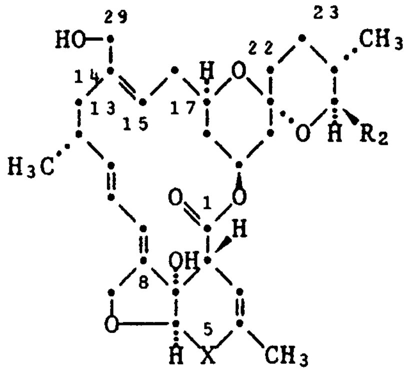

(B)

worin X und $R_2$ die unter Formel I angegebenen Bedeutungen haben, und Ueberführung der erhaltenen Verbindungen der Formel B nach an sich bekannten Methoden, z.B. durch Oxidation mit einem geeigneten Reagenz, in Verbindungen der Formel II.

Als geeignetes Reagenz zur Oxidation kommt z.B. aktiviertes Mangandioxid, Oxalylchlorid/Dimethylsulfoxid/Triethylamin, Chromtrioxid/Pyridin oder weitere dem Fachmann geläufige Oxidationsmittel in Betracht. Man arbeitet dabei normalerweise in einem reaktionsinerten Lösungsmittel.

Als Lösungsmittel kommen Kohlenwasserstoffe, wie Hexan, Heptan, Octan, aromatische Kohlenwasserstoffe, wie Benzol, Toluol, Xylole und bevorzugt chlorierte Kohlenwasserstoffe, insbesondere Methylenchlorid in Frage. Die Reaktionen werden bei Temperaturen von -80 ° C bis 60 ° C, vorzugsweise von -60 ° C bis 30 ° C durchgeführt.

Die Ausgangsverbindungen der Formel V lassen sich durch Singulett-Sauerstoff-Oxidation aus gegebenenfalls entsprechend abgewandelten Milbemycin-Derivaten der Formel VII

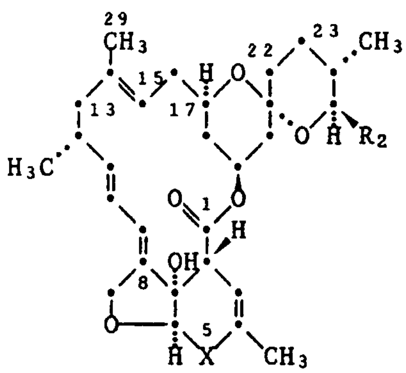

(VII),

worin X und $R_2$ die für Formel I gegebene Bedeutung haben, und anschliessende selektive Reduktion des intermediär entstandenen 15-Peroxids

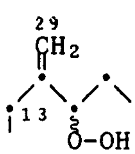

15-Peroxid

mit Natriumborhydrid, Lithiumaluminiumhydrid oder Triphenylphosphin herstellen. Die Reaktion wird bei sichtbarem Licht in Gegenwart eines Sensibilisators bei Normaldruck und bei einer Temperatur von -90 ° C bis +45 ° C, vorzugsweise 0 ° C bis +20 ° C, in einem inerten Lösungsmittel durchgeführt. Vorzugsweise wird in einer Bestrahlungsapparatur gearbeitet.

Der Reaktionsverlauf lässt sich schematisch so darstellen:

1) Sauerstoff+Licht+ Sensibilisator
2) Reduktion

Verbindung VII $\longrightarrow$ Verbindung V

(Vgl. H.H. Wassermann et al. "Singulett Oxygen"; Academic Press, New York 1979; oder B. Ranby et al. "Singulett Oxygen Reactions with Organic Compounds and Polymers", Wiley, New York 1978).

Analog zu dem hier beschriebenen Verfahren lassen sich auch die Verbindungen der Formel A herstellen.

Als Lösungsmittel eignen sich z.B. Ether und etherartige Verbindungen wie Diethylether, Diisopropylether, Dioxan und Tetrahydrofuran; aromatische Kohlenwasserstoffe wie Benzol, Toluol und Xylol; Ketone wie Aceton, Methylethylketon und Cyclohexanon; Nitrile wie Acetonitril; Ester wie Aethylacetat und Butylacetat; sowie Dimethylformamid, Dimethylsulfoxid und halogenierte Kohlenwasserstoffe, oder Mischungen dieser Lösungsmittel mit Wasser.

Als Sensibilisator eignen sich Farbstoffe wie z.B. Methylenblau, Bengalrosa, Chlorophyll, Erysathrosin, Eosin, Zinktetraphenylporphin, Haematoporphyrin, Riboflavin, Fluorescein oder Acridinorange. Ohne weitere Aufarbeitung wird nach Abschluss der Oxydation bei einer Temperatur von 0° bis 20°C selektiv reduziert.

Als Lichtquelle empfiehlt sich eine Lampe mit einer Leistung von 60-500 Watt, bevorzugt 100-350 Watt. Sofern Schutzgruppen für die 5-Hydroxi-Gruppe gewünscht werden, kommen die bereits für $R_1$ genannten Silyl- und Acyl-Gruppen in Frage oder z.B. ein Benzylether, Methoxiethoximethylether oder Dihydrofuran- oder Dihydropyran-Reste. Diese Schutzgruppen können in Verbindungen der Formel VII eingeführt und später auf übliche Art wieder abgespalten werden.

Die Verbindungen der Formel VII, worin X für die Gruppe -CH($OR_1$) steht und $R_1$ Wasserstoff und $R_2$ Methyl, Ethyl, Isopropyl oder sek.Butyl bedeutet, sind entweder aus der US-PS 3.950 360 bekannt geworden und wurden ursprünglich als "Antibiotika B-41-A", später als "Milbemycin-A"-Verbindungen bezeichnet, oder sind aus der US-PS 4.346.171 bekannt und werden als "B-41" oder "Milbemycin-D" bezeichnet oder sind aus der US-PS 4.173.571 bekannt geworden und werden als 13-Deoxi-22,23-dihydro-Avermectin ($R_2$ = sec.Butyl) bezeichnet. Die bekannten, natürlich vorkommenden Milbemycine haben folgende chemische Struktur [Formel (VIII)]:

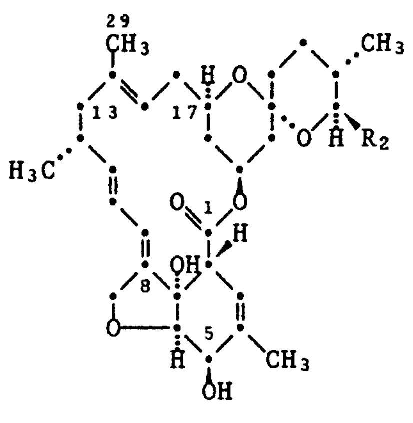

(VIII)

$R_2$ = $CH_3$ Milbemycin $A_3$

$R_2$ = $C_2H_5$ Milbemycin $A_4$

$R_2$ = iso$C_3H_7$ Milbemycin D

$R_2$ = sec.$C_4H_9$ 13-Deoxi-22,23-dihydro-C-076-Bla-aglycon, oder 13-Deoxi-22,23-dihydro-avermectin-Bla-aglykon.

Verbindungen der Formel VII worin X für Gruppe -CH($OR_1$)- steht, wobei $R_1$ Wasserstoff bedeutet, und $R_2$ für die Gruppe

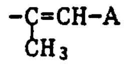

steht, worin A die unter Formel I angegebene Bedeutung hat, stellen C(23)-Deoxi-Derivate der natürlichen Antibiotika S541 dar, die aus der DE 35 32 794 bekannt geworden sind, und durch die folgende chemische Struktur charakterisiert werden:

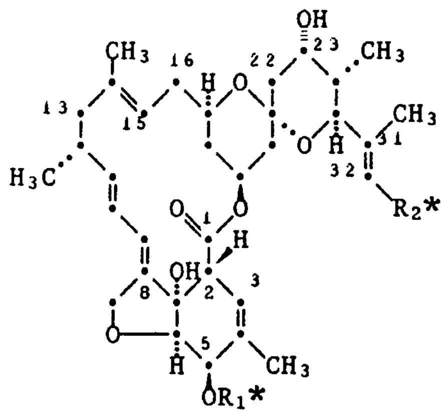

(Antibiotika S541) (S)

| | | |
|---|---|---|
| Faktor A | $R_2$*=iso$C_3H_7$ | $R_1$*=H |
| Faktor B | $R_2$*=$CH_3$ | $R_1$*=$CH_3$ |
| Faktor C | $R_2$*=$CH_3$ | $R_1$*=H |
| Faktor D | $R_2$*=$C_2H_5$ | $R_1$*=H |
| Faktor E | $R_2$*=$C_2H_5$ | $R_1$*=$CH_3$ |
| Faktor F | $R_2$*=iso$C_3H_7$ | $R_1$*=$CH_3$ |

Die in C(23)-Position befindlichen Hydroxigruppe in den Antibiotika S541 lässt sich analog zu der in US-PS 4,328,335 beschriebenen Methode entfernen, wodurch die Antibiotika S541 in die entsprechenden 23-Deoxi-Derivate überführt werden. Dabei müssen diejenigen Verbindungen mit einer freien 5-OH-Gruppe ($R_1$* = H) zunächst selektiv geschützt werden durch Reaktion mit einem der oben angegebenen Silylierungsreagenzien Y-Si($R_5$)($R_6$)($R_7$) bzw. mit tert.-Butyldimethylsilyloxiacetylchlorid. Die Umsetzung dieser geschützten Verbindungen, in denen $R_1$* durch Si($R_5$)($R_6$)($R_7$) bzw. C(=0)$CH_2OSi(CH_3)_2$t-$C_4H_9$ ersetzt und das 23 C-Atom durch OH substituiert ist, mit p-Methylphenyl-chlorthionoformiat ergibt Derivate der Antibiotika S541, welche in Position 23 durch -O-C(=S)-O-(4-$CH_3$-$C_6H_4$) substituiert sind. Diese 23-O-(4-Methylphenoxi)-thiocarbonyl-derivate von Antibiotika S541 werden dann als Ausgangsmaterialien für die Reduktion mit Tributylzinnhydrid in Toluol in Gegenwart von Azobisisobutyronitril bei 80-120 °C zu den entsprechenden 23-Deoxi-Derivaten (Position 23 unsubstituiert) eingesetzt.

Durch Acylierung oder Silylierung der 5-OH-Gruppe werden auch alle jene Derivate der Formeln I und VII hergestellt, bei denen $R_1$ eine andere Bedeutung als Wasserstoff ($R_1$ = OH-Schutzgruppe) hat sowie gewünschtenfalls Ausgangsprodukte wie Milbemycine oder S541-Antibiotika oder Ziwschenprodukte, bei denen die in 5-Position befindliche Hydroxygruppe geschützt werden soll. Die Einführung der Acylgruppe erfolgt üblicherweise mit den entsprechenden Acylhalogeniden oder Acylanhydriden und wird vorzugsweise zur Einführung der eingangs definierten $R_4$C(O)- Gruppe benutzt. Zur Silylierung verwendet man zweckmässigerweise ein Silan der Formel Y-Si($R_5$)($R_6$)($R_7$), worin $R_5$, $R_6$ und $R_7$ einen der eingangs genannten Reste darstellen, wobei der Begriff Acylhalogenid für Acylchlorid oder Acylbromid steht und wobei Y eine Silylabgangsgruppe bedeutet. Zu den Silylabgangsgruppen Y zählen beispielsweise Brom, Chlor, Cyanid, Azid, Acetamid, Trifluoracetat, Trifluormethansulfonat. Diese Aufzählung stellt keine Limitierung dar, der Fachmann kennt weitere typische Silylabgangsgruppen.

5-O-Acylierungen und 5-O-Silylierungen werden in wasserfreiem Milieu, vorzugsweise in inerten Lösungsmitteln und besonders bevorzugt in aprotischen Lösungsmitteln durchgeführt. Die Reaktion läuft vorteilhaft im Temperaturbereich von 0° bis +80 °C, bevorzugt bei +10° bis +40 °C, ab. Vorzugsweise

wird eine organische Base zugegeben. Es kommen als solche beispielsweise tertiäre Amine wie Triethylamin, Triethylendiamin, Triazol und bevorzugt Pyridin, Imidazol oder 1,8-Diazabicyclo[5.4.0]-undec-7-en (DBU) in Frage.

Die Entfernung dieser Silyl- und Acylreste $R_1$ und $R_1^*$ in der 5-Position geschieht durch selektive milde Hydrolyse (→ $R_1$; $R_1^* = H$) mit z.B. verdünnten Säuren, wie verdünnte HCl, HF, Arylsulfonsäure, in wässriger oder alkoholischer Lösung oder nach einer anderen dem Fachmann geläufigen Methode.

Acylreste werden vorzugsweise auch basisch (z.B. in alkoholischer Ammoniaklösung) abgespalten.

Das beschriebene Verfahren zur Herstellung der Verbindungen der Formel I ist in allen Teilschritten ein Bestandteil vorliegender Erfindung.

Die Verbindungen der Formel I eignen sich ausgezeichnet zur Bekämpfung von Schädlingen an Tieren und Pflanzen in allen ihren Entwicklungsstadien, darunter insbesondere von tierparasitären Ekto-Parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae, Gastrophilidae.

Die Verbindungen I sind auch einsetzbar gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen der Formel I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Loccidae, Diaspididae und Eriophydidae (z.B. die Rostmilbe auf Citrusfrüchten); der Ordnungen Hemiptera; Heteroptera und Thysanoptera; sowie bei den pflanzenfressenden Insekten der Ordnungen Lepidoptera, Coleoptera, Diptera und Orthoptera.

Sie sind ebenfalls als Bodeninsektizid gegen Schädlinge im Erdboden geeignet.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen der Formel I sind auch wirksam gegen Pflanzen-Nematoden der Arten Meloidogyne, Heterodera, Pratylenchus, Ditylenchus, Radopholus, Rizoglyphus und andere.

Ferner sind die Verbindungen gegen Helminthen in allen Entwicklungsstadien wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostomum, Oesophagostomum, Charbertia, Trichuris, Strongylus, Trichonema, Dictyocaulus, Capillaria, Heterakis, Toxocara, Ascaridia, Oxyuris, Ancylostoma, Uncinaria, Toxascaris und Parascaris. Der besondere Vorteil der Verbindungen der Formel I ist ihre Wirksamkeit gegen solche Parasiten, die gegen Wirkstoffe auf Benzimidazol-Basis resistent sind.

Gewisse Spezies der Gattungen Nematodirus, Cooperia und Oesophagostomum greifen den Intestinaltrakt des Wirtstiers an, während andere der Gattungen Haemonchus und Ostertagia im Magen und solche der Gattung Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diese Parasiten hoch wirksam.

Weiter sind die Verbindungen der Formel I zur Bekämpfung von humanpathogenen Parasiten geeignet, unter denen als typische, im Verdauungstrakt vorkommende Vertreter solche der Gattungen Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Gattungen Wuchereria Brugia, Onchocerca und Loa aus der Familie der Filariidae, die im Blut, im Gewebe und verschiedenen Organen vorkommen, ferner gegen Dracunculus und Parasiten der Gattungen Strongyloides und Trichinella, die speziell den Gastro-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht dosiert. Falls die Verbindungen der Formel I bzw. entsprechende Mittel zur Bekämpfung von endoparasitären Nematoden, Cestoden und Trematoden bei Haus- und Nutztieren, wie Rindern, Schafen, Ziegen, Katzen und Hunden, verwendet werden, können sie den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew. %. Die Mittel können in Form von Lösungen, Emulsionen, Suspensionen, Pulver, Tabletten, Bolussen oder Kapseln peroral den Tieren verabreicht werden. Soweit die physikalischen und toxikologischen Eigenschaften von Lösungen oder Emulsionen dies zulassen, können die Verbindungen der Formel I bzw. die sie enthaltenden Mittel an Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich. Ueber geschlossenen Kultur-Anbauflächen werden sie zweckmässigerweise in Mengen von 10 g bis 1000 g pro Hektar angewendet. Sie kommen ferner in Pferchen, Gehegen, Stallungen oder sonstigen Räumen zur Anwendung.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon,Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Die in der Formulierungstechnik gebräuchlichen Tenside sind u.a. in folgender Publikation beschrieben:

"1986 International Mc Cutcheon's Emulsifiers and Detergents"
The Manufacturing Confectioner Publishing Co.,
Glen Rock, New Jersey, USA.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als Wirkstoff mindestens eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

### Herstellung von Ausgangs- und Zwischenprodukten

#### Beispiel A1: Herstellung der Verbindungen $\Delta^{14,29}$-15-Hydroxi-milbemycin D (Formel V) und 14-Hydroxi-$\Delta^{15,16}$-milbemycin D aus Milbemycin D

Eine Lösung von 5,56 g Milbemycin D und 0,03 g Methylenblau in 400 ml Acetonitril wird in einer Bestrahlungsapparatur aus Glas unter Sauerstoffzugabe während 10 Stunden bei einer Temperatur von 20 ° C (Projektorlampe 200 W) mit sichtbarem Licht bestrahlt.

Anschliessend wird im Reaktionsgemisch bei einer Temperatur von 20 ° C mit 3,9 g Triphenylphosphin reduziert.

Das Reaktionsgemisch wird nach dem Einengen über einer Silicagelkolonne mit Methylenchlorid/Essigester = 3:1 als Eluiermittel chromatographiert.

Man erhält 4,10 g $\Delta^{14,29}$-15-Hydroxi-Milbemycin D, Smp. 228-229 ° C; Massenspektrum m/e: 572 ($M^+$), 554.

Daneben erhält man 0,34 g 14-Hydroxi-$\Delta^{15,16}$-milbemycin D, Smp. 252-254 ° C; Massenspektrum m/e: 572 ($M^+$), 554.

#### Beispiel A2: Herstellung der Verbindungen 5-Keto-$\Delta^{14,29}$-15-hydroxi-milbemycin D (Formel V) und 5-Keto-14-hydroxi-$\Delta^{15,16}$-milbemycin D aus 5-Keto-milbemycin D

a) Herstellung von 5-Keto-milbemycin D
1 g Milbemycin D, 2 g aktiviertes Mangandioxid und 50 ml wasserfreies Methylenchlorid werden bei 20-25 ° C 4 Stunden gerührt. Die Reaktionsmischung wird filtriert und das Filtrat wird über eine kurze (ca. 30 cm) Schicht Silicagel gereinigt. Man erhält 1 g 5-Keto-Milbemycin in Form einer gelblichen amorphen Masse, Smp. 152-157 ° C.

b) Die Singulett-Oxydation des unter a) hergestellten 5-Keto-Milbemycins und die weitere Aufarbeitung erfolgt nach der im Beispiel 1 angegebenen Methode.

Man erhält nach Chromatograpie an Silicagel 0,6 g 5-Keto-$\Delta^{14,29}$-15-hydroxi-milbemycin D, Smp. 160-165 ° C;
Massenspektrum m/e: 570 ($M^+$), 552.

Daneben erhält man 30 mg 5-Keto-14-hydroxi-$\Delta^{15,16}$-milbemycin D, Smp. 170-174 ° C.

#### Beispiel A3: Herstellung von 5-Keto-$\Delta^{14,29}$-15-hydroxi-milbemycin D (Formel V) und 5-Keto-14-hydroxi-$\Delta^{15,16}$-milbemycin D aus Milbemycin D

Die Mangandioxid-Oxidation als Folgeschritt mit den nach Beispiel 1 durch Singulett-Sauerstoff-Oxidation gewonnenen Verbindungen $\Delta^{14,29}$-15-Hydroxi-milbemycin D und 14-Hydroxi-$\Delta^{15,16}$-milbemycin D liefert in quantitativer Ausbeute 5-Keto-$\Delta^{14,29}$-15-hydroxi-milbemycin D und 5-Keto-14-hydroxi-$\Delta^{15,16}$-milbemycin D.

#### Beispiel A4: Herstellung von 5-Acetyloxi-$\Delta^{14,29}$-15-hydroxi-milbemycin D (Formel V) und von 5-Acetyloxi-14-hydroxi-$\Delta^{15,16}$-milbemycin D aus Milbemycin D

a) Herstellung von 5-Acetyloxi-milbemycin D

560 mg (1,0 mMol) Milbemycin D werden in 20 ml Pyridin mit 160 mg (1,6 mMol) Acetanhydrid versetzt und bei Raumtemperatur über Nacht gerührt. Nach dem Eindampfen des Pyridins wird der Rückstand in 20 ml Ethylacetat aufgenommen und die organische Phase einmal mit 10 ml 1N Chlorwasserstoffsäure-Lösung dann mit 10 ml gesättigter $NaHCO_3$-Lösung und zum Schluss mit 10 ml gesättigter NaCl-Lösung geschüttelt. Nach dem Abtrennen der organischen Phase wird diese über $Na_2SO_4$ getrocknet, filtriert und eingeengt. Man erhält 580 mg 5-Acetyloxi-milbemycin D (amorphes, schwachgelbes Pulver), Smp. 115-120 ° C.

Auf analoge Art lassen sich auch die Acyl-Derivate Milbemycine $A_3$, $A_4$ und das 13-Desoxi-avermectin-Derivat ($R_2$ = sek.Butyl) gewinnen.

b) 560 mg 5-Acetyloxi-milbemycin D werden mit 20 mg Methylenblau in 40 ml Acetonitril mit einer Bestrahlungsapparatur (Projektlampe 200 W) während 8 Stunden bei 18-22 ° C mit Sauerstoff behandelt. Das Reaktionsgemisch wird anschliessend bei Raumtemperatur mit 40 mg Triphenylphosphin reduziert.

Nach dem Einengen wird das Reaktionsgemisch über einer Silicasäule (Laufmittel Methylenchlorid/Ethylacetat 3:1) chromatographiert.

Man erhält 390 mg 5-Acetyloxi-$\Delta^{14,29}$-15-hydroxi-milbemycin D, Smp. 153-156 ° C;
Massenspektrum m/e: 614 ($M^+$), 596.

Daneben erhält man 42 mg 5-Acetyloxi-14-hydroxi-$\Delta^{15,16}$-milbemycin D, Smp. 151-154 ° C.

Beispiel A5: Herstellung von $\Delta^{14,29}$-15-Hydroxi-milbemycin $A_4$ (Formel V) und 14 Hydroxi-$\Delta^{15,16}$-milbemycin $A_4$ aus Milbemycin $A_4$

540 mg (1 mMol) Milbemycin $A_4$ werden in 100 ml Acetonitril analog Beispiel 1 mit Singulett-Sauerstoff oxidiert und anschliessend mit Triphenylphosphin reduziert. Nach Reinigung durch Flash-Chromatographie an Silicagel (Laufmittel Cyclohexan/Ethylacetat 1:1) erhält man 310 mg $\Delta^{14,29}$-15-hydroximilbemycin $A_4$ Smp. 222-225 ° C;
Massenspektrum m/e: 558 ($M^+$), 540.

Daneben erhält man 40 mg 14-Hydroxi-$\Delta^{15,16}$-milbemycin $A_4$, Smp. 147-152 ° C; Massenspektrum m/e: 558 ($M^+$), 540.

Beispiel A6: Herstellung von 5-(Dimethyl-tert.butylsilyloxi)-$\Delta^{14,29}$-15-hydroxi-milbemycin $A_3$ (Formel V) und von 5-(Dimethyl-tert.-butylsilyloxi)-14-hydroxi-$\Delta^{15,16}$-milbemycin $A_3$ aus Milbemycin $A_3$

a) Herstellung von 5-(Dimethyl-tert.butylsilyloxi)-milbemycin $A_3$.

Bei Raumtemperatur werden 480 mg (7 mMol) Imidazol und 460 mg (3 mMol) Dimethyl-tert.butylchlorsilan in 20 ml Methylenchlorid vorgelegt. Unter Rühren tropft man langsam eine Lösung von 655 mg (1,2 mMol) Milbemycin $A_3$ in 10 ml Methylenchlorid dazu. Anschliessend wird das Reaktionsgemisch über Nacht unter Rückfluss (~ 40 ° C) erwärmt. Nach dem Einengen, Reinigen über Silicagel und Trocknen erhält man 730 mg amorphes 5-(Dimethyl-tert.butylsilyloxi)-milbemycin $A_3$, Smp. 55-60 ° C.

Auf gleiche Weise lassen sich auch die Milbemycine $A_4$, D und das 13-Desoxi-Avemectin-Derivat ($R_2$ = sek.Butyl) silylieren, wobei auch vorteilhaft Methyldiphenylchlorsilan oder bis(Isopropyl)-methylchlorsilan eingesetzt werden können.

b) Nach der Vorschrift des Beispiels A4b) werden aus 720 mg 5-Dimethyl-tert.butylsilyl-milbemycin $A_3$ durch Singulett-Sauerstoff-Oxidation mit Bengalrosa als Sensibilisator und anschliessende Reduktion der Peroxide mit Triphenylphosphin 550 mg (5-Dimethyltert.butylsilyloxi)-$\Delta^{14,29}$-15-hydroxi-milbemycin $A_3$ gewonnen, Smp. 238-240 ° C;
Massenspektrum m/e: 658 ($M^+$), 640.

Daneben werden 42 mg 5-(Dimethyl-tert.butylsilyloxi)-14-hydroxi-$\Delta^{15,16}$-milbemycin $A_3$ in amorpher Form erhalten. Smp. 45-50 ° C.

Beispiel A7: Herstellung von $\Delta^{14,29}$-15-Hydroxi-milbemycin $A_3$ (Formel V) und von 14-Hydroxi-$\Delta^{15,16}$-milbemycin $A_3$

120 mg 5-(Dimethyl-tert.butylsilyloxi)-$\Delta^{14,29}$-15-hydroxi-milbemycin $A_3$ werden mit 2 ml einer 1%igen Lösung von p-Toluolsulfonsäure in Methanol 9 Stunden bei Raumtemperatur gerührt und dann mit 5%iger wässriger $NaHCO_3$-Lösung behandelt. Durch Ausschütteln mit dreimal 2 ml Diethylether, Einengen der organischen Phase und Chromatographie des Rohprodukts an 20 g Kieselgel (Laufmittel

Aceton/Methylenchlorid 1:12) erhält man 67 mg $\Delta^{14,29}$-15-Hydroximilbemycin $A_3$, Smp. 219-222 °C.

Entsprechend erhält man aus 60 mg 5-(Dimethyl-tert.butylsilyloxi)-14-hydroxi-$\Delta^{15,16}$-milbemycin $A_3$ 38 mg 14-Hydroxi-$\Delta^{15,16}$-milbemycin $A_3$, Smp. 128-132 °C.

Beispiel A8: Herstellung von 29-Oxo-5-acetyloxi-$\Delta^{14,15\text{-trans}}$-milbemycin D (Formel II) und von 15-Oxo-5-acetyloxi-$\Delta^{14,29}$-milbemycin D (Formel V)

600 mg 15-Hydroxi-5-acetyloxi-$\Delta^{14,29}$-milbemycin D werden bei 10 °C in 35 ml absolutem Dimethylformamid mit 570 mg Pyridiniumdichromat versetzt und 2 Stunden bei Raumtemperatur kräftig gerührt. Das Lösungsmittel wird im Hochvakuum entfernt und das resultierende Harz in Diethylether aufgeschlämmt und filtriert. Die flüssige Phase wird mit Wasser und gesättigter Kochsalzlösung gewaschen, über Natriumsulfat getrocknet und filtriert. Das gelbliche Rohprodukt wird säulenchromatographisch (Kieselgel: Methylenchlorid/Diethylether 15:1) gereinigt und führt zu 330 mg 29-Oxo-5-acetyloxi-$\Delta^{14,15}$-milbemycin D mit einem Schmelzpunkt von 155-159 °C sowie als Nebenprodukt zu 200 mg 15-Oxo-5-acetyloxi-$\Delta^{14,29}$-milbemycin D mit Schmelzpunkt 139-142 °C.

Beispiel A9: Herstellung von 29-Oxo-$\Delta^{14,15\text{cis}}$-milbemycin D (Formel II; X) und von 29-Oxo-$\Delta^{14,15\text{trans}}$-milbemycin D (Formel II; X)

136 mg 29-Oxo-$\Delta^{14,15\text{trans}}$-5-(dimethyl-tert.butyl-silyloxi)-milbemycin D werden bei Raumtemperatur in 15 ml Methanol gelöst, mit 2 ml p-Toluolsulfonsäure versetzt und 1 Stunde gerührt. Anschliessend wird das Lösungsmittel im Hochvakuum entfernt und das Rohprodukt säulenchromatographisch gereinigt (Kieselgel: Methylenchlorid/Diethylether 3:1) Man erhält 125 mg des trans-Produktes in Form eines weissen amorphen Pulvers. Smp. ca. 150 °C. Die Hälfte dieses trans-Produktes wird in Methanol gelöst, mit einigen Tropfen verdünnter Schwefelsäure versetzt und 3 Stunden bei ca. 30 °C gerührt. Dann wird das Lösungsmittel im Hochvakuum entfernt, das Rohprodukt in Methylenchiorid gelöst und durch eine kurze (5 cm lange) Silicagelsäule filtriert. Man erhält auf diese Weise 60 mg des stabileren Cisproduktes, das sich bei ca. 250 °C zersetzt.

Beispiel A10: Herstellung von 15-Mesyloxi-$\Delta^{14,29}$-5-(dimethyl-tert.butyl-silyloxi)-milbemycin-$A_4$

Zu einer Lösung von 670 mg (1 mmol) 15-Hydroxi-$\Delta^{14,29}$-5-dimethyl-tert.butyl-silyloxi-milbemycin-$A_4$ und 405 mg (4 mmol) Triäthylamin in 40 ml trockenem Tetrahydrofuran werden unter Argon bei ca. -10 °C 230 mg (2 mmol) Methansulfochlorid in 2 ml Tetrahydrofuran zugegeben. Unter gutem Rühren wird die Lösung langsam auf ca. +10 °C erwärmt und 30 Minuten weitergerührt. Dieses in situ hergestellte Zwischenprodukt kann ohne Reinigungsschritt weiterverarbeitet werden.

Beispiel A11: Herstellung von 29-Hydroxi-5-(dimethyl-tert.butylsilyloxi)-milbemycin-$A_4$

Das im vorigen Beispiel Nr. A10 hergestellte $A_4$-Derivat in der Tetrahydrofuranreaktionsmischung wird bei ca. 10 °C mit etwa 5.4 ml (300 mmol) Wasser versetzt und über Nacht bei Raumtemperatur gerührt.

Nach Zugabe von 200 ml Essigester wird mit gesättigter Kochsalzlösung ausgeschüttelt und dann über einem geeigneten Trocknungsmittel wie z.B. Natriumsulfat getrocknet. Anschliessend wird das Lösungsmittel im Vakuum entfernt. Die Reinigung mittels einer Silikagel-Kolonne (Methylenchlorid : Diethylether = 20:1) ergibt nach der Gefriertrocknung 470 mg der Titelsubstanz, welche bei 142-145 °C schmilzt.

Beispiel A12: Herstellung von 29-Hydroxi-milbemycin-$A_4$

Wird im vorherigen Beispiel Nr. A11 vor oder nach der Wasserzugabe das Reaktionsgemisch mit p-Toluolsulfonsäure oder Methansulfonsäure angesäuert, erhält man die Titelverbindung, welche nach der Gefriertrocknung bei 143-147 °C schmilzt.

Beispiel A13: Herstellung von 29-oxo-5-(dimethyl-tert.butyl-silyloxi)-milbemycin $A_4$

450 mg (0,66 mmol) 29-Hydroxi-5-(dimethyl-tert.butyl-silyloxi)milbemycin $A_4$ werden bei Raumtemperatur in 60 ml Methylenchlorid vorgelegt. 900 mg Mangandioxid werden dazugegeben, und es wird ca. 12 Stunden kräftig gerührt. Nach dem Filtrieren wird das Lösungsmittel im Vakuum entfernt und das Rohprodukt säulenchromatographisch gereinigt. (Silikagel: Petrolether/Essigester = 3/1).

Man erhält die Titelsubstanz (440 mg) als weisses Pulver, welches bei 250-253 °C schmilzt.

Beispiel A14: Herstellung von 5,29-bis-Oxo-milbemycin A4

190 mg (0,34 mmol) 29-Hydroximilbemycin A4 werden in 30 ml Methylenchlorid bei Raumtemperatur während 5 Stunden in Gegenwart von 1 Gramm Mangandioxid gut gerührt. Nach dem Filtrieren über Hyflo (Cellulose) wird das Lösungsmittel eingedampft.
Das in 122 mg erhaltene amorphe Produkt wird direkt weiterverarbeitet.

Herstellung der Endprodukte

Beispiel E1: Herstellung von 5-Acetyloxi-29-methoximino-milbemycin D

120 mg (0,2 mmol) 5-Acetyloxi-29-oxo-milbemycin D und 100 mg (1.2 mmol) Methoxiaminhydrochlorid werden in einem Gemisch von 10 ml absolutem Ethanol und 5 ml trockenem Tetrahydrofuran während 2 Stunden bei Raumtemperatur gut gerührt. Nach Zugabe von einigen Tropfen Triethylamin wird das Lösungsmittel im Vakuum entfernt und das Produkt säulenchromatographisch gereinigt (Kieselgel: Methylenchlorid/Diethylether = 10/1). Man erhält so 102 mg der Titelsubstanz, welche bei 137-139 °C schmilzt.

Beispiel E2: Herstellung von 5-(Dimethyl-tert.butyl-silyloxi)-29-hydroximino-milbemycin $A_4$

335 mg (0,5 mmol) 5-(Dimethyl-tert.butyl-silyloxi)-29-oxo-milbemycin $A_4$, 417 mg (6 mmol) Hydroxylammoniumchlorid und 1 g Molekularsiebe (Porenweite 3Å - 4Å; Natrium-Aluminium-Silikat Modul) werden in einem Gemisch von 40 ml absolutem Ethanol und 20 ml trockenem Tetrahydrofuran über Nacht bei Raumtemperatur gut gerührt. Nach dem Filtrieren wird das Lösungsmittelgemisch im Vakuum entfernt und das Rohprodukt säulenchromatographisch gereinigt (Kieselgel: Petrolether/Essigester = 5/1). Man erhält 180 mg der Titelsubstanz als amorphes weisses Pulver, welches bei ca. 132-172 °C schmilzt.

Beispiel E3: Herstellung von 29-Hydroximino-milbemycin $A_4$

135 mg (0,2 mmol) 5-(Dimethyl-tert.butyl-silyloxi)-29-hydroximinomilbemycin $A_4$ werden bei Raumtemperatur in 5 ml einer 1 prozentigen methanolischen p-Toluolsulfonsäurelösung gerührt. Nach Zugabe von einigen Tropfen Triethylamin wird das Lösungsmittel unter Vakuum entfernt und das Rohprodukt säulenchromatographisch gereinigt (Kieselgel: Diethylether). Man erhält 104 mg der Titelsubstanz als weisses amorphes Pulver, welches bei 163-167 °C schmilzt.

Beispiel E4: Herstellung von 29-Phenoximino-milbemycin $A_4$

570 mg (0.85 mmol) 5-(Dimethyl-tert.butyl-silyloxi)-29-oxo-milbemycin $A_4$, 725 mg (5 mmol) O-Phenylhydroxylaminhydrochlorid und 2 g Molekularsiebe werden in einem Gemisch aus 40 absolutem Ethanol und 20 ml trockenem Tetrahydrofuran während 3 Stunden bei Raumtemperatur gut gerührt. Nach dem Filtrieren wird das Lösungsmittel am Vakuum entfernt und das Rohprodukt säulenchromatographisch gereinigt (Kieselgel: Methylenchlorid/Petrolether = 5/1). Die erhaltenen 400 mg 5-(Dimethyl-tert.butyl-silyloxi)-29-phenoximino-milbemycin $A_4$ werden in einem Gemisch von 30 ml einer 1 prozentigen methanolischen p-Toluolsulfonsäurelösung und 15 ml trockenem Tetrahydrofuran während 2 Stunden bei Raumtemperatur gerührt. Nach der Entfernung der Lösungsmittel am Vakuum wird das Rohprodukt säulenchromatographisch gereinigt (Kieselgel: Methylenchlorid/Diethylether = 25/1). Man erhält 285 mg der Titelsubstanz als weisses Pulver, welches bei 120-125 °C schmilzt.

Beispiel E5: Herstellung 29-(Tetrahydropyran-2-yl)-oximino-milbemycin $A_4$ und 7-Tetrahydropyranyloxi-29-tetrahydropyranyloximino-milbemycin $A_4$

275 mg (0,4 mmol) 5-(Dimethyl-tert.butyl-silyloxi)-29-hydroximino-milbemycin $A_4$, 201 mg (2,4 mmol) 3,4-Dihydro-2H-pyran und 3 mg Camphersulfonsäure werden in 30 ml Methylenchlorid bei Raumtemperatur während 20 Stunden gut gerührt. Anschliessend wird das Lösungsmittel im Vakuum entfernt und das Rohprodukt in 3,5 ml Fluorwasserstoff-Pyridinkomplex während 3 Tagen in einem Polypropylengefäss in der Dunkelheit stehen gelassen. Nach dem Einengen im Hochvakuum wird säulenchromatographisch

gereinigt (Kieselgel: Petrolether/Diethylether = 1/1). Man erhält 22 mg der di-substituierten Titelverbindung und 65 mg der mono-substituierten Verbindung. Beide amorphen Pulver schmelzen bei 110 115 °C.

Beispiel E6: Herstellung von 5,29-bis-Hydroximino-milbemycin A4

95 mg (0,17 mmol) 5,29-bis-Oxo-milbemycin A4, 210 mg (3 mmol) Hydroxyammoniumchlorid und 1 g Molekularsiebe (Porenweite 3Å-4Å, Natrium-Aluminium-Silicat Modul) werden in einem Gemisch von 20 ml Methanol und 10 ml trockenem Tetrahydrofuran über Nacht bei Raumtemperatur gut gerührt. Nach dem Filtrieren wird das Lösungsmittelgemisch im Vakuum entfernt und das Rohprodukt säulenchromatographisch gereinigt (Kieselgel: Methylenchlorid/Diäethyläther = 10/1).
Man erhält 70 mg der Titelsubstanz als amorphes weisses Pulver, welches bei 184-186 Grad schmilzt.

Analog zu den beschriebenen Arbeitsweisen werden auch die nachfolgend genannten Verbindungen hergestellt:

Tabelle 1: Typische Vertreter von Zwischenprodukten der Formel II
[X = -CH(OR$_1$)-]

[ = $\Delta^{14,15trans}$-29-Oxo ]

| Verb. Nr. | $R_2$ | $R_1$ | Smp. [°C] |
|---|---|---|---|
| 1.1. | $CH_3$ | H | |
| 1.2. | $C_2H_5$ | H | |
| 1.3. | $C_3H_7$-iso | H | 120-124 |
| 1.4. | $C_4H_9$-sek | H | |
| 1.5. | $CH_3$ | $Si(CH_3)_2C_4H_9$-tert | |
| 1.6. | $C_2H_5$ | $Si(CH_3)_2C_4H_9$-tert | 250-253 |
| 1.7. | $C_3H_7$-iso | $Si(CH_3)_2C_4H_9$-tert | 215-220 |
| 1.8. | $C_4H_9$-sek | $Si(CH_3)_2C_4H_9$-tert | |
| 1.9. | $CH_3$ | $C(O)CH_3$ | |
| 1.10. | $C_2H_5$ | $C(O)CH_3$ | |
| 1.11. | $C_3H_7$-iso | $C(O)CH_3$ | 155-159 |
| 1.12. | $C_4H_9$-tert | $C(O)CH_3$ | |

Diese Auflistung hat keinen limitierenden Charakter

Tabelle 2: Typische Vertreter von Zwischenprodukten der Formel II
[X = $-CH(OR_1)-$]

[ = $\Delta^{14,29cis}$-29-Oxo ]

| Verb. Nr. | $R_2$ | $R_1$ | Smp. [°C] |
|---|---|---|---|
| 2.1. | $CH_3$ | H | |
| 2.2. | $C_2H_5$ | H | amorph, ca. 60 |
| 2.3. | $C_3H_7$-iso | H | ab 250 Zer. |
| 2.4. | $C_4H_9$-sek | H | |
| 2.5. | $CH_3$ | $Si(CH_3)_2C_4H_9$-tert | |
| 2.6. | $C_2H_5$ | $Si(CH_3)_2C_4H_9$-tert | 212-215 |
| 2.7. | $C_3H_7$-iso | $Si(CH_3)_2C_4H_9$-tert | 78-83 |
| 2.8. | $C_4H_9$-sek | $Si(CH_3)_2C_4H_9$-tert | |
| 2.9. | $CH_3$ | $C(O)CH_3$ | |
| 2.10. | $C_2H_5$ | $C(O)CH_3$ | |
| 2.11. | $C_3H_7$-iso | $C(O)CH_3$ | |
| 2.12. | $C_4H_9$-tert | $C(O)CH_3$ | |

Diese Auflistung hat keinen limitierenden Charakter

Tabelle 3: Typische Vertreter von Verbindungen der Formel I, worin X für $-CH(OR_1)-$ und $R_1$ für Wasserstoff steht:

| Verb. Nr. | $R_2$ | $R_3$ | Physik. Konst. Smp. [°C] |
|---|---|---|---|
| 3.1 | $CH_3$ | H | |
| 3.2. | $C_2H_5$ | H | 163-167 |
| 3.3. | $C_3H_7$-iso | H | |
| 3.4. | $C_4H_9$-sek | H | |
| 3.5. | $CH_3$ | $CH_3$ | |
| 3.6. | $C_2H_5$ | $CH_3$ | 95-98 |
| 3.7. | $C_3H_7$-iso | $CH_3$ | 125-130 |
| 3.8. | $C_4H_9$-sek | $CH_3$ | |
| 3.9. | $CH_3$ | $C_2H_5$ | |
| 3.10 | $C_2H_5$ | $C_2H_5$ | 127-132 |
| 3.11 | $C_3H_7$-iso | $C_2H_5$ | |
| 3.12 | $C_4H_9$-sek | $C_2H_5$ | |
| 3.13 | $CH_3$ | $C_3H_7$-n | |
| 3.14 | $C_2H_5$ | $C_3H_7$-n | |
| 3.15 | $C_3H_7$-iso | $C_3H_7$-n | |
| 3.16 | $C_4H_9$-sek | $C_3H_7$-n | |
| 3.17 | $CH_3$ | $C_4H_9$-n | |
| 3.18 | $C_2H_5$ | $C_4H_9$-n | |
| 3.19 | $C_3H_7$-iso | $C_4H_9$-n | |
| 3.20 | $C_4H_9$-sek | $C_4H_9$-n | |
| 3.21 | $CH_3$ | $C_4H_9$-tert | |
| 3.22 | $C_2H_5$ | $C_4H_9$-tert | 110-115 |
| 3.23 | $C_3H_7$-iso | $C_4H_9$-tert | |
| 3.24 | $C_4H_9$-sek | $C_4H_9$-tert | |
| 3.25 | $CH_3$ | $C_3H_7$-iso | |
| 3.26 | $C_2H_5$ | $C_3H_7$-iso | |
| 3.27 | $C_3H_7$-iso | $C_3H_7$-iso | |
| 3.28 | $C_4H_9$-sek | $C_3H_7$-iso | |
| 3.29 | $CH_3$ | $C_4H_9$-sek | |
| 3.30 | $C_2H_5$ | $C_4H_9$-sek | |

Tabelle 3: (Fortsetzung)

| Verb. Nr. | $R_2$ | $R_3$ | Physik. Konst. Smp. [°C] |
|---|---|---|---|
| 3.31 | $C_3H_7$-iso | $C_4H_9$-sek | |
| 3.32 | $C_4H_9$-sek | $C_4H_9$-sek | |
| 3.33 | $CH_3$ | Phenyl | |
| 3.34 | $C_2H_5$ | Phenyl | 120-125 |
| 3.35 | $C_3H_7$-iso | Phenyl | |
| 3.36 | $C_4H_9$-sek | Phenyl | |
| 3.37 | $CH_3$ | 2-Tetrahydropyranyl | |
| 3.38 | $C_2H_5$ | 2-Tetrahydropyranyl | 110-115 |
| 3.39 | $C_3H_7$-iso | 2-Tetrahydropyranyl | |
| 3.40 | $C_4H_9$-sek | 2-Tetrahydropyranyl | |
| 3.41 | $CH_3$ | $C_5H_{11}$-n | |
| 3.42 | $C_2H_5$ | $C_6H_{13}$-n | |
| 3.43 | $C_3H_7$-iso | $C_5H_{11}$-n | |
| 3.44 | $C_4H_9$-sek | $C_6H_{13}$-n | |
| 3.45 | $CH_3$ | Benzyl | |
| 3.46 | $C_2H_5$ | Benzyl | |
| 3.47 | $C_3H_7$-iso | Benzyl | |
| 3.48 | $C_4H_9$-sek | Benzyl | |
| 3.49 | $CH_3$ | 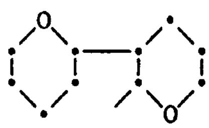 | |
| 3.50 | $C_2H_5$ | 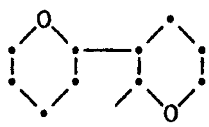 | |
| 3.51 | $C_3H_7$-iso | 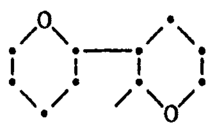 | |
| 3.52 | $C_4H_9$-sek | 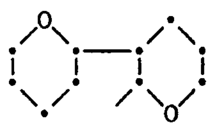 | |

<u>Tabelle 4</u>: Typische Vertreter von Verbindungen der Formel I, worin X für $-CH(OR_1)-$ und $R_1$ für eine OH-Schutzgruppe steht:

| Verb. Nr. | $R_2$ | $R_3$ | $R_1$ | Physik. Konst. Smp. [°C] |
|---|---|---|---|---|
| 4.1. | $CH_3$ | H | $COCH_3$ | |
| 4.2. | $C_2H_5$ | H | $COCH_3$ | |
| 4.3. | $C_3H_7$-iso | H | $COCH_3$ | |
| 4.4. | $C_4H_9$-sek | H | $COCH_3$ | |
| 4.5. | $CH_3$ | $CH_3$ | $COCH_3$ | |
| 4.6. | $C_2H_5$ | $CH_3$ | $COCH_3$ | |
| 4.7. | $C_3H_7$-iso | $CH_3$ | $COCH_3$ | 137-139 |
| 4.8. | $C_4H_9$-sek | $CH_3$ | $COCH_3$ | |
| 4.9. | $CH_3$ | $CH_3$ | $Si(CH_3)_2C(CH_3)_3$ | |
| 4.10 | $C_2H_5$ | $CH_3$ | $Si(CH_3)_2C(CH_3)_3$ | 96-100 |
| 4.11 | $C_3H_7$-iso | $CH_3$ | $Si(CH_3)_2C(CH_3)_3$ | |
| 4.12 | $C_4H_9$-sek | $CH_3$ | $Si(CH_3)_2C(CH_3)_3$ | |
| 4.13 | $CH_3$ | $C_2H_5$ | $Si(CH_3)_2C(CH_3)_3$ | |
| 4.14 | $C_2H_5$ | $C_2H_5$ | $Si(CH_3)_2C(CH_3)_3$ | 94-97 |
| 4.15 | $C_3H_7$-iso | $C_2H_5$ | $Si(CH_3)_2C(CH_3)_3$ | |
| 4.16 | $C_4H_9$-sek | $C_2H_5$ | $Si(CH_3)_2C(CH_3)_3$ | |
| 4.17 | $CH_3$ | H | $Si(CH_3)_2C(CH_3)_3$ | |
| 4.18 | $C_2H_5$ | H | $Si(CH_3)_2C(CH_3)_3$ | 132-137 |
| 4.19 | $C_3H_7$-iso | H | $Si(CH_3)_2C(CH_3)_3$ | |
| 4.20 | $C_4H_9$-sek | H | $Si(CH_3)_2C(CH_3)_3$ | |
| 4.21 | $CH_3$ | 2-Tetrahydropyranyl | $Si(CH_3)_2C(CH_3)_3$ | |
| 4.22 | $C_2H_5$ | 2-Tetrahydropyranyl | $Si(CH_3)_2C(CH_3)_3$ | 90-93 |
| 4.23 | $C_3H_7$-iso | 2-Tetrahydropyranyl | $Si(CH_3)_2C(CH_3)_3$ | |
| 4.24 | $C_4H_9$-sek | 2-Tetrahydropyranyl | $Si(CH_3)_2C(CH_3)_3$ | |
| 4.25 | $CH_3$ | 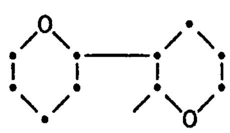 | $Si(CH_3)_2C(CH_3)_3$ | |

Tabelle 4: (Fortsetzung)

| Verb. Nr. | $R_2$ | $R_3$ | $R_1$ | Physik. Konst. Smp. [°C] |
|---|---|---|---|---|
| 4.26 | $C_2H_5$ | 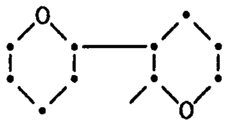 | $Si(CH_3)_2C(CH_3)_3$ | amorph |
| 4.27 | $C_3H_7$-iso | 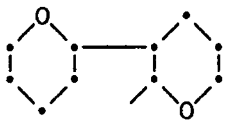 | $Si(CH_3)_2C(CH_3)_3$ | |
| 4.28 | $C_4H_9$-sek | 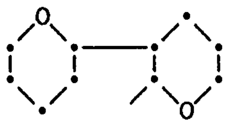 | $Si(CH_3)_2C(CH_3)_3$ | |
| 4.29 | $CH_3$ | $C(CH_3)_3$ | $Si(CH_3)_2C(CH_3)_3$ | |
| 4.30 | $C_2H_5$ | $C(CH_3)_3$ | $Si(CH_3)_2C(CH_3)_3$ | amorph |
| 4.31 | $C_3H_7$-iso | $C(CH_3)_3$ | $Si(CH_3)_2C(CH_3)_3$ | |
| 4.32 | $C_4H_9$-sek | $C(CH_3)_3$ | $Si(CH_3)_2C(CH_3)_3$ | |
| 4.33 | $CH_3$ | Phenyl | $Si(CH_3)_2C(CH_3)_3$ | |
| 4.34 | $C_2H_5$ | Phenyl | $Si(CH_3)_2C(CH_3)_3$ | amorph |
| 4.35 | $C_3H_7$-iso | Phenyl | $Si(CH_3)_2C(CH_3)_3$ | |
| 4.36 | $C_4H_9$-sek | Phenyl | $Si(CH_3)_2C(CH_3)_3$ | |

Tabelle 5: Typische Vertreter von Verbindungen der Formel I, worin X für die Gruppe -C(O)- steht:

| Verb. Nr. | $R_2$ | $R_3$ | Physik. Konst. Smp. [°C] |
|---|---|---|---|
| 5.1 | $CH_3$ | H | |
| 5.2. | $C_2H_5$ | H | |
| 5.3. | $C_3H_7$-iso | H | |
| 5.4. | $C_4H_9$-sek | H | |
| 5.5. | $CH_3$ | $CH_3$ | |
| 5.6. | $C_2H_5$ | $CH_3$ | amorph |
| 5.7. | $C_3H_7$-iso | $CH_3$ | |
| 5.8. | $C_4H_9$-sek | $CH_3$ | |
| 5.9. | $CH_3$ | $C_2H_5$ | |
| 5.10 | $C_2H_5$ | $C_2H_5$ | |
| 5.11 | $C_3H_7$-iso | $C_2H_5$ | |
| 5.12 | $C_4H_9$-sek | $C_2H_5$ | |
| 5.13 | $CH_3$ | $C_3H_7$-n | |
| 5.14 | $C_2H_5$ | $C_3H_7$-n | |
| 5.15 | $C_3H_7$-iso | $C_3H_7$-n | |
| 5.16 | $C_4H_9$-sek | $C_3H_7$-n | |
| 5.17 | $CH_3$ | $C_4H_9$-n | |
| 5.18 | $C_2H_5$ | $C_4H_9$-n | |
| 5.19 | $C_3H_7$-iso | $C_4H_9$-n | |
| 5.20 | $C_4H_9$-sek | $C_4H_9$-n | |
| 5.21 | $CH_3$ | $C_4H_9$-tert | |
| 5.22 | $C_2H_5$ | $C_4H_9$-tert | |
| 5.23 | $C_3H_7$-iso | $C_4H_9$-tert | |
| 5.24 | $C_4H_9$-sek | $C_4H_9$-tert | |
| 5.25 | $CH_3$ | $C_3H_7$-iso | |
| 5.26 | $C_2H_5$ | $C_3H_7$-iso | |
| 5.27 | $C_3H_7$-iso | $C_3H_7$-iso | |
| 5.28 | $C_4H_9$-sek | $C_3H_7$-iso | |
| 5.29 | $CH_3$ | $C_4H_9$-sek | |
| 5.30 | $C_2H_5$ | $C_4H_9$-sek | |

Tabelle 5: (Fortsetzung)

| Verb. Nr. | $R_2$ | $R_3$ | Physik. Konst. Smp. [°C] |
|---|---|---|---|
| 5.31 | $C_3H_7$-iso | $C_4H_9$-sek | |
| 5.32 | $C_4H_9$-sek | $C_4H_9$-sek | |
| 5.33 | $CH_3$ | Phenyl | |
| 5.34 | $C_2H_5$ | Phenyl | amorph |
| 5.35 | $C_3H_7$-iso | Phenyl | |
| 5.36 | $C_4H_9$-sek | Phenyl | |
| 5.37 | $CH_3$ | 2-Tetrahydropyranyl | |
| 5.38 | $C_2H_5$ | 2-Tetrahydropyranyl | |
| 5.39 | $C_3H_7$-iso | 2-Tetrahydropyranyl | |
| 5.40 | $C_4H_9$-sek | 2-Tetrahydropyranyl | |
| 5.41 | $CH_3$ | $C_5H_{11}$-n | |
| 5.42 | $C_2H_5$ | $C_6H_{13}$-n | |
| 5.43 | $C_3H_7$-iso | $C_5H_{11}$-n | |
| 5.44 | $C_4H_9$-sek | $C_6H_{13}$-n | |
| 5.45 | $CH_3$ | Benzyl | |
| 5.46 | $C_2H_5$ | Benzyl | |
| 5.47 | $C_3H_7$-iso | Benzyl | |
| 5.48 | $C_4H_9$-sek | Benzyl | |
| 5.49 | $CH_3$ | 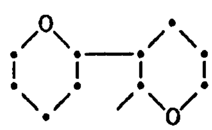 | |
| 5.50 | $C_2H_5$ | 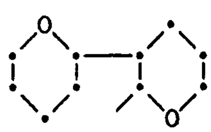 | |
| 5.51 | $C_3H_7$-iso | 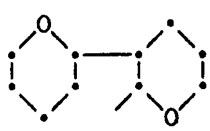 | |
| 5.52 | $C_4H_9$-sek | 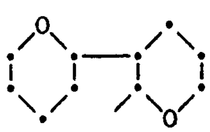 | |

Tabelle 5: (Fortsetzung)

| Verb. Nr. | $R_2$ | $R_3$ | Physik. Konst. Smp. [°C] |
|---|---|---|---|
| 5.53 | $CH_3$ | $C(CH_3)_3$ | |
| 5.54 | $C_2H_5$ | $C(CH_3)_3$ | amorph |
| 5.55 | $C_3H_7$-iso | $C(CH_3)_3$ | |
| 5.56 | $C_4H_9$-sek | $C(CH_3)_3$ | |

Tabelle 6: Typische Vertreter von Verbindungen der Formel I, worin X für die Gruppe -C(=N-OR) steht und R Wasserstoff bedeutet.

| Verb. Nr. | $R_2$ | $R_3$ | Physik. Konst. Smp. [°C] |
|---|---|---|---|
| 6.1 | $CH_3$ | H | |
| 6.2. | $C_2H_5$ | H | 184-186 |
| 6.3. | $C_3H_7$-iso | H | |
| 6.4. | $C_4H_9$-sek | H | |
| 6.5. | $CH_3$ | $CH_3$ | |
| 6.6. | $C_2H_5$ | $CH_3$ | 143-148 |
| 6.7. | $C_3H_7$-iso | $CH_3$ | |
| 6.8. | $C_4H_9$-sek | $CH_3$ | |
| 6.9. | $CH_3$ | $C_2H_5$ | |
| 6.10 | $C_2H_5$ | $C_2H_5$ | |
| 6.11 | $C_3H_7$-iso | $C_2H_5$ | |
| 6.12 | $C_4H_9$-sek | $C_2H_5$ | |
| 6.13 | $CH_3$ | $C_3H_7$-n | |
| 6.14 | $C_2H_5$ | $C_3H_7$-n | |
| 6.15 | $C_3H_7$-iso | $C_3H_7$-n | |
| 6.16 | $C_4H_9$-sek | $C_3H_7$-n | |
| 6.17 | $CH_3$ | $C_4H_9$-n | |
| 6.18 | $C_2H_5$ | $C_4H_9$-n | |
| 6.19 | $C_3H_7$-iso | $C_4H_9$-n | |
| 6.20 | $C_4H_9$-sek | $C_4H_9$-n | |

Tabelle 6: (Fortsetzung)

| Verb. Nr. | $R_2$ | $R_3$ | Physik. Konst. Smp. [°C] |
|---|---|---|---|
| 6.21 | $CH_3$ | $C_4H_9$-tert | |
| 6.22 | $C_2H_5$ | $C_4H_9$-tert | |
| 6.23 | $C_3H_7$-iso | $C_4H_9$-tert | |
| 6.24 | $C_4H_9$-sek | $C_4H_9$-tert | |
| 6.25 | $CH_3$ | $C_3H_7$-iso | |
| 6.26 | $C_2H_5$ | $C_3H_7$-iso | |
| 6.27 | $C_3H_7$-iso | $C_3H_7$-iso | |
| 6.28 | $C_4H_9$-sek | $C_3H_7$-iso | |
| 6.29 | $CH_3$ | $C_4H_9$-sek | |
| 6.30 | $C_2H_5$ | $C_4H_9$-sek | |
| 6.31 | $C_3H_7$-iso | $C_4H_9$-sek | |
| 6.32 | $C_4H_9$-sek | $C_4H_9$-sek | |
| 6.33 | $CH_3$ | Phenyl | |
| 6.34 | $C_2H_5$ | Phenyl | 131-133 |
| 6.35 | $C_3H_7$-iso | Phenyl | |
| 6.36 | $C_4H_9$-sek | Phenyl | |
| 6.37 | $CH_3$ | 2-Tetrahydropyranyl | |
| 6.38 | $C_2H_5$ | 2-Tetrahydropyranyl | |
| 6.39 | $C_3H_7$-iso | 2-Tetrahydropyranyl | |
| 6.40 | $C_4H_9$-sek | 2-Tetrahydropyranyl | |
| 6.41 | $CH_3$ | $C_5H_{11}$-n | |
| 6.42 | $C_2H_5$ | $C_6H_{13}$-n | |
| 6.43 | $C_3H_7$-iso | $C_5H_{11}$-n | |
| 6.44 | $C_4H_9$-sek | $C_6H_{13}$-n | |
| 6.45 | $CH_3$ | Benzyl | |
| 6.46 | $C_2H_5$ | Benzyl | |
| 6.47 | $C_3H_7$-iso | Benzyl | |
| 6.48 | $C_4H_9$-sek | Benzyl | |
| 6.49 | $CH_3$ | 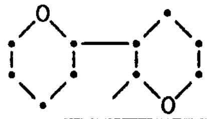 | |

Tabelle 6: (Fortsetzung)

| Verb. Nr. | $R_2$ | $R_3$ | Physik. Konst. Smp. [°C] |
|---|---|---|---|
| 6.50 | $C_2H_5$ | 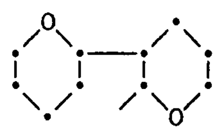 | |
| 6.51 | $C_3H_7$-iso | 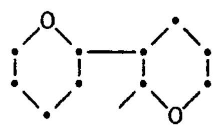 | |
| 6.52 | $C_4H_9$-sek | 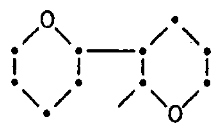 | |
| 6.53 | $CH_3$ | $C(CH_3)_3$ | |
| 6.54 | $C_2H_5$ | $C(CH_3)_3$ | 153-157 |
| 6.55 | $C_3H_7$-iso | $C(CH_3)_3$ | |
| 6.56 | $C_4H_9$-sek | $C(CH_3)_3$ | |

Formulierungsbeispiele für den Wirkstoff der Formel I

(% = Gewichtsprozent)

| Spritzpulver | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Emulsions-Konzentrat | |
|---|---|
| Wirkstoff aus den Tabellen | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| Extruder Granulat | |
|---|---|
| Wirkstoff aus den Tabellen | 10 % |
| Na-Ligninsulfonat | 2 % |
| Carboximethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| Tabletten bzw. Boli | | |
|---|---|---|
| I | Ein Wirkstoff aus den Tabellen | 33,0 % |
| | Methylcellulose | 0,80 % |
| | Kieselsäure hochdispers | 0,80 % |
| | Maisstärke | 8,40 % |

Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb (Maschenweite 12 M) granulieren und dann trocknen.

| | | |
|---|---|---|
| II | Milchzucker krist. | 22,50 % |
| | Maisstärke | 17,00 % |
| | mikrokrist. Cellulose | 16,50 % |
| | Magnesiumstearat | 1,00 % |
| Alle 4 Hilfsstoffe gut mischen | | |

Phasen I und II mischen und zu Tabletten oder Boli verpressen.

Injektabiles

A. Oeliges Vehikel (langsame Freisetzung)

| | |
|---|---|
| Ein Wirkstoff aus den Tabellen | 0,1-1,0 g |
| Erdnussöl | ad 100 ml |
| Ein Wirkstoff aus den Tabellen | 0,1-1,0 g |
| Sesamöl | ad 100 ml |

Herstellung: Der Wirkstoff wird in einem Teil des Oels unter Rühren und gegebenenfalls leichtem Erwärmen gelöst, nach Abkühlung auf das Sollvolumen aufgefüllt und durch ein geeignetes Membranfilter mit 0,22 µm sterilfiltriert.

B. Wassermischbares Lösungsmittel (mittlere Freisetzungsgeschwindigkeit)

| | |
|---|---|
| Ein Wirkstoff aus den Tabellen | 0,1-1,0 g |
| 4-Hydroxymethyl-1,3-dioxolan (Glycerol Formal) | 40 g |
| 1,2-Propandiol | ad 100 ml |
| Ein Wirkstoff aus den Tabellen | 0,1-1,0 g |
| Glycerindimethylketal | 40 g |
| 1,2-Propandiol | ad 100 ml |

Herstellung: Der Wirkstoff wird in einem Teil des Lösungsmittels unter Rühren gelöst, auf das Sollvolumen aufgefüllt und durch ein geeignetes Membranfilter mit 0.22 µm sterilfiltriert.

C. Wässriges Solubilisat (rasche Freisetzung)

| | |
|---|---|
| Ein Wirkstoff aus den Tabellen | 0,1-1,0 g |
| Polyäthoxyliertes Ricinusöl (40 Aethylenoxideinheiten)* | 10 g |
| 1,2-Propandiol | 20 g |
| Benzylalkohol | 1 g |
| Aqua ad injekt. | ad 100 ml |

* Im Handel erhältlich unter der Bezeichnung CREMOPHOR® EL (BASF AG);

| | |
|---|---|
| Ein Wirkstoff aus den Tabellen | 0,1-1,0 g |
| Polyäthoxyliertes Sorbitanmonooleat (20 Aethylenoxideinheiten)** | 8 g |
| 4-Hydroxymethyl-1,3-dioxolan (Glycerol Formal) | 20 g |
| Benzylalkohol | 1 g |
| Aqua ad injekt. | ad 100 ml |

** Im Handel erhältlich unter der Bezeichnung TWEEN® 80 (ICI);

Herstellung: Der Wirkstoff wird in den Lösungsmitteln und dem Tensid gelöst und mit Wasser auf das Sollvolumen aufgefüllt. Sterilfiltration durch geeignetes Membranfilter mit 0,22 um Porendurchmesser.

Die wässrigen Systeme können bevorzugterweise auch für die orale und/oder intraruminale Applikation eingesetzt werden.

Biologische Beispiele

**B-1. Wirkung gegen $L_1$-Larven von Lucilia sericata**

1 ml einer wässrigen Suspension der zu prüfenden Aktivsubstanz werden so mit 3 ml eines speziellen

Larvenzuchtmediums bei ca. 50°C vermischt, dass ein Homogenisat von wahlweise 250 ppm oder 125 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Verbindungen der Formel I wie z.B. Nr. 3.2, 3.6, 3.7, 3.10, 3.34 und 3.38 erzielten mit 125 ppm eine Wirkung von 100 %.

B-2. Akarizide Wirkung gegen Boophilus microplus (Biarra-Stamm)

Auf einer PVC-Platte wird waagrecht ein Klebstreifen so befestigt, das darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injektionsnadel 1 µl einer Flüssigkeit injiziert, die eine 1:1-Mischung von Polyethylenglykol und Aceton darstellt und in der eine bestimmte Wirkstoffmenge von 1,0 µg pro Zecke gelöst ist. Kontrolltiere erhalten eine wirkstofffreie Injektion. Nach der Behandlung werden die Tiere unter Normalbedingungen in einem Insektarium bei ca. 28°C und 80 % relativer Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zeckenweibchen (= 90 %) Eier ablegen, die nicht schlupffähig sind.

Verbindungen der Formeln I, wie z.B. Nr. 3.2, 3.6, 3.7, 3.10, 3.34 und 3.38 erzielen eine $IR_{90}$ von 1,0 µg.

B-3. Versuch an mit Nematoden (Haemonchus contortus und Trichostrongylus colubriformis) infizierten Schafen

Der Wirkstoff wird als Suspension formuliert mit einer Magensonde oder durch Injektion in den Pansen eines Schafes gegeben, das mit Haemonchus contortus und Trychostrongylus colubriformis künstlich infiziert worden ist. Pro Dosis werden 1 bis 3 Tiere verwendet. Jedes Schaf wird nur einmal mit einer einzigen Dosis behandelt, und zwar wahlweise mit 1 mg oder 0,5 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit Verbindungen der Formeln I, wie z.B. Nr. 3.2, 3.6, 3.7, 3.10, 3.34 und 3.38 bei 1 mg/kg behandelt wurden, zeigten im Vergleich zu unbehandelten, aber infizierten Vergleichsgruppen keinen Nematodenbefall (= komplette Reduktion der Wurmeier im Kot).

B-4. Larvizidwirkung gegen Aedes aegypti

Auf die Oberfläche von 150 ml Wasser, das sich in einem Behälter befindet, wird soviel einer 0,1 %igen acetonischen Lösung des Wirkstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Verbindungen der Formeln I, wie z.B. Nr. 3.2, 3.6, 3.7, 3.10, 3.34 und 3.38 bewirkten in diesem Test bei einer Konzentration von 1,6 ppm bereits nach einem Tag eine vollständige Abtötung sämtlicher Larven.

B-5. Milbizide Wirkung gegen Dermanyssus gallinae

2 bis 3 ml einer Testlösung (100, 10, 1 und 0,1 ppm Aktivsubstanz) werden in einen nach oben offenen Glasbehälter gegeben und ca. 200 Milben in unterschiedlichen Entwicklungsstadien in diesen Behälter eingesetzt. Der Glasbehälter wird mit einam Wattebausch verschlossen und 10 Minuten gleichmässig, bis zur vollständigen Benetzung der Milben geschüttelt. Danach wird der Behälter umgekehrt hingestellt, bis die überschüssige Testlösung von der Watte aufgesogen ist. Der Behälter wird erneut gekehrt und die behandelten Milben zur Evaluierung der Wirksamkeit der Testsubstanzen drei Tage unter Laborbedingungen beobachtet, wobei die Mortalität als Massstab für Wirksamkeit herangezogen wird.

Verbindungen aus den Herstellungsbeispielen wie z.B. die Verb. Nr. 3.2, 3.6, 3.7, 3.10 und 3.38 zeigen bei 100 ppm eine Wirkung von 100 %.

**Patentansprüche**

**Patentansprüche für folgenden Vertragsstaaten : AT, BE, CH, DE, FR, GB, GR, IT, LI, LU, NL, SE**

**1.** Verbindungen der Formel I

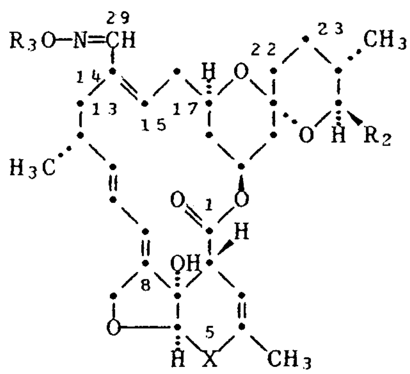

(I)

in welcher

X eine der Gruppen -CH($OR_1$)-, -C(O)- oder -C(=N-OR)- repräsentiert;

$R_1$ Wasserstoff oder eine OH-Schutzgruppe bedeutet;

R für Wasserstoff, eine OH-Schutzgruppe, eine Alkyl-, Cycloalkylgruppe steht; wobei unter OH-Schutzgruppe für die Substituenten R und $R_1$ die Reste $R_4$-C(O)- oder-Si($R_5$)($R_6$)($R_7$) zu verstehen sind, wobei $R_4$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxi, $C_1$-$C_3$-Haloalkoxi, Cyano und/oder Nitro substituierte Gruppe aus der Reihe Phenyl und Benzyl steht; $R_5$, $R_6$, $R_7$ unabhängig voneinander für $C_1$-$C_4$-Alkyl, Benzyl oder Phenyl stehen;

$R_2$ für Methyl, Ethyl, Isopropyl, sek.Butyl oder für die Gruppe

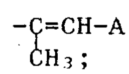

steht, worin A Methyl, Ethyl oder Isopropyl bedeutet, und

$R_3$ für Wasserstoff, $C_1$-$C_6$-Alkyl, $C_2$-$C_6$-Alkenyl, einen unsubstituierten oder einen ein- oder mehrfach durch Halogen, $C_1$-$C_3$-Alkyl, $C_1$-$C_3$-Alkoxy, $C_1$-$C_3$-Alkylthio, $C_1$-$C_3$-Haloalkyl, Nitro oder Cyano substituierten Rest aus der Reihe Phenyl und Benzyl steht oder die Gruppe U

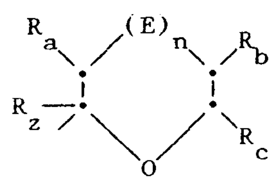

(U)

repräsentiert, worin n für eine der Zahlen 0, 1, 2, 3, 4 oder 5 steht, E für Sauerstoff oder -CH($R_d$)- und $R_z$ für Wasserstoff steht, $R_a$ Wasserstoff, Halogen, $C_1$-$C_3$-Alkyl, $C_1$-$C_3$-Alkoxy oder 2-Tetrahydropyranyl bedeutet und $R_b$, $R_c$ und $R_d$ unabhängig voneinander für Wasserstoff, Halogen, $C_1$-$C_3$-Alkyl oder $C_1$-$C_3$-Alkoxy stehen, wobei die Gruppe U auch in ungesättigter Form vorliegen kann.

**2.** Verbindungen der Formel I gemäss Anspruch 1, worin

$R_2$ für Methyl, Ethyl, Isopropyl oder sek.Butyl steht.

**3.** Verbindung der Formel I gemäss Anspruch 2, worin

X die Gruppe -CH($OR_1$)- repräsentiert;

$R_1$ Wasserstoff oder eine OH-Schutzgruppe bedeutet; wobei unter OH-Schutzgruppe die Reste $R_4$-C(O)- oder -Si($R_5$)($R_6$)($R_7$) zu verstehen sind, wobei $R_4$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxi, $C_1$-$C_3$-Haloalkoxi, Cyano und/oder Nitro substituierte Gruppe aus der Reihe Phenyl und Benzyl steht; $R_5$, $R_6$, $R_7$ unabhängig voneinander für $C_1$-$C_4$-Alkyl, Benzyl oder Phenyl stehen.

**4.** Verbindungen der Formel I nach Anspruch 2, worin X eine der Gruppen -CH($OR_1$)-, -C(O)- oder -C(=N-OH)- repräsentiert, wobei $R_1$ Wasserstoff, $R_4$-C(O)- oder -Si ($R_5$) ($R_6$) ($R_7$) bedeutet, wobei $R_4$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxi, $C_1$-$C_3$-Haloalkoxi, Cyano und/oder Nitro substituierte Gruppe aus der Reihe Phenyl und Benzyl steht; $R_5$, $R_6$, $R_7$ unabhängig voneinander für $C_1$-$C_4$-Alkyl, Benzyl oder Phenyl stehen; $R_2$ für Methyl, Ethyl, Isopropyl oder sek.-Butyl steht; und $R_3$ für Wasserstoff, $C_1$-$C_4$-Alkyl, einen unsubstituierten oder einen ein- bis dreifach durch Halogen, $C_1$-$C_2$-Alkyl, $C_1$-$C_2$-Alkoxy, $C_1$-$C_2$-Alkylthio, $C_1$-$C_2$-Haloalkyl, Nitro oder Cyano substituierten Rest aus der Reihe Phenyl und Benzyl steht oder die Gruppe U

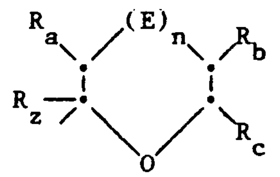

(U)

repräsentiert, worin n für eine der Zahlen 0 oder 1 steht, E für sauerstoff oder $CH_2$ und $R_z$ für Wasserstoff steht, $R_a$ Wasserstoff, Fluor, Chlor, Brom, Methyl oder 2-Tetrahydropyranyl bedeutet und $R_b$ und $R_c$ unabhängig voneinander für Wasserstoff, Halogen oder Methyl stehen.

**5.** Verbindungen der Formel I nach Anspruch 1, worin X die Gruppe -CH($OR_1$)-repräsentiert, wobei $R_1$ Wasserstoff, $R_4$-C(O)- oder -Si($R_5$)($R_6$)($R_7$) bedeutet, wobei $R_4$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxi, $C_1$-$C_3$-Haloalkoxi, Cyano und/oder Nitro substituierte Gruppe aus der Reihe Phenyl und Benzyl steht; $R_5$, $R_6$, $R_7$ unabhängig voneinander für $C_1$-$C_4$-Alkyl, Benzyl oder Phenyl stehen; $R_2$ für Methyl, Ethyl, Isopropyl oder sek.-Butyl steht; und $R_3$ für Wasserstoff, $C_1$-$C_4$-Alkyl, einen unsubstituierten oder einen ein- bis dreifach durch Halogen, $C_1$-$C_2$-Alkyl, $C_1$-$C_2$-Alkoxy, $C_1$-$C_2$-Alkylthio, $C_1$-$C_2$-Haloalkyl, Nitro oder Cyano substituierten Rest aus der Reihe Phenyl und Benzyl steht oder die Gruppe U

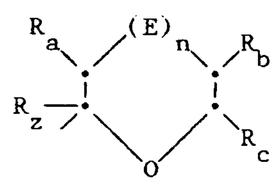

(U)

repräsentiert, worin n für eine der Zahlen 0 oder 1 steht, E für Sauerstoff oder $CH_2$ und $R_z$ für Wasserstoff steht, $R_a$ Wasserstoff, Fluor, Chlor, Brom, Methyl oder 2-Tetrahydropyranyl bedeutet und $R_b$ und $R_c$ unabhängig voneinander für Wasserstoff, Halogen oder Methyl stehen.

**6.** Verbindungen der Formel I nach Anspruch 3, worin

$R_3$ für Wasserstoff, $C_1$-$C_6$-Alkyl, $C_2$-$C_6$-Alkenyl, einen unsubstituierten oder einen ein- oder mehrfach durch Halogen, $C_1$-$C_3$-Alkyl, $C_1$-$C_3$-Alkoxy, $C_1$-$C_3$-Alkylthio, $C_1$-$C_3$-Haloalkyl, Nitro oder Cyano substituierten Rest aus der Reihe Phenyl und Benzyl steht oder die Gruppe U für eine der Gruppen

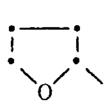 , 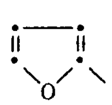 , 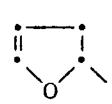 , 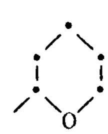 , 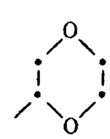 ,

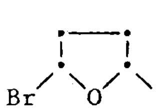 , 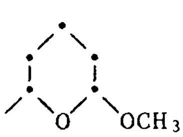 , 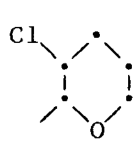 , 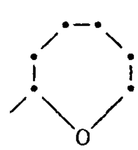 oder 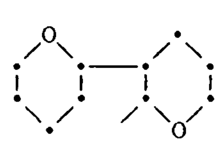

steht.

**7.** Verbindungen der Formel I nach Anspruch 6, worin $R_1$ Wasserstoff bedeutet; und $R_3$ Wasserstoff, $C_1$-$C_4$-Alkyl, Phenyl, Benzyl, oder eine der Gruppen

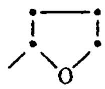 , 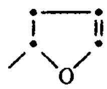 , 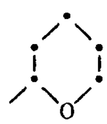 oder 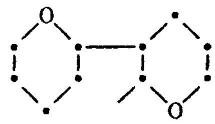

repräsentiert.

**8.** Verbindungen der Formel I nach einem der Ansprüche 1 bis 7, worin $R_2$ für Methyl steht.

**9.** Verbindungen der Formel I nach einem der Ansprüche 1 bis 7, worin $R_2$ für Ethyl steht.

**10.** Eine Verbindung der Formel I nach Anspruch 1, ausgewählt aus der Reihe:
5-Acetoxi-29-methoximino-milbemycin D,
5-(Dimethyl-tert.-butyl-silyloxi)-29-hydroximino-milbemycin $A_4$,
29-Hydroximino-milbemycin $A_4$,
29-Methoximino-milbemycin $A_4$,
29-Phenoximino-milbemycin $A_4$,
29-(Tetrahydropyran-2-yl)-oximino-milbemycin $A_4$
5,29-bis-Hydroximino-milbemycin $A_4$.

**11.** Verfahren zur Herstellung einer Verbindung der Formel I nach Anspruch 1, dadurch gekennzeichnet, dass man ein 29-Oxo-Milbemycin der Formel II

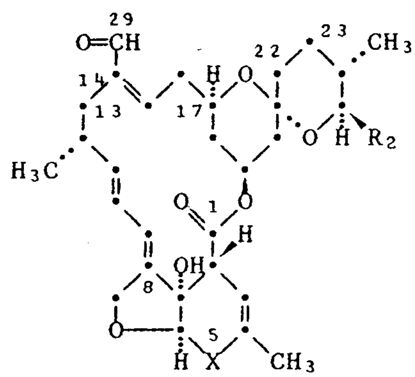

(II)

mit einem primären Oxamin der Formel III

$H_2N$-$OR_3$ (III)

oder einem seiner Salze umsetzt, und sofern gewünscht, die resultierende Verbindung der Formel I, sofern $R_3$ für Wasserstoff steht mit einem Halogenid der Formel IV

$R_3'$-Hal (IV)

weiterreagieren lässt, wobei $R_2$ für Methyl, Ethyl, Isopropyl oder sek.Butyl oder für die Gruppe

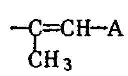

steht, worin A Methyl, Ethyl oder Isopropyl bedeutet und der Substituent $R_3$ die unter Formel I

angegebenen Bedeutungen hat, X für -C(OR$_1$) steht, wobei R$_1$ Wasserstoff oder eine der in Anspruch 1 genannten OH-Schutzgruppen bedeutet und Hal für Halogen steht, und $R_3'$ alle Bedeutungen von R$_3$, mit Ausnahme von Wasserstoff hat, wobei man in der Regel von Verbindungen der Formel II ausgeht, die entweder eine freie 5 Hydroxigruppe enthalten und, sofern gewünscht, die resultierenden Produkte am 5-O-Atom nachacyliert oder nachsilyliert oder bereits von einer in 5-Position silylierten oder acylierten Verbindung der Formel II ausgeht und die Schutzgruppe, sofern gewünscht, abspaltet; oder sofern Verbindungen der Formel I, worin X für die Gruppe -C(O)-steht, das Herstellungsziel sind, von Verbindungen der Formel II ausgeht, die eine freie 5-Hydroxygruppe enthalten und diese mit einem zur Oxidation geeigneten Reagenz behandelt; oder aber, sofern Verbindungen der Formel I, worin X für die Gruppe -C(=N-OR)- steht, wobei R die unter Formel I angegebene Bedeutung hat, gewünscht sind, von einer Verbindung der Formel I ausgeht, in der X für -C(O)- steht und diese mit Hydroxylamin oder einem seiner Salze umsetzt und gewünschtenfalls anschliessend den Substituenten R einführt oder aber die Umsetzung mit einer Verbindung der Formel $NH_2$-OR oder einem Salz davon vornimmt.

**12.** Verfahren zur Herstellung einer Verbindung der Formel I gemäss Anspruch 2, dadurch gekennzeichnet, dass man ein 29-Oxo-Milbemycin der Formel II

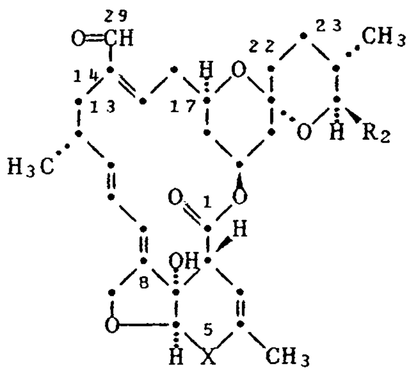

(II)

mit einem primären Oxamin der Formel III

$H_2N-OR_3$ (III)

oder einem seiner Salze umsetzt, und sofern gewünscht, die resultierende Verbindung der Formel I, sofern R$_3$ für Wasserstoff steht mit einem Halogenid der Formel IV

$R_3'$-Hal (IV)

weiterreagieren lässt, wobei R$_2$ für Methyl, Ethyl, Isopropyl oder sek.Butyl steht und der Substituent R$_3$ die unter Formel I angegebenen Bedeutungen hat, X für -C(OR$_1$)- steht, wobei R$_1$ Wasserstoff oder eine in Anspruch 2 angegebene OH-Schutzgruppe bedeutet und Hal für Halogen steht, und $R_3'$ alle Bedeutungen von R$_3$, mit Ausnahme von Wasserstoff hat, wobei man in der Regel von Verbindungen der Formel II ausgeht, die entweder eine freie 5 Hydroxigruppe enthalten und, sofern gewünscht, die resultierenden Produkte am 5-O-Atom nachacyliert oder nachsilyliert oder bereits von einer in 5-Position silylierten oder acylierten Verbindung der Formel II ausgeht und die Schutzgruppe, sofern gewünscht, abspaltet; oder sofern Verbindungen der Formel I, worin X für die Gruppe -C(O)- steht, das Herstellungsziel sind, von Verbindungen der Formel II ausgeht, die eine freie 5-Hydroxygruppe enthalten und diese mit einem zur Oxidation geeigneten Reagenz behandelt; oder aber, sofern Verbindungen der Formel I, worin X für die Gruppe -C(=N-OR)- steht, wobei R die unter Formel I angegebene Bedeutung hat, gewünscht sind, von einer Verbindung der Formel I ausgeht, in der X für -C(O)- steht und diese mit Hydroxylamin oder einem seiner Salze umsetzt und gewünschtenfalls anschliessend den Substituenten R einführt oder aber die Umsetzung mit einer Verbindung der Formel $NH_2$-OR oder einem Salz davon vornimmt.

**13.** Verfahren zur Herstellung einer Verbindung der Formel I gemäss Anspruch 3, dadurch gekennzeichnet, dass man ein 29-Oxo-Milbemycin der Formel II

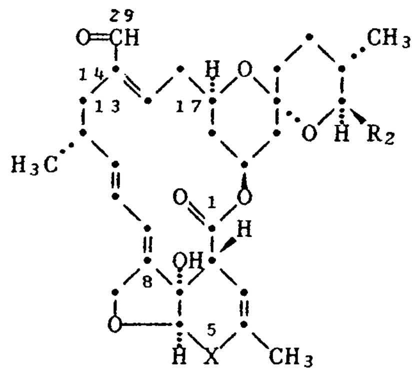

(II)

mit einem primären Oxamin der Formel III

$H_2N\text{-}OR_3$ (III)

oder einem seiner Salze umsetzt, und sofern gewünscht, die resultierende Verbindung der Formel I, sofern $R_3$ für Wasserstoff steht mit einem Halogenid der Formel IV

$R_3'$ -Hal (IV)

weiterreagieren lässt, wobei $R_2$ für Methyl, Ethyl, Isopropyl oder sek.Butyl steht und die Substituenten $R_1$ und $R_3$ die unter Formel I angegebenen Bedeutungen haben, Hal für Halogen steht, und $R_3'$ alle Bedeutungen von $R_3$, mit Ausnahme von Wasserstoff hat, wobei man von Verbindungen der Formel II ausgeht, die entweder eine freie 5 Hydroxigruppe enthalten und, sofern gewünscht, die resultierenden Produkte am 5-O-Atom nachacyliert oder nachsyliliert oder bereits von einer in 5-Position sylilierten oder acylierten Verbindung der Formel II ausgeht und die Schutzgruppe, sofern gewünscht, abspaltet.

**14.** Mittel zur Bekämpfung von Ekto- und Endoparasiten am Nutztier oder zur Bekämpfung von Schadinsekten, dadurch gekennzeichnet, dass es neben üblichen Trägerstoffen und Verteilungsmitteln eine Verbindung der Formel I nach Anspruch 1 enthält.

**15.** Mittel zur Bekämpfung von Ekto- und Endoparasiten am Nutztier oder zur Bekämpfung von Schadinsekten gemäss Anspruch 14, dadurch gekennzeichnet, dass es neben üblichen Trägerstoffen und Verteilungsmitteln eine Verbindung der Formel I nach Anspruch 2 enthält.

**16.** Mittel zur Bekämpfung von Ekto- und Endoparasiten am Nutztier oder zur Bekämpfung von Schadinsekten gemäss Anspruch 14, dadurch gekennzeichnet, dass es neben den üblichen Trägerstoffen und Vertilgungsmitteln eine Verbindung der Formel I nach Anspruch 3 enthält.

**17.** Mittel nach Anspruch 14, dadurch gekennzeichnet, dass es als Wirkstoff eine Verbindung der Formel I gemäss einem der Ansprüche 4 bis 10 enthält.

**18.** Verbindung der Formel I nach Ansprüche 1 bis 10 zur Verwendung in einem Verfahren zur Bekämpfung von Parasiten an Tieren.

**19.** Verfahren zur Bekämpfung von pflanzenschädigenden Parasiten, dadurch gekennzeichnet, dass man eine parasitizid bzw. insektizid wirksame Menge einer Verbindung der Formel I nach einem der Ansprüche 1 bis 10 auf den Parasiten oder seinen Lebensraum appliziert.

**20.** Verwendung einer Verbindung der Formel I gemäss Anspruch 1 zur Herstellung eines Mittels zur Bekämpfung von Ekto- und Endoparasiten am Nutztier oder von Schadinsekten in allen Entwicklungsstadien.

**21.** Verwendung nach Anspruch 20 einer Verbindung der Formel I gemäss einem der Ansprüche 4 bis 10 zur Herstellung eines Mittels Bekämpfung von Ekto- und Endoparasiten am Nutztier oder von Schadinsekten in allen Entwicklungsstadien.

**Patentansprüche für folgenden Vertragsstaat : ES**

**1.** Mittel zur Bekämpfung von Ekto- und Endoparasiten am Nutztier oder zur Bekämpfung von Schadinsekten, dadurch gekennzeichnet, dass es neben üblichen Trägerstoffen und Verteilungsmitteln eine Verbindung der Formel I enthält

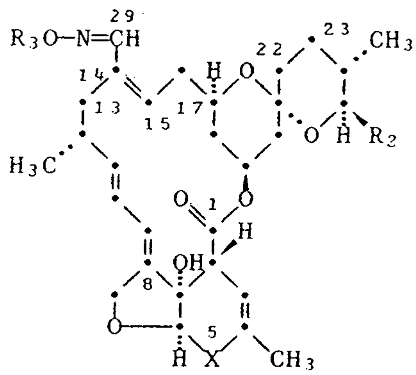

(I) ,

in welcher

X eine der Gruppen -CH($OR_1$)-, -C(O)- oder -C(=N-OR)- repräsentiert;

$R_1$ Wasserstoff oder eine OH-Schutzgruppe bedeutet;

R für Wasserstoff, eine OH-Schutzgruppe, eine Alkyl-, Cycloalkylgruppe steht; wobei unter OH-Schutzgruppe für die Substituenten R und $R_1$ die Reste $R_4$-(CO)- oder-Si($R_5$)($R_6$)($R_7$) zu verstehen sind, wobei $R_4$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxi, $C_1$-$C_3$-Haloalkoxi, Cyano und/oder Nitro substituierte Gruppe aus der Reihe Phenyl und Benzyl steht; $R_5$, $R_6$, $R_7$ unabhängig voneinander für $C_1$-$C_4$-Alkyl, Benzyl oder Phenyl stehen;

$R_2$ für Methyl, Ethyl, Isopropyl, sek.Butyl oder für die Gruppe

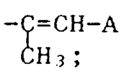

steht, worin A Methyl, Ethyl oder Isopropyl bedeutet, und

$R_3$ für Wasserstoff, $C_1$-$C_6$-Alkyl, $C_2$-$C_6$-Alkenyl, einen unsubstituierten oder einen ein- oder mehrfach durch Halogen, $C_1$-$C_3$-Alkyl, $C_1$-$C_3$-Alkoxy, $C_1$-$C_3$-Alkylthio, $C_1$-$C_3$-Haloalkyl, Nitro oder Cyano substituierten Rest aus der Reihe Phenyl und Benzyl steht oder die Gruppe U

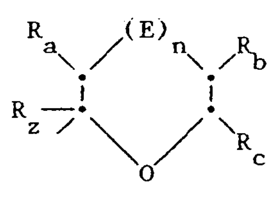

(U)

repräsentiert, worin n für eine der Zahlen 0, 1, 2, 3, 4 oder 5 steht, E für Sauerstoff oder -CH($R_d$)- und $R_z$ für Wasserstoff steht, $R_a$ Wasserstoff, Halogen, $C_1$-$C_3$-Alkyl, $C_1$-$C_3$-Alkoxy oder 2-Tetrahydropyranyl bedeutet und $R_b$, $R_c$ und $R_d$ unabhängig voneinander für Wasserstoff, Halogen, $C_1$-$C_3$-Alkyl oder $C_1$-$C_3$-Alkoxy stehen, wobei die Gruppe U auch in ungesättigter Form vorliegen kann.

**2.** Mittel nach Anspruch 1, dadurch gekennzeichnet, dass es als Aktivsubstanz eine Verbindung der Formel I enthält, worin $R_2$ für Methyl, Ethyl, Isopropyl oder sek.Butyl steht und die übrigen Substituenten wie in Anspruch 1 definiert sind.

**3.** Mittel nach Anspruch 2, dadurch gekennzeichnet, dass es als Aktivsubstanz eine Verbindung der Formel I enthält, worin $R_2$ für Methyl, Ethyl, Isopropyl oder sek.Butyl steht;

X die Gruppe -CH($OR_1$)- repräsentiert;

$R_1$ Wasserstoff oder eine OH-Schutzgruppe bedeutet; wobei unter OH-Schutzgruppe die Reste $R_4$-C(O)- oder -Si($R_5$)($R_6$)($R_7$) zu verstehen sind, wobei $R_4$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxi, $C_1$-$C_3$-Haloalkoxi, Cyano und/oder Nitro substituierte Gruppe aus der Reihe Phenyl und Benzyl steht; $R_5$, $R_6$, $R_7$ unabhängig voneinander für $C_1$-$C_4$-Alkyl, Benzyl oder Phenyl stehen und die übrigen Substituenten wie in Anspruch 1 definiert sind.

**4.** Mittel nach Anspruch 2, dadurch gekennzeichnet, dass es als Aktivsubstanz eine Verbindung der Formel I enthält, worin X eine der Gruppen -CH($OR_1$)-, -C(O)- oder -C( = N-OH)- repräsentiert, wobei $R_1$ Wasserstoff, $R_4$-C(O)- oder -Si($R_5$)($R_6$)($R_7$) bedeutet, wobei $R_4$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxi, $C_1$-$C_3$-Haloalkoxi, Cyano und/oder Nitro substituierte Gruppe aus der Reihe Phenyl und Benzyl steht; $R_5$, $R_6$, $R_7$ unabhängig voneinander für $C_1$-$C_4$-Alkyl, Benzyl oder Phenyl stehen; $R_2$ für Methyl, Ethyl, Isopropyl oder sek.-Butyl steht; und $R_3$ für Wasserstoff, $C_1$-$C_4$-Alkyl, einen unsubstituierten oder einen ein- bis dreifach durch Halogen, $C_1$-$C_2$-Alkyl, $C_1$-$C_2$-Alkoxy, $C_1$-$C_2$-Alkylthio, $C_1$-$C_2$-Haloalkyl, Nitro oder Cyano substituierten Rest aus der Reihe Phenyl und Benzyl steht oder die Gruppe U

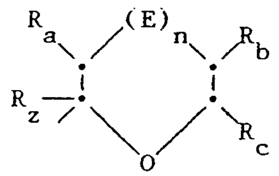

(U)

repräsentiert, worin n für eine der Zahlen 0 oder 1 steht, E für Sauerstoff oder $CH_2$ und $R_z$ für Wasserstoff steht, $R_a$ Wasserstoff, Fluor, Chlor, Brom, Methyl oder 2-Tetrahydropyranyl bedeutet und $R_b$ und $R_c$ unabhängig voneinander für Wasserstoff, Halogen oder Methyl stehen und die übrigen Substituenten wie in Anspruch 2 definiert sind.

**5.** Mittel nach Anspruch 1, dadurch gekennzeichnet, dass es als Aktivsubstanz eine Verbindung der Formel I enthält, worin X die Gruppe -CH($OR_1$)-repräsentiert, wobei $R_1$ Wasserstoff, $R_4$-C(O)- oder -Si-($R_5$($R_6$)($R_7$) bedeutet, wobei $R_4$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxi, $C_1$-$C_3$-Haloalkoxi, Cyano und/oder Nitro substituierte Gruppe aus der Reihe Phenyl und Benzyl steht; $R_5$, $R_6$, $R_7$ unabhängig voneinander für $C_1$-$C_4$-Alkyl, Benzyl oder Phenyl stehen; $R_2$ für Methyl, Ethyl, Isopropyl oder sek.-Butyl steht; und $R_3$ für Wasserstoff, $C_1$-$C_4$-Alkyl, einen unsubstituierten oder einen ein- bis dreifach durch Halogen, $C_1$-$C_2$-Alkyl, $C_1$-$C_2$-Alkoxy, $C_1$-$C_2$-Alkylthio, $C_1$-$C_2$-Haloalkyl, Nitro oder Cyano substituierten Rest aus der Reihe Phenyl und Benzyl steht oder die Gruppe U

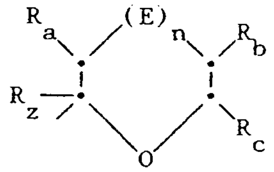

(U)

repräsentiert, worin n für eine der Zahlen 0 oder 1 steht, E für Sauerstoff oder $CH_2$ und $R_z$ für Wasserstoff steht, $R_a$ Wasserstoff, Fluor, Chlor, Brom, Methyl oder 2-Tetrahydropyranyl bedeutet und $R_b$ und $R_c$ unabhängig voneinander für Wasserstoff, Halogen oder Methyl stehen und die übrigen Substituenten wie in Anspruch 1 definiert sind.

6. Mittel nach Anspruch 3, dadurch gekennzeichnet, dass es als Aktivsubstanz eine Verbindung der Formel I enthält, worin

   $R_3$ für Wasserstoff, $C_1$-$C_6$-Alkyl, $C_2$-$C_6$-Alkenyl, einen unsubstituierten oder einen ein- oder mehrfach durch Halogen, $C_1$-$C_3$-Alkyl, $C_1$-$C_3$-Alkoxy, $C_1$-$C_3$-Alkylthio, $C_1$-$C_3$-Haloalkyl, Nitro oder Cyano substituierten Rest aus der Reihe Phenyl und Benzyl steht oder die Gruppe U für eine der Gruppen

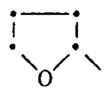 , 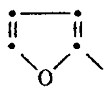 , 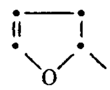 , 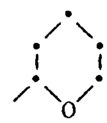 , 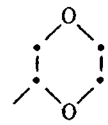 ,

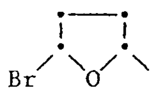 , 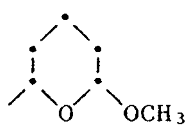 , 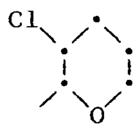 , 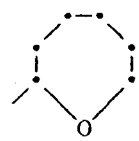 oder 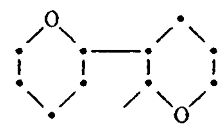

   steht und die übrigen Substituenten wie in Anspruch 3 definiert sind.

7. Mittel nach Anspruch 6, dadurch gekennzeichnet, dass es als Aktivsubstanz eine Verbindung der Formel I enthält, worin $R_1$ Wasserstoff bedeutet und

   $R_3$ Wasserstoff, $C_1$-$C_4$-Alkyl, Phenyl, Benzyl, oder eine der Gruppen

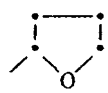 , 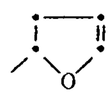 , 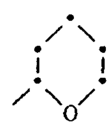 oder 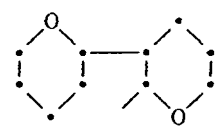

   repräsentiert und die übrigen Substituenten wie in Anspruch 6 definiert sind.

8. Mittel nach Anspruch 1, dadurch gekennzeichnet, dass es als Aktivsubstanz eine Verbindung der Formel I enthält, worin $R_2$ für Methyl steht und die übrigen Substituenten wie in einem der Anspruche 1 bis 7 definiert sind.

9. Mittel nach Anspruch 1, dadurch gekennzeichnet, dass es als Aktivsubstanz eine Verbindung der Formel I enthält, worin $R_2$ für Ethyl steht und die übrigen Substituenten wie in einem der Anspruche 1 bis 7 definiert sind.

10. Mittel nach Anspruch 1, dadurch gekennzeichnet, dass es als Aktivsubstanz eine Verbindung der Formel I ausgewählt aus der nachfolgenden Reihe enthält:
    5-Acetoxi-29-methoximino-milbemycin D,
    5-(Dimethyl-tert.-butyl-silyloxi)-29-hydroximino-milbemycin $A_4$,
    29-Hydroximino-milbemycin $A_4$,
    29-Methoximino-milbemycin $A_4$,
    29-Phenoximino-milbemycin $A_4$,
    29-Tetrahydropyran-2-yl-oximino-milbemycin $A_4$
    5,29-bis-Hydroximino-milbemycin $A_4$.

11. Verfahren zur Herstellung einer Verbindung der Formel I,

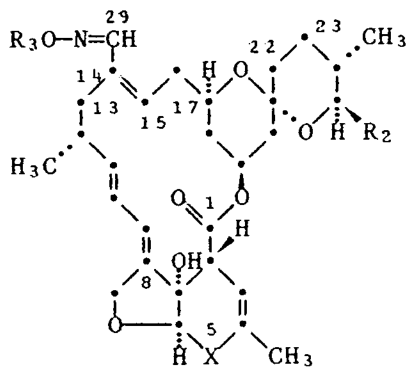

(I)

in welcher

- X eine der Gruppen $-CH(OR_1)-$, $-C(O)-$ oder $C(=N\text{-}OR)-$ repräsentiert;
- $R_1$ Wasserstoff oder eine OH-Schutzgruppe bedeutet;
- R für Wasserstoff, eine OH-Schutzgruppe, eine Alkyl-, Cycloalkyl-oder Acylgruppe steht, gruppe steht; wobei unter OH-Schutzgruppe für die Substituenten R und $R_1$ die Reste $R_4$-C(O)- oder-$Si(R_5)(R_6)(R_7)$ zu verstehen sind, wobei $R_4$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxi, $C_1$-$C_3$-Haloalkoxi, Cyano und/oder Nitro substituierte Gruppe aus der Reihe Phenyl und Benzyl steht; $R_5$, $R_6$, $R_7$ unabhängig voneinander für $C_1$-$C_4$-Alkyl, Benzyl oder Phenyl stehen;
- $R_2$ für Methyl, Ethyl, Isopropyl, sek.Butyl oder für die Gruppe

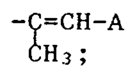

steht, worin A Methyl, Ethyl oder Isopropyl bedeutet, und

- $R_3$ für Wasserstoff, $C_1$-$C_6$-Alkyl, $C_2$-$C_6$-Alkenyl, einen unsubstituierten oder einen ein- oder mehrfach durch Halogen, $C_1$-$C_3$-Alkyl, $C_1$-$C_3$-Alkoxy, $C_1$-$C_3$-Alkylthio, $C_1$-$C_3$-Haloalkyl, Nitro oder Cyano substituierten Rest aus der Reihe Phenyl und Benzyl steht oder die Gruppe U

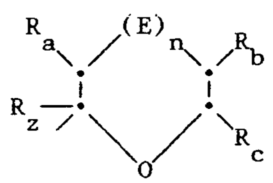

(U)

repräsentiert, worin n für eine der Zahlen 0, 1, 2, 3, 4 oder 5 steht, E für Sauerstoff oder $-CH(R_d)-$ und $R_z$ für Wasserstoff steht, $R_a$ Wasserstoff, Halogen, $C_1$-$C_3$-Alkyl, $C_1$-$C_3$-Alkoxy oder 2-Tetrahydropyranyl bedeutet und $R_b$, $R_c$ und $R_d$ unabhängig voneinander für Wasserstoff, Halogen, $C_1$-$C_3$-Alkyl oder $C_1$-$C_3$-Alkoxy stehen, wobei die Gruppe U auch in ungesättigter Form vorliegen kann, dadurch gekennzeichnet, dass man ein 29-Oxo-Milbemycin der Formel II

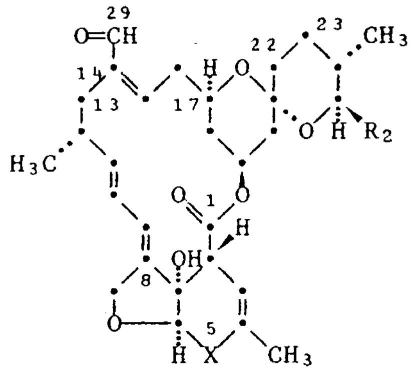

(II)

mit einem primären Oxamin der Formel III

$H_2N\text{-}OR_3$ (III)

oder einem seiner Salze umsetzt, und sofern gewünscht, die resultierende Verbindung der Formel I, sofern $R_3$ für Wasserstoff steht mit einem Halogenid der Formel IV

$R_3'$ -Hal (IV)

weiterreagieren lässt, wobei $R_2$ für Methyl, Ethyl, Isopropyl oder sek.Butyl oder für die Gruppe

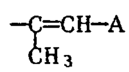

steht, worin A Methyl, Ethyl oder Isopropyl bedeutet und der Substituent $R_3$ die unter Formel I angegebenen Bedeutungen hat, X für -C(OR$_1$) steht, wobei $R_1$ Wasserstoff oder eine der unter Formel I genannten OH-Schutzgruppen bedeutet und Hal für Halogen steht, und $R_3'$ alle Bedeutungen von $R_3$, mit Ausnahme von Wasserstoff hat, wobei man in der Regel von Verbindungen der Formel II ausgeht, die entweder eine freie 5 Hydroxigruppe enthalten und, sofern gewünscht, die resultierenden Produkte am 5-O-Atom nachacyliert oder nachsilyliert oder bereits von einer in 5-Position silylierten oder acylierten Verbindung der Formel II ausgeht und die Schutzgruppe, sofern gewünscht, abspaltet; oder sofern Verbindungen der Formel I, worin X für die Gruppe -C(O)-steht, das Herstellungsziel sind, von Verbindungen der Formel II ausgeht, die eine freie 5-Hydroxygruppe enthalten und diese mit einem zur Oxidation geeigneten Reagenz behandelt; oder aber, sofern Verbindungen der Formel I, worin X für die Gruppe -C(=N-OR)- steht, wobei R die unter Formel I angegebene Bedeutung hat, gewünscht sind, von einer Verbindung der Formel I ausgeht, in der X für -C(O)- steht und diese mit Hydroxylamin oder einem seiner Salze umsetzt und gewünschtenfalls anschliessend den Substituenten R einführt oder aber die Umsetzung mit einer Verbindung der Formel $NH_2$-OR oder einem Salz davon vornimmt.

**12.** Verfahren nach Anspruch 11 zur Herstellung einer Verbindung der Formel I

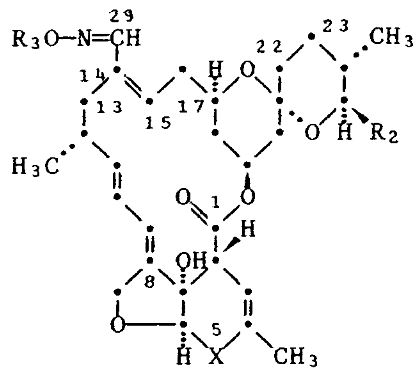

(I)

in welcher

X eine der Gruppen $-CH(OR_1)-$, -C(O)- oder -C( = N-OR)- repräsentiert

$R_1$ Wasserstoff oder eine OH-Schutzgruppe bedeutet;

R für Wasserstoff, eine OH-Schutzgruppe, eine Alkyl-, Cycloalkylgruppe steht; wobei unter OH-Schutzgruppe für die Substituenten R und $R_1$ die Reste $R_4$-C(O)- oder-Si($R_5$)($R_6$)($R_7$) zu verstehen sind, wobei $R_4$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxi, $C_1$-$C_3$-Haloalkoxi, Cyano und/oder Nitro substituierte Gruppe aus der Reihe Phenyl und Benzyl steht; $R_5$, $R_6$, $R_7$ unabhängig voneinander für $C_1$-$C_4$-Alkyl, Benzyl oder Phenyl stehen;

$R_2$ für Methyl, Ethyl, Isopropyl oder sek.Butyl steht; und

$R_3$ für Wasserstoff, $C_1$-$C_6$-Alkyl, $C_2$-$C_6$-Alkenyl, einen unsubstituierten oder einen ein- oder mehrfach durch Halogen, $C_1$-$C_3$-Alkyl, $C_1$-$C_3$-Alkoxy, $C_1$-$C_3$-Alkylthio, $C_1$-$C_3$-Haloalkyl, Nitro oder Cyano substituierten Rest aus der Reihe Phenyl und Benzyl steht oder die Gruppe U

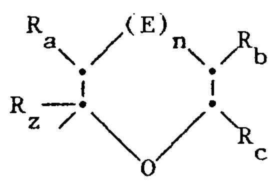

(U)

repräsentiert, worin n für eine der Zahlen 0, 1, 2, 3, 4 oder 5 steht, E für Sauerstoff oder $-CH(R_d)-$ und $R_z$ für Wasserstoff steht, $R_a$ Wasserstoff, Halogen, $C_1$-$C_3$-Alkyl, $C_1$-$C_3$-Alkoxy oder 2-Tetrahydropyranyl bedeutet und $R_b$, $R_c$ und $R_d$ unabhängig voneinander für Wasserstoff, Halogen, $C_1$-$C_3$-Alkyl oder $C_1$-$C_3$-Alkoxy stehen, wobei die Gruppen U auch in ungesättigter Form vorliegen kann, dadurch gekennzeichnet, dass man ein 29-oxo-Milbemycin der Formel II

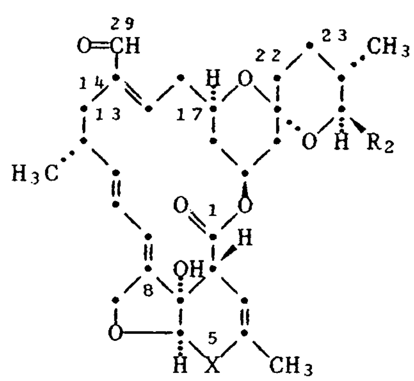

(II)

mit einem primären Oxamin der Formel III

$H_2N\text{-}OR_3$ (III)

oder einem seiner Salze umsetzt, und sofern gewünscht, die resultierende Verbindung der Formel I, sofern $R_3$ für Wasserstoff steht mit einem Halogenid der Formel IV

$R_3'$ -Hal (IV)

weiterreagieren lässt, wobei $R_2$ für Methyl, Ethyl, Isopropyl oder sek.Butyl steht und der Substituent $R_3$ die unter Formel I angegebenen Bedeutungen hat, X für $-C(OR_1)-$ steht, wobei $R_1$ Wasserstoff oder eine der unter Formel I genannten OH-Schutzgruppen bedeutet und Hal für Halogen steht, und $R_3'$ alle Bedeutungen von $R_3$, mit Ausnahme von Wasserstoff hat, wobei man in der Regel von Verbindungen der Formel II ausgeht, die entweder eine freie 5 Hydroxigruppe enthalten und, sofern gewünscht, die resultierenden Produkte am 5-O-Atom nachacyliert oder nachsilyliert oder bereits von einer in 5-Position silylierten oder acylierten Verbindung der Formel II ausgeht und die Schutzgruppe, sofern gewünscht, abspaltet; oder sofern Verbindungen der Formel I, worin X für die Gruppe -C(O)- steht, das Herstellungsziel sind, von Verbindungen der Formel II ausgeht, die eine freie 5-Hydroxygruppe enthalten und diese mit einem zur Oxidation geeigneten Reagenz behandelt; oder aber, sofern Verbindungen der Formel I, worin X für die Gruppe -C(=N-OR)- steht, wobei R die unter Formel I angegebene Bedeutung hat, gewünscht sind, von einer Verbindung der Formel I ausgeht, in der X für -C(O)- steht und diese mit Hydroxylamin oder einem seiner Salze umsetzt und gewünschtenfalls anschliessend den Substituenten R einführt oder aber die Umsetzung mit einer Verbindung der Formel $NH_2\text{-}OR$ oder einem Salz davon vornimmt.

**13.** Verfahren zur Herstellung einer Verbindung der Formel I gemäss Anspruch 1,

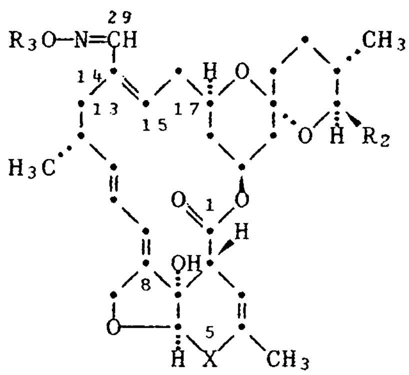

(I)

in welcher

X die Gruppe $-CH(OR_1)-$ repräsentiert;

$R_1$ Wasserstoff oder eine OH-Schutzgruppe bedeutet; wobei unter OH-Schutzgruppe die Reste $R_4\text{-}C(O)\text{-}$ oder $\text{-}Si(R_5)(R_6)(R_7)$ zu verstehen sind, wobei $R_4$ für $C_1\text{-}C_{10}$-Alkyl, $C_1\text{-}C_{10}$-Haloalkyl oder eine unsubstituierte oder durch Halogen, $C_1\text{-}C_3$-Alkyl, $C_1\text{-}C_3$-Haloalkyl, $C_1\text{-}C_3$-Alkoxi, $C_1\text{-}C_3$-Haloalkoxi, Cyano und/oder Nitro substituierte Gruppe aus der Reihe Phenyl und Benzyl steht; $R_5$, $R_6$, $R_7$ unabhängig voneinander für $C_1\text{-}C_4$-Alkyl, Benzyl oder Phenyl stehen;

$R_2$ für Methyl, Ethyl, Isopropyl oder sek.Butyl steht; und

$R_3$ für Wasserstoff, $C_1\text{-}C_6$-Alkyl, $C_2\text{-}C_6$-Alkenyl, einen unsubstituierten oder einen ein- oder mehrfach durch Halogen, $C_1\text{-}C_3$-Alkyl, $C_1\text{-}C_3$-Alkoxy, $C_1\text{-}C_3$-Alkylthio, $C_1\text{-}C_3$-Haloalkyl, Nitro oder Cyano substituierten Rest aus der Reihe Phenyl und Benzyl steht oder die Gruppe U

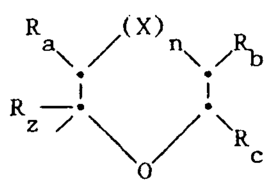

(U)

repräsentiert, worin n für eine der Zahlen 0, 1, 2, 3, 4 oder 5 steht, X für Sauerstoff oder -CH($R_d$)- und $R_z$ für Wasserstoff steht, $R_a$ Wasserstoff, Halogen, $C_1$-$C_3$-Alkyl, $C_1$-$C_3$-Alkoxy oder 2-Tetrahydropyranyl bedeutet und $R_b$, $R_c$ und $R_d$ unabhängig voneinander für Wasserstoff, Halogen, $C_1$-$C_3$-Alkyl oder $C_1$-$C_3$-Alkoxy stehen, wobei die Gruppen U auch in ungesättigter Form vorliegen kann, dadurch gekennzeichnet, dass man ein 29-Oxo-Milbemycin der Formel II

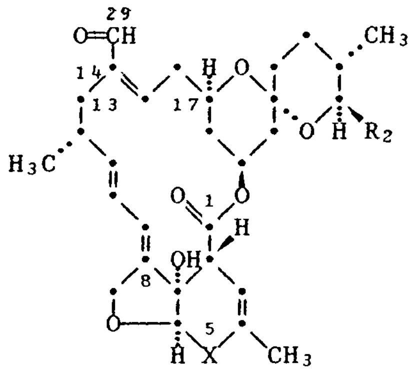

(II)

mit einem primären Oxamin der Formel III

$H_2N$-$OR_3$ (III)

oder einem seiner Salze umsetzt, und sofern gewünscht, die resultierende Verbindung der Formel I, sofern $R_3$ für Wasserstoff steht mit einem Halogenid der Formel IV

$R_3'$ -Hal (IV)

weiterreagieren lässt, wobei $R_2$ für Methyl, Ethyl, Isopropyl oder sek.Butyl steht und die Substituenten $R_1$ und $R_3$ die unter Formel I angegebenen Bedeutungen haben, Hal für Halogen steht, und $R_3'$ alle Bedeutungen von $R_3$, mit Ausnahme von Wasserstoff hat, wobei man von Verbindungen der Formel II ausgeht, die entweder eine freie 5 Hydroxigruppe enthalten und, sofern gewünscht, die resultierenden Produkte am 5-O-Atom nachacyliert oder nachsyliliert oder bereits von einer in 5-Position sylilierten oder acylierten Verbindung der Formel II ausgeht und die Schutzgruppe, sofern gewünscht, abspaltet.

**14.** Verfahren zur Bekämpfung von Parasiten an Pflanzen oder von Schadinsekten, dadurch gekennzeichnet, dass man eine parasitizid bzw. insektizid wirksame Menge einer Verbindung der Formel I

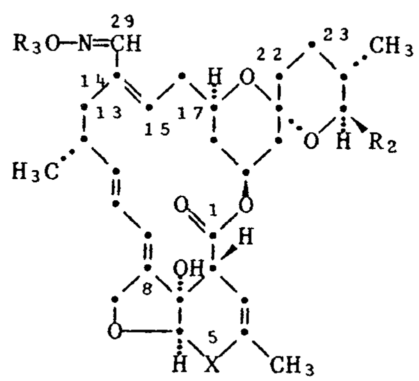

(I)

in welcher

- X eine der Gruppen -CH($OR_1$)-, -C(O)- oder -C( = N-OR)- repräsentiert;
- $R_1$ Wasserstoff oder eine OH-Schutzgruppe bedeutet;
- R für Wasserstoff, eine OH-Schutzgruppe, eine Alkyl-, Cycloalkylgruppe steht; wobei unter OH-Schutzgruppe für die Substituenten R und $R_1$ die Reste $R_4$-C(O)- oder-Si($R_5$)($R_6$)($R_7$) zu

verstehen sind, wobei $R_4$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxi, $C_1$-$C_3$-Haloalkoxi, Cyano und/oder Nitro substituierte Gruppe aus der Reihe Phenyl und Benzyl steht; $R_5$, $R_6$, $R_7$ unabhängig voneinander für $C_1$-$C_4$-Alkyl, Benzyl oder Phenyl stehen;

$R_2$ für Methyl, Ethyl, Isopropyl, sek.Butyl oder für die Gruppe

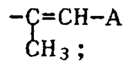

steht, worin A Methyl, Ethyl oder Isopropyl bedeutet, und

$R_3$ für Wasserstoff, $C_1$-$C_6$-Alkyl, $C_2$-$C_6$-Alkenyl, einen unsubstituierten oder einen ein- oder mehrfach durch Halogen, $C_1$-$C_3$-Alkyl, $C_1$-$C_3$-Alkoxy, $C_1$-$C_3$-Alkylthio, $C_1$-$C_3$-Haloalkyl, Nitro oder Cyano substituierten Rest aus der Reihe Phenyl und Benzyl steht oder die Gruppe U

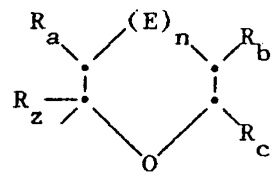

(U)

repräsentiert, worin n für eine der Zahlen 0, 1, 2, 3, 4 oder 5 steht, E für Sauerstoff oder -CH($R_d$)- und $R_z$ für Wasserstoff steht, $R_a$ Wasserstoff, Halogen, $C_1$-$C_3$-Alkyl, $C_1$-$C_3$-Alkoxy oder 2-Tetrahydropyranyl bedeutet und $R_b$, $R_c$ und $R_d$ unabhängig voneinander für Wasserstoff, Halogen, $C_1$-$C_3$-Alkyl oder $C_1$-$C_3$-Alkoxy stehen, wobei die Gruppe U auch in ungesättigter Form vorliegen kann, auf den Parasiten, das Schadinsekt oder seinen Lebensraum appliziert.

**15.** Verfahren zur Bekämpfung von Parasiten an Pflanzen oder von Schadinsekten gemäss Anspruch 14, dadurch gekennzeichnet, dass man eine parasitizid bzw. insektizid wirksame Menge einer Verbindung der Formel I

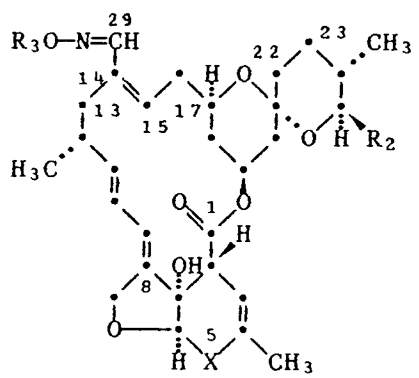

(I)

in welcher

X eine der Gruppen -CH($OR_1$)-, -C(O)- oder -C(=N-OR)- repräsentiert,

$R_1$ Wasserstoff oder eine OH-Schutzgruppe bedeutet;

R für Wasserstoff, eine OH-Schutzgruppe, eine Alkyl-, Cycloalkylgruppe steht; wobei unter OH-Schutzgruppe für die Substituenten R und $R_1$ die Reste $R_4$-C(O)- oder-Si($R_5$)($R_6$)($R_7$) zu verstehen sind, wobei $R_4$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxi, $C_1$-$C_3$-Haloalkoxi, Cyano und/oder Nitro substituierte Gruppe aus der Reihe Phenyl und Benzyl steht; $R_5$, $R_6$, $R_7$ unabhängig voneinander für $C_1$-$C_4$-Alkyl, Benzyl oder Phenyl stehen;

$R_2$ für Methyl, Ethyl, Isopropyl oder sek.Butyl steht; und

$R_3$ für Wasserstoff, $C_1$-$C_6$-Alkyl, $C_2$-$C_6$-Alkenyl, einen unsubstituierten oder einen ein- oder mehrfach durch Halogen, $C_1$-$C_3$-Alkyl, $C_1$-$C_3$-Alkoxy, $C_1$-$C_3$-Alkylthio, $C_1$-$C_3$-Haloalkyl, Nitro

oder Cyano substituierten Rest aus der Reihe Phenyl und Benzyl steht oder die Gruppe U

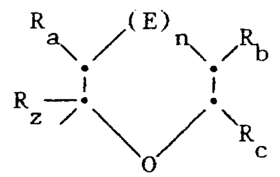

(U)

repräsentiert, worin n für eine der Zahlen 0, 1, 2, 3, 4 oder 5 steht, E für Sauerstoff oder -CH($R_d$)- und $R_z$ für Wasserstoff steht, $R_a$ Wasserstoff, halogen, $C_1$-$C_3$-Alkyl, $C_1$-$C_3$-Alkoxy oder 2-Tetrahydropyranyl bedeutet und $R_b$, $R_c$ und $R_d$ unabhängig voneinander für Wasserstoff, Halogen, $C_1$-$C_3$-Alkyl oder $C_1$-$C_3$-Alkoxy stehen, wobei die Gruppen U auch in ungesättigter Form vorliegen kann, auf den Parasiten, das Schadinsekt oder deren Lebensraum appliziert.

**16.** Verfahren zur Bekämpfung von Parasiten an Pflanzen oder von Schadinsekten gemäss Anspruch 14, dadurch gekennzeichnet, dass man eine parasitizid bzw. insektizid wirksame Menge einer Verbindung der Formel I

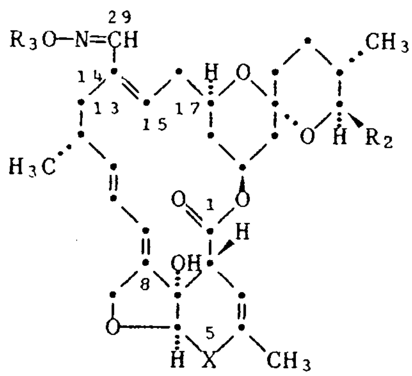

(I)

in welcher

X die Gruppe -CH($OR_1$)- repräsentiert;

$R_1$ Wasserstoff oder eine OH-Schutzgruppe bedeutet; wobei unter OH-Schutzgruppe die Reste $R_4$-C(O)- oder-Si($R_5$)($R_6$)($R_7$) zu verstehen sind, wobei $R_4$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxi, $C_1$-$C_3$-Haloalkoxi, Cyano und/oder Nitro substituierte Gruppe aus der Reihe Phenyl und Benzyl steht; $R_5$, $R_6$, $R_7$ unabhängig voneinander für $C_1$-$C_4$-Alkyl, Benzyl oder Phenyl stehen;

$R_2$ für Methyl, Ethyl, Isopropyl oder sek.Butyl steht; und

$R_3$ für Wasserstoff, $C_1$-$C_6$-Alkyl, $C_2$-$C_6$-Alkenyl, einen unsubstituierten oder einen ein- oder mehrfach durch Halogen, $C_1$-$C_3$-Alkyl, $C_1$-$C_3$-Alkoxy, $C_1$-$C_3$-Alkylthio, $C_1$-$C_3$-Haloalkyl, Nitro oder Cyano substituierten Rest aus der Reihe Phenyl und Benzyl steht oder die Gruppe U

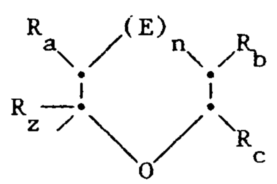

(U)

repräsentiert, worin n für eine der Zahlen 0, 1, 2, 3, 4 oder 5 steht, E für Sauerstoff oder -CH($R_d$)- und $R_z$ für Wasserstoff steht, $R_a$ Wasserstoff, Halogen, $C_1$-$C_3$-Alkyl, $C_1$-$C_3$-Alkoxy oder 2-Tetrahydropyranyl bedeutet und $R_b$, $R_c$ und $R_d$ unabhängig voneinander für Wasserstoff, Halogen, $C_1$-$C_3$-Alkyl oder $C_1$-

$C_3$-Alkoxy stehen, wobei die Gruppe U auch in ungesättigter Form vorliegen kann, auf den Parasiten, das Schadinsekt oder deren Lebensraum appliziert.

**17.** Verfahren gemäss Anspruch 14, dadurch gekennzeichnet, dass es sich bei den Parasiten um Nematoden handelt.

**18.** Verwendung einer Verbindung der Formel I gemäss Anspruch 1 zur Herstellung eines Mittels zur Bekämpfung von Ekto- und Endoparasiten am Nutztier oder von Schadinsekten in allen Entwicklungsstadien.

**Claims**

**Claims for the following Contracting States : AT, BE, CH, DE, FR, GB, GR, IT, LI, LU, NL, SE**

**1.** A compound of formula I

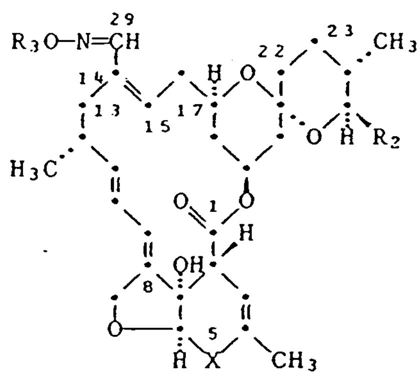

(I)

wherein

X is one of the groups $-CH(OR_1)-$, $-C(O)-$ or $-C(=N-OR)-$;

$R_1$ is hydrogen or an OH-protecting group;

R is hydrogen, an OH-protecting group, or an alkyl or cycloalkyl group; there being understood by OH-protecting group for the substituents R and $R_1$ the radical $R_4$-C(O)- or $-Si(R_5)(R_6)(R_7)$ wherein $R_4$ is $C_1$-$C_{10}$alkyl, $C_1$-$C_{10}$haloalkyl or a group from the series phenyl and benzyl that is unsubstituted or substituted by halogen, $C_1$-$C_3$alkyl, $C_1$-$C_3$haloalkyl, $C_1$-$C_3$alkoxy, $C_1$-$C_3$haloalkoxy, cyano and/or by nitro, and $R_5$, $R_6$ and $R_7$ are each independently $C_1$-$C_4$alkyl, benzyl or phenyl;

$R_2$ is methyl, ethyl, isopropyl, sec-butyl or the group

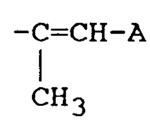

wherein A is methyl, ethyl or isopropyl; and

$R_3$ is hydrogen, $C_1$-$C_6$alkyl, $C_2$-$C_6$alkenyl, a radical from the series phenyl and benzyl that is unsubstituted or mono- or poly-substituted by halogen, $C_1$-$C_3$alkyl, $C_1$-$C_3$alkoxy, $C_1$-$C_3$alkylthio, $C_1$-$C_3$haloalkyl, nitro or by cyano or is the group U

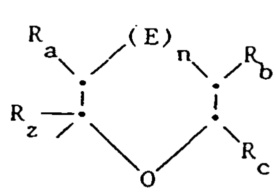

(U)

wherein n is one of the integers 0, 1, 2, 3, 4 or 5, E is oxygen or $-CH(R_d)-$ and $R_z$ is

hydrogen, $R_a$ is hydrogen, halogen, $C_1$-$C_3$alkyl, $C_1$-$C_3$alkoxy or 2-tetrahydropyranyl and $R_b$, $R_c$ and $R_d$ are each independently hydrogen, halogen, $C_1$-$C_3$alkyl or $C_1$-$C_3$alkoxy, which group U may also be in unsaturated form.

**2.** A compound of formula I according to claim 1, wherein
$R_2$ is methyl, ethyl, isopropyl or sec-butyl.

**3.** A compound of formula I according to claim 2 wherein
X is the group -CH($OR_1$)-; and
$R_1$ is hydrogen or an OH-protecting group, there being understood by OH-protecting group the radical $R_4$-C(O)-or -Si($R_5$)($R_6$)($R_7$) wherein $R_4$ is $C_1$-$C_{10}$alkyl, $C_1$-$C_{10}$haloalkyl or a group from the series phenyl and benzyl that is unsubstituted or substituted by halogen, $C_1$-$C_3$alkyl, $C_1$-$C_3$haloalkyl, $C_1$-$C_3$alkoxy, $C_1$-$C_3$haloalkoxy, cyano and/or by nitro, and $R_5$, $R_6$ and $R_7$ are each independently $C_1$-$C_4$alkyl, benzyl or phenyl.

**4.** A compound of formula I according to claim 2, wherein X is one of the groups -CH($OR_1$)-, -C(O)- or -C-(=NH-OH)-in which $R_1$ is hydrogen, $R_4$-C(O)- or -Si($R_5$)($R_6$)($R_7$) wherein $R_4$ is $C_1$-$C_{10}$alkyl, $C_1$-$C_{10}$haloalkyl or a group from the series phenyl and benzyl that is unsubstituted or substituted by halogen, $C_1$-$C_3$alkyl, $C_1$-$C_3$haloalkyl, $C_1$-$C_3$alkoxy, $C_1$-$C_3$haloalkoxy, cyano and/or by nitro, and $R_5$, $R_6$ and $R_7$ are each independently $C_1$-$C_4$alkyl, benzyl or phenyl; $R_2$ is methyl, ethyl, isopropyl or sec-butyl; and $R_3$ is hydrogen, $C_1$-$C_4$alkyl, a radical from the series phenyl and benzyl that is unsubstituted or mono- to tri-substituted by halogen, $C_1$-$C_2$alkyl, $C_1$-$C_2$alkoxy, $C_1$-$C_2$alkylthio, $C_1$-$C_2$haloalkyl, nitro or by cyano or is the group U

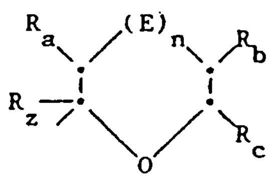

(U)

wherein n is one of the integers 0 or 1, E is oxygen or $CH_2$ and $R_z$ is hydrogen, $R_a$ is hydrogen, fluorine, chlorine, bromine, methyl or 2-tetrahydropyranyl and $R_b$ and $R_c$ are each independently hydrogen, halogen or methyl.

**5.** A compound of formula I according to claim 1, wherein X is the group -CH($OR_1$)- in which $R_1$ is hydrogen, $R_4$-C(O)- or -Si($R_5$)($R_6$)($R_7$) wherein $R_4$ is $C_1$-$C_{10}$alkyl, $C_1$-$C_{10}$haloalkyl or a group from the series phenyl and benzyl that is unsubstituted or substituted by halogen, $C_1$-$C_3$alkyl, $C_1$-$C_3$haloalkyl, $C_1$-$C_3$alkoxy, $C_1$-$C_3$haloalkoxy,cyano and/or by nitro, and $R_5$, $R_6$ and $R_7$ are each independently $C_1$-$C_4$alkyl, benzyl or phenyl; $R_2$ is methyl, ethyl, isopropyl or sec-butyl; and $R_3$ is hydrogen, $C_1$-$C_4$alkyl, a radical from the series phenyl and benzyl that is unsubstituted or mono- to tri-substituted by halogen, $C_1$-$C_2$alkyl, $C_1$-$C_2$alkoxy, $C_1$-$C_2$alkylthio, $C_1$-$C_2$haloalkyl, nitro or by cyano or is the group U

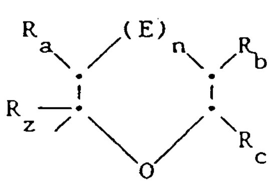

(U)

wherein n is one of the integers 0 or 1, E is oxygen or $CH_2$ and $R_z$ is hydrogen, $R_a$ is hydrogen, fluorine, chlorine, bromine, methyl or 2-tetrahydropyranyl and $R_b$ and $R_c$ are each independently hydrogen, halogen or methyl.

**6.** A compound of formula I according to claim 3, wherein $R_3$ is hydrogen, $C_1$-$C_6$alkyl, $C_2$-$C_6$alkenyl, a radical from the series phenyl and benzyl that is unsubstituted or mono- or poly-substituted by halogen,

$C_1$-$C_3$alkyl, $C_1$-$C_3$alkoxy, $C_1$-$C_3$alkylthio, $C_1$-$C_3$haloalkyl, nitro or by cyano, or the group U is one of the groups

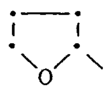 , 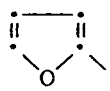 , 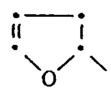 , 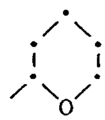 , 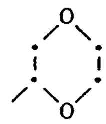 ,

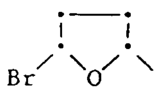 , 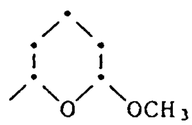 , 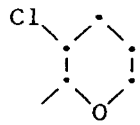 , 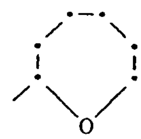 or 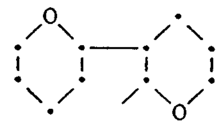 .

**7.** A compound of formula I according to claim 6 wherein $R_1$ is hydrogen; and $R_3$ is hydrogen, $C_1$-$C_4$alkyl, phenyl, benzyl, or one of the groups

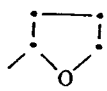 , 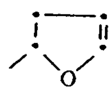 , 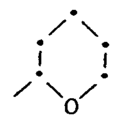 or 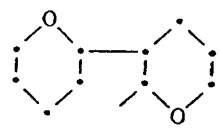 .

**8.** A compound of formula I according to any one of claims 1 to 7, wherein $R_2$ is methyl.

**9.** A compound of formula I according to any one of claims 1 to 7, wherein $R_2$ is ethyl.

**10.** A compound of formula I according to claim 1 selected from the group:
5-acetoxy-29-methoximino-milbemycin D,
5-(dimethyl-tert-butylsilyloxy)-29-hydroximino-milbemycin $A_4$,
29-hydroximino-milbemycin $A_4$,
29-methoximino-milbemycin $A_4$,
29-phenoximino-milbemycin $A_4$,
29-tetrahydropyran-2-yloximino-milbemycin $A_4$,
5,29-bis-hydroximino-milbemycin $A_4$.

**11.** A process for the preparation of a compound of formula I according to claim 1, which comprises reacting a 29-oxo-milbemycin of formula II

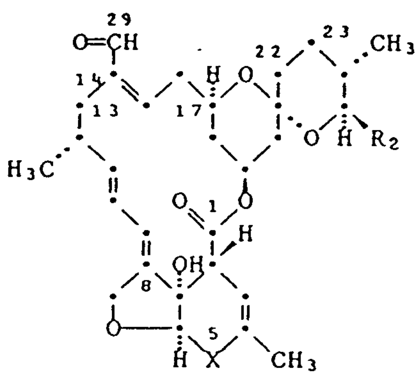

(II)

with a primary oxamine of formula III

$H_2N$-$OR_3$ (III)

or a salt thereof and, if desired, further reacting the resulting compound of formula I, if $R_3$ is hydrogen, with a halide of formula IV

$R_3'$ -Hal (IV)

in which formulae $R_2$ is methyl, ethyl, isopropyl or sec-butyl or the group

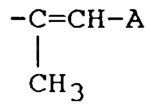

wherein A is methyl, ethyl or isopropyl and the substituent $R_3$ is as defined for formula I, X is -C($OR_1$) wherein $R_1$ is hydrogen or one of the OH-protecting groups mentioned in claim 1, and Hal is halogen, and $R_3'$ has all the meanings of $R_3$ except hydrogen, in which process generally either a compound of formula II that contains a free 5-hydroxy group is used as starting material and, if desired, the resulting product is subsequently acylated or silylated at the 5-O atom, or a compound of formula II already silylated or acylated in the 5-position is used as starting material and, if desired, the protecting group is removed; or, if a compound of formula I wherein X is the group -C(O)- is the aim of the preparation, there is used as starting material a compound of formula II that contains a free 5-hydroxy group, which is treated with a reagent suitable for oxidation; or, alternatively, if a compound of formula I is desired wherein X is the group -C(=N-OR)- in which R is as defined for formula I, there is used as starting material a compound of formula I wherein X is -C(O)-, which is reacted with hydroxylamine or a salt thereof and, if desired, the substituent R is introduced subsequently, or the reaction is carried out with a compound of formula $NH_2$-OR or a salt thereof.

**12.** A process for the preparation of a compound of formula I according to claim 2, which comprises reacting a 29-oxo-milbemycin of formula II

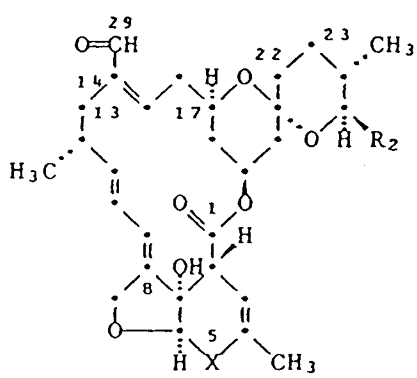

(II)

with a primary oxamine of formula III

$H_2N$-$OR_3$ (III)

or a salt thereof and, if desired, further reacting the resulting compound of formula I, if $R_3$ is hydrogen, with a halide of formula IV

$R_3'$ -Hal (IV)

in which formulae $R_2$ is methyl, ethyl, isopropyl or sec-butyl and the substituent $R_3$ is as defined for formula I, X is -C($OR_1$)- wherein $R_1$ is hydrogen or one of the OH-protecting groups mentioned in claim 2, and Hal is halogen, and $R_3'$ has all the meanings of $R_3$ except hydrogen, in which process generally either a compound of formula II that contains a free 5-hydroxy group is used as starting material and, if desired, the resulting product is subsequently acylated or silylated at the 5-O atom, or a compound of

formula II already silylated or acylated in the 5-position is used as starting material and, if desired, the protecting group is removed; or, if a compound of formula I wherein X is the group -C(O)- is the aim of the preparation, there is used as starting material a compound of formula II that contains a free 5-hydroxy group, which is treated with a reagent suitable for oxidation; or, alternatively, if a compound of formula I is desired wherein X is the group -C( = N-OR)- in which R is as defined for formula I, there is used as starting material a compound of formula I wherein X is -C(O)-, which is reacted with hydroxylamine or a salt thereof and, if desired, the substituent R is introduced subsequently, or the reaction is carried out with a compound of formula $NH_2$-OR or a salt thereof.

**13.** A process for the preparation of a compound of formula I according to claim 3, which process comprises reacting a 29-oxo-milbemycin of formula II

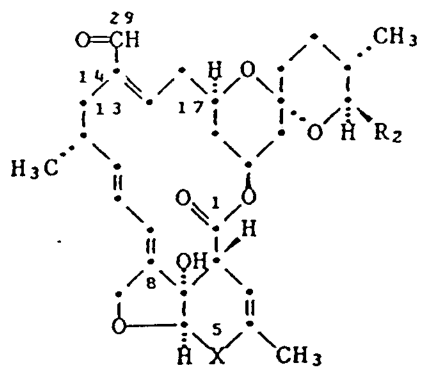

(II)

with a primary oxamine of formula III

$H_2N$-$OR_3$ (III)

or a salt thereof and, if desired, further reacting the resulting compound of formula I, if $R_3$ is hydrogen, with a halide of formula IV

$R_3'$ -Hal (IV)

in which formulae $R_2$ is methyl, ethyl, isopropyl or sec-butyl and the substituents $R_1$ and $R_3$ are as defined for formula I, Hal is halogen, and $R_3'$ has all the meanings of $R_3$ except hydrogen, in which process either a compound of formula II that contains a free 5-hydroxy group is used as starting material and, if desired, the resulting product is subsequently acylated or silylated at the 5-O atom, or a compound of formula II already silylated or acylated in the 5-position is used as starting material and, if desired, the protecting group is removed.

**14.** A composition for controlling ecto- and endo-parasites of productive livestock or for controlling harmful insects, which comprises, in addition to conventional carriers and diluents, a compound of formula I according to claim 1.

**15.** A composition according to claim 14 for controlling ecto- and endo-parasites of productive livestock or for controlling harmful insects, which comprises, in addition to conventional carriers and diluents, a compound of formula I according to claim 2.

**16.** A composition according to claim 14 for controlling ecto- and endo-parasites of productive livestock or for controlling harmful insects, which comprises, in addition to conventional carriers and diluents, a compound of formula I according to claim 3.

**17.** A composition according to claim 14, which comprises as active ingredient a compound of formula I according to any one of claims 4 to 10.

**18.** A compound of formula I according to any one of claims 1 to 10 for use in a method of controlling

parasites of animals.

**19.** A method of controlling plant-destructive parasites, which comprises applying to the parasites or to the locus thereof a parasiticidally or insecticidally effective amount of a compound of formula I according to any one of claims 1 to 10.

**20.** The use of a compound of formula I according to claim 1 for the preparation of a composition for controlling ecto- and endo-parasites of productive livestock or harmful insects in all development stages.

**21.** The use according to claim 20 of a compound of formula I according to any one of claims 4 to 10 for the preparation of a composition for controlling ecto- and endo-parasites of productive livestock or harmful insects in all development stages.

**Claims for the following Contracting State : ES**

**1.** A composition for controlling ecto- and endo-parasites of productive livestock or for controlling harmful insects, which comprises, in addition to conventional carriers and diluents, a compound of formula I

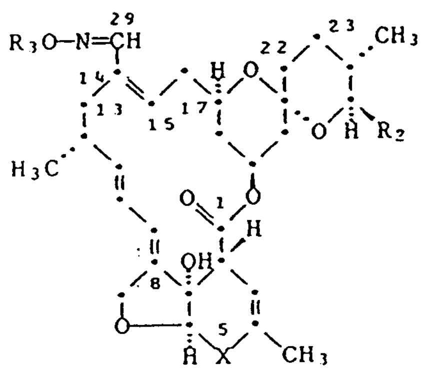

(I)

wherein

X is one of the groups -CH($OR_1$)-, -C(O)- or -C(=N-OR)-;

$R_1$ is hydrogen or an OH-protecting group;

R is hydrogen, an OH-protecting group, or an alkyl or cycloalkyl group; there being understood by OH-protecting group for the substituents R and $R_1$ the radical $R_4$-C(O)- or -Si($R_5$)($R_6$)($R_7$) wherein $R_4$ is $C_1$-$C_{10}$alkyl, $C_1$-$C_{10}$haloalkyl or a group from the series phenyl and benzyl that is unsubstituted or substituted by halogen, $C_1$-$C_3$alkyl, $C_1$-$C_3$haloalkyl, $C_1$-$C_3$alkoxy, $C_1$-$C_3$haloalkoxy, cyano and/or by nitro, and $R_5$, $R_6$ and $R_7$ are each independently $C_1$-$C_4$alkyl, benzyl or phenyl;

$R_2$ is methyl, ethyl, isopropyl, sec-butyl or the group

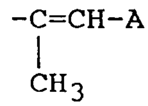

wherein A is methyl, ethyl or isopropyl; and

$R_3$ is hydrogen, $C_1$-$C_6$alkyl, $C_2$-$C_6$alkenyl, a radical from the series phenyl and benzyl that is unsubstituted or mono- or poly-substituted by halogen, $C_1$-$C_3$alkyl, $C_1$-$C_3$alkoxy, $C_1$-$C_3$alkylthio, $C_1$-$C_3$haloalkyl, nitro or by cyano or is the group U

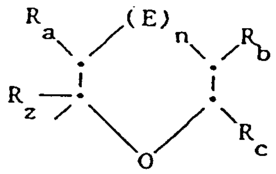

(U)

wherein n is one of the integers 0, 1, 2, 3, 4 or 5, E is oxygen or -CH($R_d$)- and $R_z$ is hydrogen, $R_a$ is hydrogen, halogen, $C_1$-$C_3$alkyl, $C_1$-$C_3$alkoxy or 2-tetrahydropyranyl and $R_b$, $R_c$ and $R_d$ are each independently hydrogen, halogen, $C_1$-$C_3$alkyl or $C_1$-$C_3$alkoxy, which group U may also be in unsaturated form.

2. A composition according to claim 1 which comprises as active substance a compound of formula I wherein $R_2$ is methyl, ethyl, isopropyl or sec-butyl, and the remaining substituents are as defined in claim 1.

3. A composition according to claim 2 which comprises as active substance a compound of formula I wherein

$R_2$ is methyl, ethyl, isopropyl or sec-butyl;

X is the group -CH($OR_1$)-; and

$R_1$ is hydrogen or an OH-protecting group, there being understood by OH-protecting group the radical $R_4$-C(O)-or -Si($R_5$)($R_6$)($R_7$) wherein $R_4$ is $C_1$-$C_{10}$alkyl, $C_1$-$C_{10}$haloalkyl or a group from the series phenyl and benzyl that is unsubstituted or substituted by halogen, $C_1$-$C_3$alkyl, $C_1$-$C_3$haloalkyl, $C_1$-$C_3$alkoxy, $C_1$-$C_3$haloalkoxy, cyano and/or by nitro, and $R_5$, $R_6$ and $R_7$ are each independently $C_1$-$C_4$alkyl, benzyl or phenyl,

and the remaining substituents are as defined in claim 1.

4. A composition according to claim 2 which comprises as active substance a compound of formula I wherein X is one of the groups -CH($OR_1$)-, -C(O)- or -C(=NH-OH)- in which $R_1$ is hydrogen, $R_4$-C(O)- or -Si($R_5$)($R_6$)($R_7$) wherein $R_4$ is $C_1$-$C_{10}$alkyl, $C_1$-$C_{10}$haloalkyl or a group from the series phenyl and benzyl that is unsubstituted or substituted by halogen, $C_1$-$C_3$alkyl, $C_1$-$C_3$haloalkyl, $C_1$-$C_3$alkoxy, $C_1$-$C_3$haloalkoxy, cyano and/or by nitro, and $R_5$, $R_6$ and $R_7$ are each independently $C_1$-$C_4$alkyl, benzyl or phenyl; $R_2$ is methyl, ethyl, isopropyl or sec-butyl; and $R_3$ is hydrogen, $C_1$-$C_4$alkyl, a radical from the series phenyl and benzyl that is unsubstituted or mono- to tri-substituted by halogen, $C_1$-$C_2$alkyl, $C_1$-$C_2$alkoxy, $C_1$-$C_2$-alkylthio, $C_1$-$C_2$haloalkyl, nitro or by cyano or is the group U

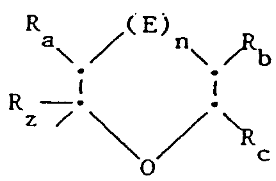

(U)

wherein n is one of the integers 0 or 1, E is oxygen or $CH_2$ and $R_z$ is hydrogen, $R_a$ is hydrogen, fluorine, chlorine, bromine, methyl or 2-tetrahydropyranyl and $R_b$ and $R_c$ are each independently hydrogen, halogen or methyl, and the remaining substituents are as defined in claim 2.

5. A composition according to claim 1 which comprises as active substance a compound of formula I wherein X is the group -CH($OR_1$)- in which $R_1$ is hydrogen, $R_4$-C(O)- or -Si($R_5$)($R_6$)($R_7$) wherein $R_4$ is $C_1$-$C_{10}$alkyl, $C_1$-$C_{10}$haloalkyl or a group from the series phenyl and benzyl that is unsubstituted or substituted by halogen, $C_1$-$C_3$alkyl, $C_1$-$C_3$haloalkyl, $C_1$-$C_3$alkoxy, $C_1$-$C_3$haloalkoxy, cyano and/or by nitro, and $R_5$, $R_6$ and $R_7$ are each independently $C_1$-$C_4$alkyl, benzyl or phenyl; $R_2$ is methyl, ethyl, isopropyl or sec-butyl; and $R_3$ is hydrogen, $C_1$-$C_4$alkyl, a radical from the series phenyl and benzyl that is unsubstituted or mono- to tri-substituted by halogen, $C_1$-$C_2$alkyl, $C_1$-$C_2$alkoxy, $C_1$-$C_2$alkylthio, $C_1$-$C_2$haloalkyl, nitro or by cyano or is the group U

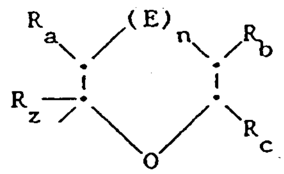

(U)

wherein n is one of the integers 0 or 1, E is oxygen or $CH_2$ and $R_z$ is hydrogen, $R_a$ is hydrogen, fluorine, chlorine, bromine, methyl or 2-tetrahydropyranyl and $R_b$ and $R_c$ are each independently hydrogen, halogen or methyl, and the remaining substituents are as defined in claim 1.

**6.** A composition according to claim 3 which comprises as active substance a compound of formula I, wherein $R_3$ is hydrogen, $C_1$-$C_6$alkyl, $C_2$-$C_6$alkenyl, a radical from the series phenyl and benzyl that is unsubstituted or mono- or poly-substituted by halogen, $C_1$-$C_3$alkyl, $C_1$-$C_3$alkoxy, $C_1$-$C_3$alkylthio, $C_1$-$C_3$haloalkyl, nitro or by cyano, or the group U is one of the groups

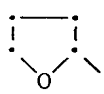 , 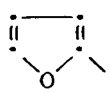 , 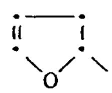 , 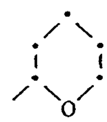 , 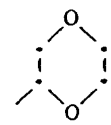 ,

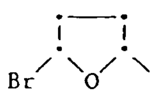 , 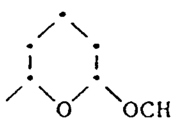 , 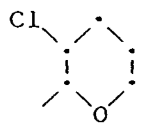 , 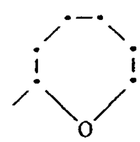 or 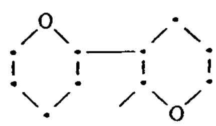

and the remaining substituents are as defined in claim 3.

**7.** A composition according to claim 6 which comprises as active substance a compound of formula I wherein $R_1$ is hydrogen, and $R_3$ is hydrogen, $C_1$-$C_4$alkyl, phenyl, benzyl, or one of the groups

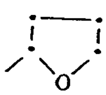 , 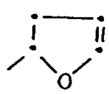 , 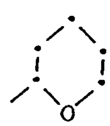 or 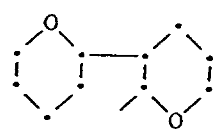 ,

and the remaining substituents are as defined in claim 6.

**8.** A composition according to claim 1 which comprises as active substance a compound of formula I wherein $R_2$ is methyl and the remaining substituents are as defined in any one of claims 1 to 7.

**9.** A composition according to claim 1 which comprises as active substance a compound of formula I wherein $R_2$ is ethyl and the remaining substituents are as defined in any one of claims 1 to 7.

**10.** A composition according to claim 1 which comprises as active substance a compound of formula I selected from the following group:
5-acetoxy-29-methoximino-milbemycin D,
5-(dimethyl-tert-butylsilyloxy)-29-hydroximino-milbemycin $A_4$,
29-hydroximino-milbemycin $A_4$,
29-methoximino-milbemycin $A_4$,
29-phenoximino-milbemycin $A_4$,
29-tetrahydropyran-2-yloximino-milbemycin $A_4$,
5,29-bis-hydroximino-milbemycin $A_4$.

**11.** A process for the preparation of a compound of formula I

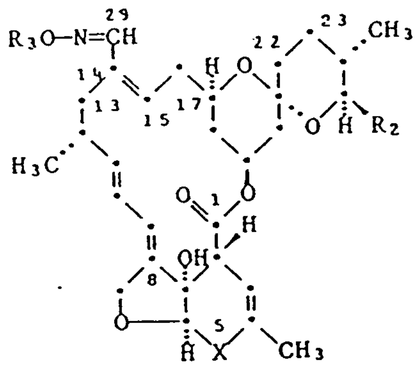

(I)

wherein

X is one of the groups $-CH(OR_1)-$, $-C(O)-$ or $-C(=N-OR)-$;

$R_1$ is hydrogen or an OH-protecting group;

R is hydrogen, an OH-protecting group, or an alkyl, cycloalkyl or acyl group; there being understood by OH-protecting group for the substituents R and $R_1$ the radical $R_4-C(O)-$ or $-Si(R_5)(R_6)(R_7)$ wherein $R_4$ is $C_1$-$C_{10}$alkyl, $C_1$-$C_{10}$haloalkyl or a group from the series phenyl and benzyl that is unsubstituted or substituted by halogen, $C_1$-$C_3$alkyl, $C_1$-$C_3$haloalkyl, $C_1$-$C_3$alkoxy, $C_1$-$C_3$haloalkoxy, cyano and/or by nitro, and $R_5$, $R_6$ and $R_7$ are each independently $C_1$-$C_4$alkyl, benzyl or phenyl;

$R_2$ is methyl, ethyl, isopropyl, sec-butyl or the group

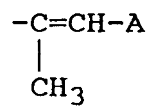

wherein A is methyl, ethyl or isopropyl; and

$R_3$ is hydrogen, $C_1$-$C_6$alkyl, $C_2$-$C_6$alkenyl, a radical from the series phenyl and benzyl that is unsubstituted or mono- or poly-substituted by halogen, $C_1$-$C_3$alkyl, $C_1$-$C_3$alkoxy, $C_1$-$C_3$alkylthio, $C_1$-$C_3$haloalkyl, nitro or by cyano or is the group U

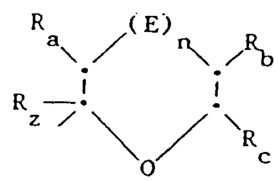

(U)

wherein n is one of the integers 0, 1, 2, 3, 4 or 5, E is oxygen or $-CH(R_d)-$ and $R_z$ is hydrogen, $R_a$ is hydrogen, halogen, $C_1$-$C_3$alkyl, $C_1$-$C_3$alkoxy or 2-tetrahydropyranyl and $R_b$, $R_c$ and $R_d$ are each independently hydrogen, halogen, $C_1$-$C_3$alkyl or $C_1$-$C_3$alkoxy, which group U may also be in unsaturated form,

which comprises reacting a 29-oxo-milbemycin of formula II

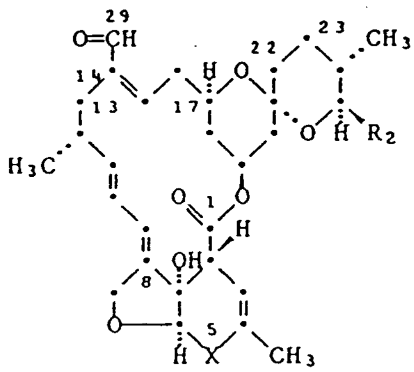

(II)

with a primary oxamine of formula III

$H_2N\text{-}OR_3$ (III)

or a salt thereof and, if desired, further reacting the resulting compound of formula I, if $R_3$ is hydrogen, with a halide of formula IV

$R_3'$ -Hal (IV)

in which formulae $R_2$ is methyl, ethyl, isopropyl or sec-butyl or the group

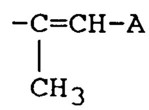

wherein A is methyl, ethyl or isopropyl and the substituent $R_3$ is as defined for formula I, X is -C($OR_1$) wherein $R_1$ is hydrogen or one of the OH-protecting groups mentioned under formula I, and Hal is halogen, and $R_3'$ has all the meanings of $R_3$ except hydrogen, in which process generally either a compound of formula II that contains a free 5-hydroxy group is used as starting material and, if desired, the resulting product is subsequently acylated or silylated at the 5-O atom, or a compound of formula II already silylated or acylated in the 5-position is used as starting material and, if desired, the protecting group is removed; or, if a compound of formula I wherein X is the group -C(O)- is the aim of the preparation, there is used as starting material a compound of formula II that contains a free 5-hydroxy group, which is treated with a reagent suitable for oxidation; or, alternatively, if a compound of formula I is desired wherein X is the group -C( = N-OR)- in which R is as defined for formula I, there is used as starting material a compound of formula I wherein X is -C(O)-, which is reacted with hydroxylamine or a salt thereof and, if desired, the substituent R is introduced subsequently, or the reaction is carried out with a compound of formula $NH_2$-OR or a salt thereof.

**12.** A process according to claim 11 for the preparation of a compound of formula I

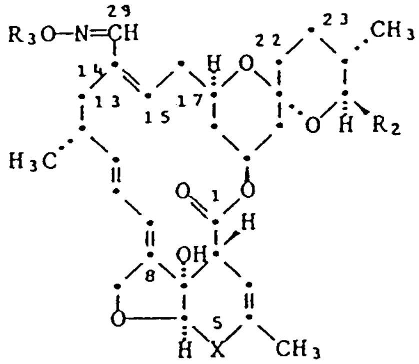

(I)

wherein

X is one of the groups -CH($OR_1$)-, -C(O)- or -C(=N-OR)-;

$R_1$ is hydrogen or an OH-protecting group;

R is hydrogen, an OH-protecting group, or an alkyl or cycloalkyl group; there being understood by OH-protecting group for the substituents R and $R_1$ the radical $R_4$-C(O)- or -Si($R_5$)($R_6$)($R_7$) wherein $R_4$ is $C_1$-$C_{10}$alkyl, $C_1$-$C_{10}$haloalkyl or a group from the series phenyl and benzyl that is unsubstituted or substituted by halogen, $C_1$-$C_3$alkyl, $C_1$-$C_3$haloalkyl, $C_1$-$C_3$alkoxy, $C_1$-$C_3$haloalkoxy, cyano and/or by nitro, and $R_5$, $R_6$ and $R_7$ are each independently $C_1$-$C_4$alkyl, benzyl or phenyl;

$R_2$ is methyl, ethyl, isopropyl or sec-butyl; and

$R_3$ is hydrogen, $C_1$-$C_6$alkyl, $C_2$-$C_6$alkenyl, a radical from the series phenyl and benzyl that is unsubstituted or mono- or poly-substituted by halogen, $C_1$-$C_3$alkyl, $C_1$-$C_3$alkoxy, $C_1$-$C_3$alkylthio, $C_1$-$C_3$haloalkyl, nitro or by cyano or is the group U

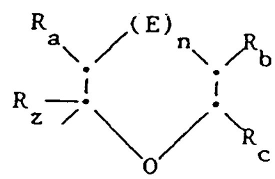

(U)

wherein n is one of the integers 0, 1, 2, 3, 4 or 5, E is oxygen or -CH($R_d$)- and $R_z$ is hydrogen, $R_a$ is hydrogen, halogen, $C_1$-$C_3$alkyl, $C_1$-$C_3$alkoxy or 2-tetrahydropyranyl and $R_b$, $R_c$ and $R_d$ are each independently hydrogen, halogen, $C_1$-$C_3$alkyl or $C_1$-$C_3$alkoxy, which group U may also be in unsaturated form,

which comprises reacting a 29-oxo-milbemycin of formula II

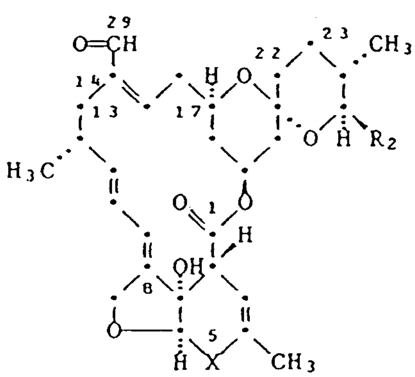

(II)

with a primary oxamine of formula III

$H_2N\text{-}OR_3$ (III)

or a salt thereof and, if desired, further reacting the resulting compound of formula I, if $R_3$ is hydrogen, with a halide of formula IV

$R_3'$ -Hal (IV)

in which formulae $R_2$ is methyl, ethyl, isopropyl or sec-butyl and the substituent $R_3$ is as defined for formula I, X is -C($OR_1$)- wherein $R_1$ is hydrogen or one of the OH-protecting groups mentioned under formula I, and Hal is halogen, and $R_3'$ has all the meanings of $R_3$ except hydrogen, in which process generally either a compound of formula II that contains a free 5-hydroxy group is used as starting material and, if desired, the resulting product is subsequently acylated or silylated at the 5-O atom, or a compound of formula II already silylated or acylated in the 5-position is used as starting material and, if desired, the protecting group is removed; or, if a compound of formula I wherein X is the group -C(O)- is the aim of the preparation, there is used as starting material a compound of formula II that contains a free 5-hydroxy group, which is treated with a reagent suitable for oxidation; or, alternatively, if a compound of formula I is desired wherein X is the group -C(=N-OR)- in which R is as defined for formula I, there is used as starting material a compound of formula I wherein X is -C(O)-, which is reacted with hydroxylamine or a salt thereof and, if desired, the substituent R is introduced subsequently, or the reaction is carried out with a compound of formula $NH_2$-OR or a salt thereof.

**13.** A process for the preparation of a compound of formula I according to claim 1,

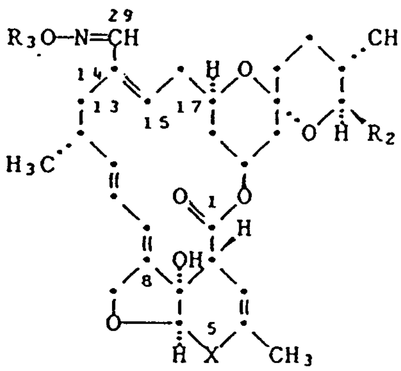

(I)

wherein

X is the group -CH($OR_1$)-;

$R_1$ is hydrogen or an OH-protecting group, there being understood by OH-protecting group the radical $R_4$-C(O)- or -Si($R_5$)($R_6$)($R_7$) wherein $R_4$ is $C_1$-$C_{10}$alkyl, $C_1$-$C_{10}$-haloalkyl or a group from the series phenyl and benzyl that is unsubstituted or substituted by halogen, $C_1$-$C_3$alkyl, $C_1$-$C_3$haloalkyl, $C_1$-$C_3$alkoxy, $C_1$-$C_3$haloalkoxy, cyano and/or by nitro, and $R_5$, $R_6$ and $R_7$ are each independently $C_1$-$C_4$alkyl, benzyl or phenyl;

$R_2$ is methyl, ethyl, isopropyl or sec-butyl; and

$R_3$ is hydrogen, $C_1$-$C_6$alkyl, $C_2$-$C_6$alkenyl, a radical from the series phenyl and benzyl that is unsubstituted or mono- or poly-substituted by halogen, $C_1$-$C_3$alkyl, $C_1$-$C_3$alkoxy, $C_1$-$C_3$alkylthio, $C_1$-$C_3$haloalkyl, nitro or by cyano or is the group U

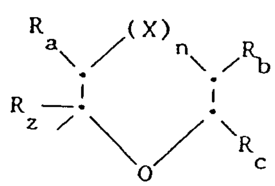

(U)

wherein n is one of the integers 0, 1, 2, 3, 4 or 5, X is oxygen or -CH($R_d$)- and $R_z$ is

hydrogen, $R_a$ is hydrogen, halogen, $C_1$-$C_3$alkyl, $C_1$-$C_3$alkoxy or 2-tetrahydropyranyl and $R_b$, $R_c$ and $R_d$ are each independently hydrogen, halogen, $C_1$-$C_3$alkyl or $C_1$-$C_3$alkoxy, which group U may also be in unsaturated form,

which comprises reacting a 29-oxo-milbemycin of formula II

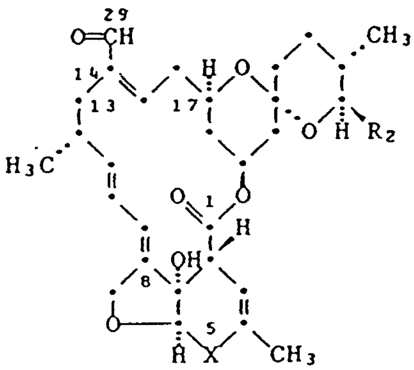

(II)

with a primary oxamine of formula III

$H_2N$-$OR_3$ (III)

or a salt thereof and, if desired, further reacting the resulting compound of formula I, if $R_3$ is hydrogen, with a halide of formula IV

$R_3'$-Hal (IV)

in which formulae $R_2$ is methyl, ethyl, isopropyl or sec-butyl and the substituents $R_1$ and $R_3$ are as defined for formula I, Hal is halogen, and $R_3'$ has all the meanings of $R_3$ except hydrogen, in which process either a compound of formula II that contains a free 5-hydroxy group is used as starting material and, if desired, the resulting product is subsequently acylated or silylated at the 5-O atom, or a compound of formula II already silylated or acylated in the 5-position is used as starting material and, if desired, the protecting group is removed.

**14.** A method of controlling parasites of plants or harmful insects, which comprises applying to the parasite, harmful insect or its locus a parasiticidally or insecticidally effective amount of a compound of formula I

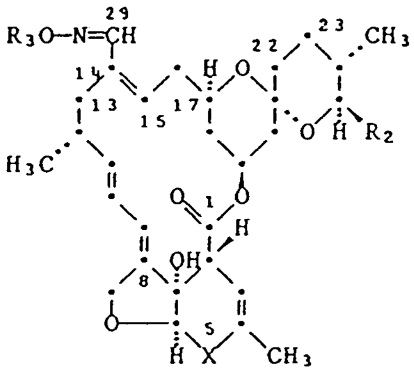

(I)

wherein

X is one of the groups -CH($OR_1$)-, -C(O)- or -C(=N-OR)-;

$R_1$ is hydrogen or an OH-protecting group;

R is hydrogen, an OH-protecting group, or an alkyl or cycloalkyl group; there being understood by OH-protecting group for the substituents R and $R_1$ the radical $R_4$-C(O)- or -Si($R_5$)($R_6$)($R_7$) wherein $R_4$ is $C_1$-$C_{10}$alkyl, $C_1$-$C_{10}$haloalkyl or a group from the series phenyl and benzyl that

is unsubstituted or substituted by halogen, $C_1$-$C_3$alkyl, $C_1$-$C_3$haloalkyl, $C_1$-$C_3$alkoxy, $C_1$-$C_3$haloalkoxy, cyano and/or by nitro, and $R_5$, $R_6$ and $R_7$ are each independently $C_1$-$C_4$alkyl, benzyl or phenyl;

$R_2$ is methyl, ethyl, isopropyl, sec-butyl or the group

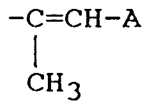

wherein A is methyl, ethyl or isopropyl; and

$R_3$ is hydrogen, $C_1$-$C_6$alkyl, $C_2$-$C_6$alkenyl, a radical from the series phenyl and benzyl that is unsubstituted or mono- or poly-substituted by halogen, $C_1$-$C_3$alkyl, $C_1$-$C_3$alkoxy, $C_1$-$C_3$alkylthio, $C_1$-$C_3$haloalkyl, nitro or by cyano or is the group U

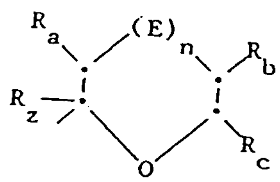

(U)

wherein n is one of the integers 0, 1, 2, 3, 4 or 5, E is oxygen or -CH($R_d$)- and $R_z$ is hydrogen, $R_a$ is hydrogen, halogen, $C_1$-$C_3$alkyl, $C_1$-$C_3$alkoxy or 2-tetrahydropyranyl and $R_b$, $R_c$ and $R_d$ are each independently hydrogen, halogen, $C_1$-$C_3$alkyl or $C_1$-$C_3$alkoxy, which group U may also be in unsaturated form.

**15.** A method of controlling parasites of plants or harmful insects according to claim 14, which comprises applying to the parasite, harmful insect or its locus a parasiticidally or insecticidally effective amount of a compound of formula I

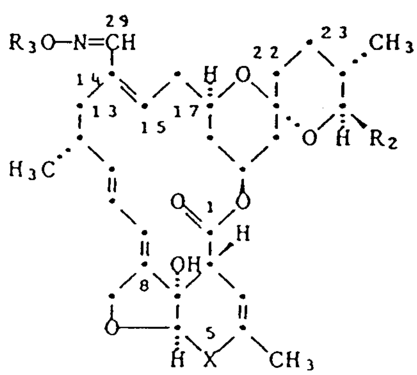

(I)

wherein

X is one of the groups -CH($OR_1$)-, -C(O)- or -C(=N-OR)-;

$R_1$ is hydrogen or an OH-protecting group;

R is hydrogen, an OH-protecting group, or an alkyl or cycloalkyl group; there being understood by OH-protecting group for the substituents R and $R_1$ the radical $R_4$-C(O)- or -Si($R_5$)($R_6$)($R_7$) wherein $R_4$ is $C_1$-$C_{10}$alkyl, $C_1$-$C_{10}$haloalkyl or a group from the series phenyl and benzyl that is unsubstituted or substituted by halogen, $C_1$-$C_3$alkyl, $C_1$-$C_3$haloalkyl, $C_1$-$C_3$alkoxy, $C_1$-$C_3$haloalkoxy, cyano and/or by nitro, and $R_5$, $R_6$ and $R_7$ are each independently $C_1$-$C_4$alkyl, benzyl or phenyl;

$R_2$ is methyl, ethyl, isopropyl or sec-butyl;

and

$R_3$ is hydrogen, $C_1$-$C_6$alkyl, $C_2$-$C_6$alkenyl, a radical from the series phenyl and benzyl that is unsubstituted or mono- or poly-substituted by halogen, $C_1$-$C_3$alkyl, $C_1$-$C_3$alkoxy, $C_1$-$C_3$alkylthio, $C_1$-$C_3$haloalkyl, nitro or by cyano or is the group U

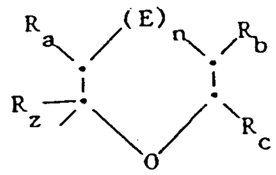

(U)

wherein n is one of the integers 0, 1, 2, 3, 4 or 5, E is oxygen or -CH($R_d$)- and $R_z$ is hydrogen, $R_a$ is hydrogen, halogen, $C_1$-$C_3$alkyl, $C_1$-$C_3$alkoxy or 2-tetrahydropyranyl and $R_b$, $R_c$ and $R_d$ are each independently hydrogen, halogen, $C_1$-$C_3$alkyl or $C_1$-$C_3$alkoxy, which group U may also be in unsaturated form.

**16.** A method of controlling parasites of plants or harmful insects according to claim 14, which comprises applying to the parasite, harmful insect or its locus a parasiticidally or insecticidally effective amount of a compound of formula I

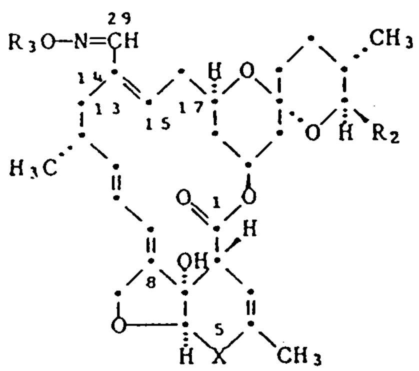

(I)

wherein

X is the group -CH($OR_1$)-;

$R_1$ is hydrogen or an OH-protecting group, there being understood by OH-protecting group the radical $R_4$-C(O)-or -Si($R_5$)($R_6$)($R_7$) wherein $R_4$ is $C_1$-$C_{10}$alkyl, $C_1$-$C_{10}$-haloalkyl or a group from the series phenyl and benzyl that is unsubstituted or substituted by halogen, $C_1$-$C_3$alkyl, $C_1$-$C_3$haloalkyl, $C_1$-$C_3$alkoxy, $C_1$-$C_3$haloalkoxy, cyano and/or by nitro, and $R_5$, $R_6$ and $R_7$ are each independently $C_1$-$C_4$alkyl, benzyl or phenyl;

$R_2$ is methyl, ethyl, isopropyl or sec-butyl; and

$R_3$ is hydrogen, $C_1$-$C_6$alkyl, $C_2$-$C_6$alkenyl, a radical from the series phenyl and benzyl that is unsubstituted or mono- or poly-substituted by halogen, $C_1$-$C_3$alkyl, $C_1$-$C_3$alkoxy, $C_1$-$C_3$alkylthio, $C_1$-$C_3$haloalkyl, nitro or by cyano or is the group U

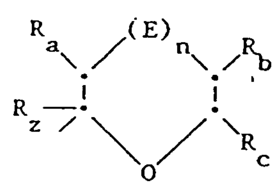

(U)

wherein n is one of the integers 0, 1, 2, 3, 4 or 5, E is oxygen or -CH($R_d$)- and $R_z$ is hydrogen, $R_a$ is hydrogen, halogen, $C_1$-$C_3$alkyl, $C_1$-$C_3$alkoxy or 2-tetrahydropyranyl and $R_b$, $R_c$ and $R_d$ are each independently hydrogen, halogen, $C_1$-$C_3$alkyl or $C_1$-$C_3$alkoxy, which group U may also be in unsaturated form.

**17.** A method according to claim 14, wherein the parasites are nematodes.

**18.** The use of a compound of formula I according to claim 1 for the preparation of a composition for controlling ecto- and endo-parasites of productive livestock or for controlling harmful insects in all development stages.

**Revendications**

**Revendications pour les Etats contractants suivants : AT, BE, CH, DE, FR, GB, GR, IT, LI, LU, NL, SE**

**1.** Composés de formule I

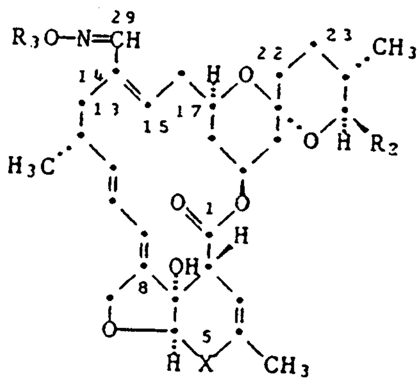

(I)

dans laquelle

X représente un des groupes -CH($OR_1$)-, -C(O)- ou -C(=N-OR)-;

$R_1$ représente un hydrogène ou un groupe protégeant OH;

R représente un hydrogène, un groupe protecteur de OH, un groupe alkyle, cyclo-alkyle; où par groupe protecteur de OH on entend, pour les substituants R et $R_1$, les restes $R_4$-C(O)- ou -Si-($R_5$)($R_6$)($R_7$), où $R_4$ représente un alkyle en $C_1$-$C_{10}$, un halo-alkyle en $C_1$-$C_{10}$, ou un groupe de la série du phényle et du benzyle, non substitué ou substitué par un halogène, un alkyle en $C_1$-$C_3$, un halo-alkyle en $C_1$-$C_3$, un alcoxy en $C_1$-$C_3$, un halo-alcoxy en $C_1$-$C_3$, un cyano et/ou un nitro; $R_5$, $R_6$, $R_7$, indépendamment les uns des autres, représentent un alkyle en $C_1$-$C_4$, un benzyle ou un phényle;

$R_2$ représente un méthyle, un éthyle, un isopropyle, un butyle secondaire ou le groupe

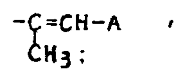

où A représente un méthyle, un éthyle ou un isopropyle, et

$R_3$ représente un hydrogène, un alkyle en $C_1$-$C_6$, un alcényle en $C_2$-$C_6$, un reste de la série du phényle et du benzyle, non substitué ou substitué une fois ou plusieurs fois par un halogène, un alkyle en $C_1$-$C_3$, un alcoxy en $C_1$-$C_3$ : un alkylthio en $C_1$-$C_3$, un halo-alkyle en $C_1$-$C_3$, un nitro ou un cyano, ou représente le groupe U

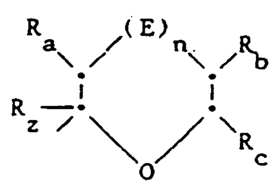

(U)

où n représente un des nombres 0, 1, 2, 3, 4 ou 5, E représente un oxygène ou -CH($R_d$)- et $R_z$ représente un hydrogène, $R_a$ représente un hydrogène, un halogène, un alkyle en $C_1$-$C_3$, un alcoxy en $C_1$-$C_3$ ou un 2-tétrahydropyranyle et $R_b$, $R_c$ et $R_d$, indépendamment les uns des autres, représentent un hydrogène, un halogène, un alkyle en $C_1$-$C_3$ ou un alcoxy en $C_1$-$C_3$,

où le groupe U peut aussi se présenter sous une forme insaturée.

**2.** Composés de formule I selon la revendication I, où $R_2$ représente un méthyle, un éthyle, un isopropyle ou un butyle secondaire.

**3.** Composés de formule I selon la revendication 2, où
X représente le groupe $-CH(OR_1)-$;
$R_1$ représente un hydrogène ou un groupe protégeant OH; où par groupe protecteur de OH on entend les restes $R_4-C(O)-$ ou $-Si(R_5)(R_6)(R_7)$, où $R_4$ représente un alkyle en $C_1$-$C_{10}$, un halo-alkyle en $C_1$-$C_{10}$, ou un groupe de la série du phényle et du benzyle, non substitué ou substitué par un halogène, un alkyle en $C_1$-$C_3$, un halo-alkyle en $C_1$-$C_3$, un alcoxy en $C_1$-$C_3$, un halo-alcoxy en $C_1$-$C_3$, un cyano et/ou un nitro; $R_5$, $R_6$, $R_7$, indépendamment les uns des autres, représentent un alkyle en $C_1$-$C_4$, un benzyle ou un phényle .

**4.** Composés de formule I selon la revendication 2, où X représente un des groupes $-CH(OR_1)-$, -(CO)- ou -C(=N-OH)-, où $R_1$ représente un hydrogène, $R_4-C(O)-$ ou $-Si(R_5)(R_6)(R_7)$, où $R_4$ représente un alkyle en $C_1$-$C_{10}$, un halo-alkyle en $C_1$-$C_{10}$, ou un reste de la série du phényle et du benzyle non substitué ou substitué par un halogène, un alkyle en $C_1$-$C_3$, un halo-alkyle en $C_1$-$C_3$, un alcoxy en $C_1$-$C_3$, un halo-alcoxy en $C_1$-$C_3$, un cyano et/ou un nitro; $R_5$, $R_6$, $R_7$, indépendamment les uns des autres, représentent un alkyle en $C_1$-$C_4$, un benzyle ou un phényle; $R_2$ représente un méthyle, un éthyle, un isopropyle ou un butyle secondaire; et $R_3$ représente un hydrogène, un alkyle en $C_1$-$C_4$, un reste de la série du phényle et du benzyle, non substitué ou substitué de une à trois fois par un halogène, un alkyle en $C_1$-$C_2$, un alcoxy en $C_1$-$C_2$, un alkylthio en $C_1$-$C_2$, un halo-alkyle en $C_1$-$C_2$, un nitro ou un cyano, ou représente le groupe U

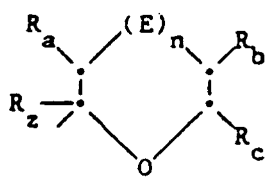
(U)

où n représente un des nombres 0 ou 1, E représente un oxygène ou $CH_2$ et $R_z$ représente un hydrogène, $R_a$ représente un hydrogène, un fluor, un chlore, un brome, un méthyle ou un 2-tétrahydropyranyle et $R_b$ et $R_c$, indépendamment l'un de l'autre représentent un hydrogène, un halogène ou un méthyle.

**5.** Composés de formule I selon la revendication I, où X représente le groupe $-CH(OR_1)-$ , où $R_1$ représente un hydrogène, $R_4-C(O)-$ ou $-Si(R_5)(R_6)(R_7)$, où $R_4$ représente un alkyle en $C_1$-$C_{10}$, un halo-alkyle en $C_1$-$C_{10}$, ou un reste de la série du phényle et du benzyle non substitué ou substitué par un halogène, un alkyle en $C_1$-$C_3$, un halo-alkyle en $C_1$-$C_3$, un alcoxy en $C_1$-$C_3$, un halo-alcoxy en $C_1$-$C_3$, un cyano et/ou un nitro; $R_5$, $R_6$, $R_7$, indépendamment les uns des autres, représentent un alkyle en $C_1$-$C_4$, un benzyle ou un phényle; $R_2$ représente un méthyle, un éthyle, un isopropyle ou un butyle secondaire; et $R_3$ représente un hydrogène, un alkyle en $C_1$-$C_4$, un reste de la série du phényle et du benzyle, non substitué ou substitué de une à trois fois par un halogène, un alkyle en $C_1$-$C_2$, un alcoxy en $C_1$-$C_2$, un alkylthio en $C_1$-$C_2$, un halo-alkyle en $C_1$-$C_2$, un nitro ou un cyano, ou représente le groupe U

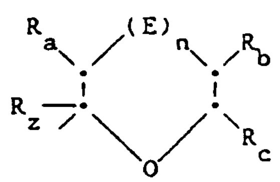
(U)

où n représente un des nombres 0 ou 1, E représente un oxygène ou $CH_2$ et $R_z$ représente un

hydrogène, $R_a$ représente un hydrogène, un fluor, un chlore, un brome, un méthyle ou un 2-tétrahydropyranyle et $R_b$ et $R_c$, indépendamment l'un de l'autre représentent un hydrogène, un halogène ou un méthyle.

**6.** Composés de formule I selon la revendication 3, où

$R_3$ représente un hydrogène, un alkyle en $C_1$-$C_6$, un alcényle en $C_2$-$C_6$, un reste de la série du phényle et du benzyle, non substitué ou substitué une ou plusieurs fois par un halogène, un alkyle en $C_1$-$C_3$, un alcoxy en $C_1$-$C_3$, un alkylthio en $C_1$-$C_3$, un halo-alkyle en $C_1$-$C_3$, un nitro ou un cyano, ou représente le groupe U pour un des groupes

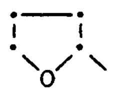 , 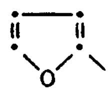 , 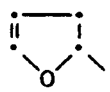 , 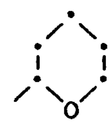 , 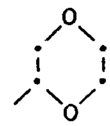 ,

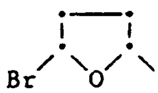 , 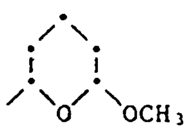 , 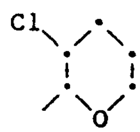 , 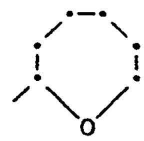 ou 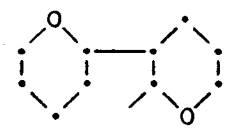

**7.** Composés de formule I selon la revendication 6, où $R_1$ représente un hydrogène; et $R_3$ représente un hydrogène, un alkyle en $C_1$-$C_4$, un phényle, un benzyle ou un des groupes

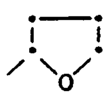 , 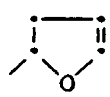 , 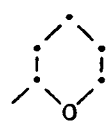 ou 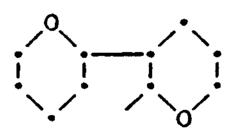

**8.** Composés de formule I selon l'une des revendications 1 à 7, où $R_2$ représente un méthyle.

**9.** Composés de formule I selon l'une des revendications 1 à 7, où $R_2$ représente un éthyle.

**10.** Composé de formule I selon la revendication 1, choisi dans la série :
5-acétoxy-29-méthoximino-milbémycine D,
5-(diméthyl-tert.-butyl-silyloxy)-29-hydroximino-milbémycine $A_4$,
29-hydroximino-milbémycine $A_4$,
29-méthoximino-milbémycine $A_4$:
29-phénoximino-milbémycine $A_4$,
29- tétrahydropyranne-2-yl -oximino-milbémycine $A_4$:
5,29-bis-hydroximino-milbémycine $A_4$.

**11.** Procédé pour préparer un composé de formule I selon la revendication 1,caractérisé en ce que l'on fait réagir une 29-oxo-milbémycine de formule II

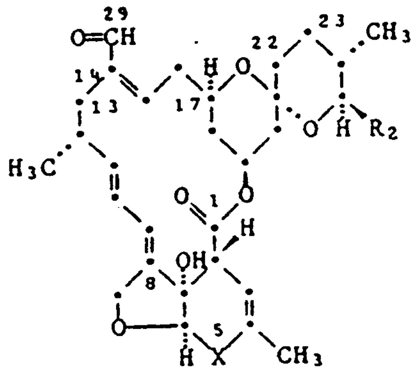

(II)

avec une oxamine primaire de formule III

$H_2N\text{-}OR_3$ (III)

ou un de ses sels, et on fait réagir ensuite, si on le désire, le composé résultant de formule I, si $R_3$ représente un hydrogène, avec un halogénure de formule IV

$R_3'$ -Hal (IV)

où $R_2$ représente un méthyle, un éthyle, un isopropyle ou un butyle secondaire, ou le groupe

$$\begin{array}{l}-C{=}CH{-}A\ ,\\ \ \ |\\ \ CH_3\end{array}$$

où A représente un méthyle, un éthyle ou un isopropyle et le substituant $R_3$ a les significations données pour la formule I, X représente -C($OR_1$), où $R_1$ représente un hydrogène ou un des groupes protecteurs de OH indiqués à la revendication 1, et Hal représente un halogène, et $R'_3$ a toutes les significations de $R_3$, à l'exception de l'hydrogène, de telle sorte que l'on part en général de composés de formule II qui soit comportent un groupe 5 hydroxy libre et, si on le désire, on acyle ou silyle ensuite les produits résultants sur l'atome 5-O, ou soit on part d'un composé de formule II déjà silylé ou acylé dans la position 5, et l'on sépare, si on le désire, le groupe protecteur; ou, si le but de la préparation est d'obtenir des composés de formule I, où X représente le groupe -C(O)-, on part de composés de formule II qui comportent un groupe 5-hydroxy libre, et on traite ceux-ci avec un réactif approprié pour une oxydation; ou, si l'on désire des composés de formule I, où X représente le groupe -C( = N-OR)-, où R a la signification donnée pour la formule I, on part d'un composé de formule I, dans lequel X représente -C(O)-, et on fait réagir celui-ci avec de l'hydroxylamine ou un de ses sels, et ensuite, si on le désire, on introduit le substituant R, ou on effectue la réaction avec un composé de formule $NH_2$-OR, ou un de ses sels.

**12.** Procédé pour préparer un composé de formule I selon la revendication 2, caractérisé en ce que l'on fait réagir une 29-oxo-milbémycine de formule II

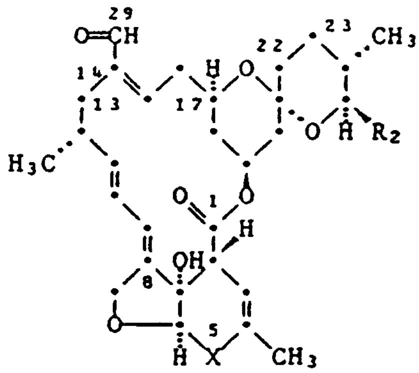

(II)

avec une oxamine primaire de formule III

$H_2N\text{-}OR_3$ (III)

ou un de ses sels, et, si on le désire, on fait ensuite réagir le composé résultant de formule I, si $R_3$ représente un hydrogène, avec un halogénure de formule IV

$R_3'$ -Hal (IV)

où $R_2$ représente un méthyle, un éthyle, un isopropyle ou un butyle secondaire et le substituant $R_3$ a les significations données pour la formule I, X représente $-C(OR_1)-$, où $R_1$ représente un hydrogène ou un groupe protecteur de OH indiqué à la formule 2, et Hal représente un halogène, et $R'_3$ a toutes les significations de $R_3$, à l'exception de l'hydrogène, de telle sorte que l'on part en général de composés de formule II qui soit comportent un groupe 5 hydroxy libre et, si on le désire, on acyle ou silyle ensuite les produits résultants sur l'atome 5-O, ou soit on part d'un composé de formule II déjà silylé ou acylé dans la position 5, et l'on sépare, si on le désire, le groupe protecteur; ou, si le but de la préparation est d'obtenir des composés de formule I, où X représente le groupe -C(O)-, on part de composés de formule II qui comportent un groupe 5-hydroxy libre, et on traite ceux-ci avec un réactif approprié pour une oxydation; ou, si l'on désire des composés de formule I, où X représente le groupe -C( = N-OR)-, où R a la signification donnée pour la formule I, on part d'un composé de formule I, dans lequel X représente -C(O)-, et on fait réagir celui-ci avec de l'hydroxylamine ou un de ses sels, et ensuite, si on le désire, on introduit le substituant R, ou on effectue la réaction avec un composé de formule $NH_2\text{-}OR$, ou un de ses sels.

**13.** Procédé pour préparer un composé de formule I selon la revendication 3,caractérisé en ce que l'on fait réagir une 29-oxo-milbémycine de formule II

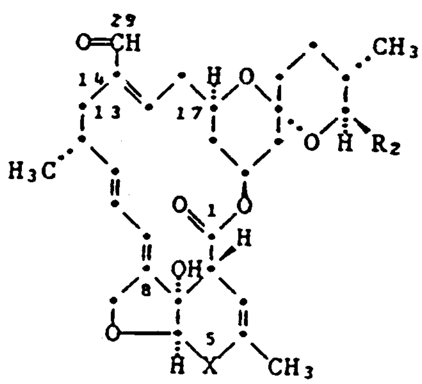

(II)

avec une oxamine primaire de formule III

$H_2N\text{-}OR_3$ (III)

ou un de ses sels, et, si onle désire, on fait ensuite réagir le composé résultant de formule I, si $R_3$ représente un hydrogène, avec un halogénure de formule IV

$R_3'$ -Hal (IV)

où $R_2$ représente un méthyle, un éthyle, un isopropyle ou un butyle secondaire, et les substituants $R_1$ et $R_3$ ont les significations données pour la formule I, Hal représente un halogène, et $R'_3$ a toutes les significations données pour $R_3$, à l'exception de l'hydrogène, et on part soit de composés de formule II qui comportent un groupe 5-hydroxy libre et, si on le désire, on acyle ou on silyle ensuite les produits résultants sur l'atome 5-O, soit d'un composé de formule II déjà sylilé ou acylé en position 5, et on sépare , si on le désire, le groupe de protection.

**14.** Moyen pour lutter contre les ecto et endoparasites sur les animaux utiles ou pour lutter contre les insectes nuisibles, caractérisé en ce que, en plus des matières supports et des agents de répartition habituels, il comporte un composé de formule I selon la revendication 1.

**15.** Moyen pour lutter contre les ecto et endoparasites sur les animaux utiles, ou pour lutter contre les insectes nuisibles, selon la revendication 14, caractérisé en ce que, en plus des matières supports et des agents de répartition habituels, il comporte un composé de formule I selon la revendication 2.

**16.** Moyen pour lutter contre les ecto et endoparasites sur les animaux utiles ou pour lutter contre les insectes nuisibles, selon la revendication 14, caractérisé en ce que, en plus des matières supports et des agents de répartition habituels il comporte un composé de formule I selon la revendication 3.

**17.** Moyen selon la revendication 14, caractérisé en ce que, comme matière active, il comporte un composé de formule I selon l'une des revendications 4 à 10.

**18.** Composé de formule I selon l'une des revendications 1 à 10 pour utilisation dans un procédé pour lutter contre les parasites sur les animaux.

**19.** Procédé pour lutter contre les parasites nuisibles aux plantes, caractérisé en ce que l'on applique une quantité parasiticide ou insecticide efficace d'un composé de formule I selon l'une des revendications 1 à 10, sur les parasites ou leur biotope.

**20.** Utilisation d'un composé de formule I selon la revendication 1 pour préparer un moyen pour lutter contre les ecto et endoparasites sur les animaux utiles, ou contre les insectes nuisibles à tous leurs stades de développement.

**21.** Utilisation selon la revendication 20 d'un composé de formule I conforme à l'une des revendications 4 à 10 pour préparer un moyen de lutte contre les ecto et endoparasites sur les animaux utiles, ou contre les insectes nuisibles à tous les stades de leur développement.

**Revendications pour l'Etat contractant suivant : ES**

**1.** Moyen pour lutter contre des ecto et endoparasites sur les animaux utiles ou pour lutter contre les insectes nuisibles, caractérisé en ce que, en plus des matières support et des agents de répartition habituels, il comporte un composé de formule I :

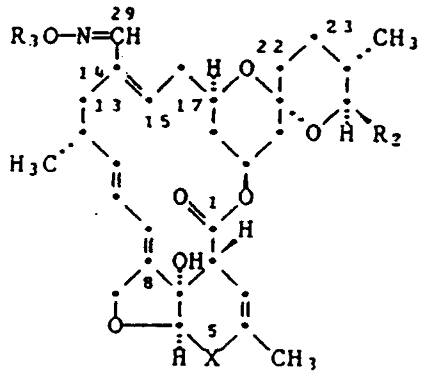

(I),

dans laquelle

X représente un des groupes -CH($OR_1$)-, -C(O)- ou -C(= N-OR)-;

$R_1$ représente un hydrogène ou un groupe protégeant OH;

R représente un hydrogène, un groupe protecteur de OH, un groupe alkyle, cyclo-alkyle; où par groupe protecteur de OH on entend, pour les substituants R et $R_1$, les restes $R_4$-C(O)- ou -Si-($R_5$)($R_6$)($R_7$), où $R_4$ représente un alkyle en $C_1$-$C_{10}$, un halo-alkyle en $C_1$-$C_{10}$, ou un groupe de la série du phényle et du benzyle, non substitué ou substitué par un halogène, un alkyle en $C_1$-$C_3$, un halo-alkyle en $C_1$-$C_3$, un alcoxy en $C_1$-$C_3$, un halo-alcoxy en $C_1$-$C_3$, un cyano et/ou un nitro; $R_5$, $R_6$, $R_7$, indépendamment les uns des autres, représentent un alkyle en $C_1$-$C_4$, un benzyle ou un phényle;

$R_2$ représente un méthyle, un éthyle, un isopropyle, un butyle secondaire ou le groupe

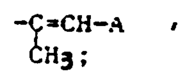

où A représente un méthyle, un éthyle ou un isopropyle, et

$R_3$ représente un hydrogène, un alkyle en $C_1$-$C_6$, un alcényle en $C_2$-$C_6$, un reste de la série du phényle et du benzyle, non substitué ou substitué une fois ou plusieurs fois par un halogène, un alkyle en $C_1$-$C_3$, un alcoxy en $C_1$-$C_3$ , un alkylthio en $C_1$-$C_3$, un halo-alkyle en $C_1$-$C_3$, un nitro ou un cyano, ou représente le groupe U

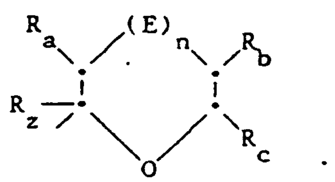

(U)

où n représente un des nombres 0, 1, 2, 3, 4 ou 5, E représente un oxygène ou -CH($R_d$)- et $R_z$ représente un hydrogène, $R_a$ représente un hydrogène, un halogène, un alkyle en $C_1$-$C_3$, un alcoxy en $C_1$-$C_3$ ou un 2-tétrahydropyranyle et $R_b$, $R_c$ et $R_d$, indépendamment les uns des autres, représentent un hydrogène, un halogène, un alkyle en $C_1$-$C_3$ ou un alcoxy en $C_1$-$C_3$, où le groupe U peut aussi se présenter sous une forme insaturée.

2. Moyen selon la revendication 1, caractérisé en ce que, comme substance active, il comporte un composé de formule I, où $R_2$ représente un méthyle, un éthyle, un isopropyle ou un butyle secondaire, et les substances restantes sont définies comme à la revendication 1.

3. Moyen selon la revendication 2, caractérisé en ce que, comme substance active, il comporte un composé de formule I, où $R_2$ représente un méthyle, un éthyle, un isopropyle ou un butyle secondaire;

X représente le groupe -CH($OR_1$)-;

$R_1$ représente un hydrogène ou un groupe protégeant OH; où par groupe protecteur de OH on

entend les restes $R_4$-C(O)- ou -Si($R_5$)($R_6$)($R_7$), où $R_4$ représente un alkyle en $C_1$-$C_{10}$, un halo-alkyle en $C_1$-$C_{10}$, ou un groupe de la série du phényle et du benzyle, non substitué ou substitué par un halogène, un alkyle en $C_1$-$C_3$, un halo-alkyle en $C_1$-$C_3$, un alcoxy en $C_1$-$C_3$, un halo-alcoxy en $C_1$-$C_3$, un cyano et/ou un nitro; $R_5$, $R_6$, $R_7$, indépendamment les uns des autres, représentent un alkyle en $C_1$-$C_4$, un benzyle ou un phényle, et les substituants restants sont définis comme à la revendication 1.

**4.** Moyen selon la revendication 2, caractérisé en ce que, comme substance active, il comporte un composé de formule I , où
X représente un des groupes -CH($OR_1$)-, -(CO)- ou -C(=N-OH)-, où $R_1$ représente un hydrogène, $R_4$-C(O)- ou -Si($R_5$)($R_6$)($R_7$), où $R_4$ représente un alkyle en $C_1$-$C_{10}$, un halo-alkyle en $C_1$-$C_{10}$, ou un reste de la série du phényle et du benzyle non substitué ou substitué par un halogène, un alkyle en $C_1$-$C_3$, un halo-alkyle en $C_1$-$C_3$, un alcoxy en $C_1$-$C_3$, un halo-alcoxy en $C_1$-$C_3$, un cyano et/ou un nitro; $R_5$, $R_6$, $R_7$, indépendamment les uns des autres, représentent un alkyle en $C_1$-$C_4$, un benzyle ou un phényle; $R_2$ représente un méthyle, un éthyle, un isopropyle ou un butyle secondaire; et $R_3$ représente un hydrogène, un alkyle en $C_1$-$C_4$, un reste de la série du phényle et du benzyle, non substitué ou substitué de une à trois fois par un halogène, un alkyle en $C_1$-$C_2$, un alcoxy en $C_1$-$C_2$, un alkylthio en $C_1$-$C_2$, un halo-alkyle en $C_1$-$C_2$, un nitro ou un cyano, ou représente le groupe U

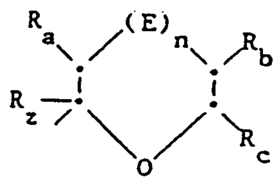

(U)

où n représente un des nombres 0 ou 1, E représente un oxygène ou $CH_2$ et $R_z$ représente un hydrogène, $R_a$ représente un hydrogène, un fluor, un chlore, un brome, un méthyle ou un 2-tétrahydropyranyle et $R_b$ et $R_c$, indépendamment l'un de l'autre représentent un hydrogène, un halogène ou un méthyle et les substituants restants sont définis comme à la revendication 2.

**5.** Moyen selon la revendication 1, caractérisé en ce que, comme substance active, il comporte un composé de formule I, où X représente le groupe -CH($OR_1$)-où $R_1$ représente un hydrogène, $R_4$-C(O)- ou -Si($R_5$)($R_6$)($R_7$), où $R_4$ représente un alkyle en $C_1$-$C_{10}$, un halo-alkyle en $C_1$-$C_{10}$, ou un reste de la série du phényle et du benzyle non substitué ou substitué par un halogène, un alkyle en $C_1$-$C_3$, un halo-alkyle en $C_1$-$C_3$, un alcoxy en $C_1$-$C_3$, un halo-alcoxy en $C_1$-$C_3$, un cyano et/ou un nitro; $R_5$, $R_6$, $R_7$, indépendamment les uns des autres, représentent un alkyle en $C_1$-$C_4$, un benzyle ou un phényle; $R_2$ représente un méthyle, un éthyle, un isopropyle ou un butyle secondaire; et $R_3$ représente un hydrogène, un alkyle en $C_1$-$C_4$, un reste de la série du phényle et du benzyle, non substitué ou substitué de une à trois fois par un halogène, un alkyle en $C_1$-$C_2$, un alcoxy en $C_1$-$C_2$, un alkylthio en $C_1$-$C_2$, un halo-alkyle en $C_1$-$C_2$, un nitro ou un cyano, ou représente le groupe U

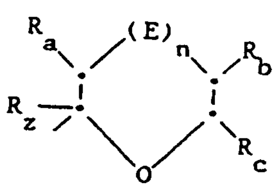

(U)

où n représente un des nombres 0 ou 1, E représente un oxygène ou $CH_2$ et $R_z$ représente un hydrogène, $R_a$ représente un hydrogène, un fluor, un chlore, un brome, un méthyle ou un 2-tétrahydropyranyle et $R_b$ et $R_c$, indépendamment l'un de l'autre représentent un hydrogène, un halogène ou un méthyle et les substituants restants étant définis comme à la revendication 1.

**6.** Moyen selon la revendication 3, caractérisé en ce que, comme substance active, il comporte un composé de formule I: où

$R_3$ représente un hydrogène, un alkyle en $C_1$-$C_6$, un alcényle en $C_2$-$C_6$, un reste de la série du phényle et du benzyle, non substitué ou substitué une ou plusieurs fois par un halogène, un alkyle en $C_1$-$C_3$, un alcoxy en $C_1$-$C_3$, un alkylthio en $C_1$-$C_3$, un halo-alkyle en $C_1$-$C_3$, un nitro ou un cyano, ou représente le groupe U pour un des groupes

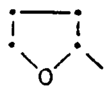 , 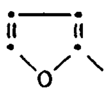 , 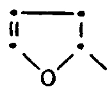 , 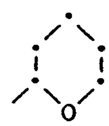 , 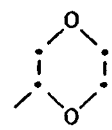 ,

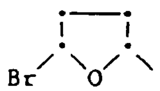 , 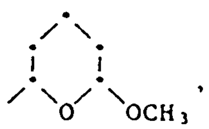 , 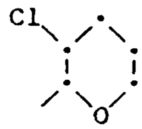 , 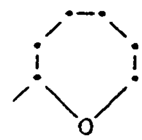 ou 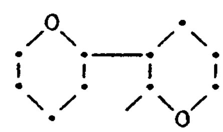

et les substituants restants sont définis comme à la revendication 3.

**7.** Moyen selon la revendication 6, caractérisé en ce que, comme substance active, il comporte un composé de formule I, où $R_1$ représente un hydrogène et $R_3$ représente un hydrogène, un alkyle en $C_1$-$C_4$, un phényle, un benzyle ou un des groupes

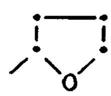 , 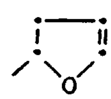 , 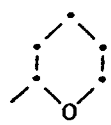 ou 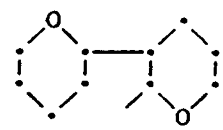

et les substituants restants sont définis comme à la revendication 6.

**8.** Moyen selon la revendication 1, caractérisé en ce que, comme substance active, il comporte un composé de formule I où $R_2$ représente un méthyle, et les substituants restants sont définis comme dans l'une des revendications 1 à 7.

**9.** Moyen selon la revendication 1, caractérisé en ce que, comme substance active, il comporte un composé de formule I où $R_2$ représente un éthyle et les substituants restants sont définis comme dans l'une des revendications 1 à 7.

**10.** Moyen selon la revendication 1, caractérisé en ce que, comme substance active, il comporte un composé de formule I choisi dans la série suivante :

5-acétoxy-29-méthoximino -milbémycine D,
5-(diméthyl-tert.-butyl-silyloxy)-29-hydroximino-milbémycine $A_4$,
29-hydroximino-milbémycine $A_4$,
29-méthoximino-milbémycine $A_4$,
29-phénoximino-milbémycine $A_4$,
29- tétrahydropyranne-2-yl -oximino-milbémycine $A_4$,
5,29-bis-hydroximino-milbémycine $A_4$.

**11.** Procédé pour préparer un composé de formule I,

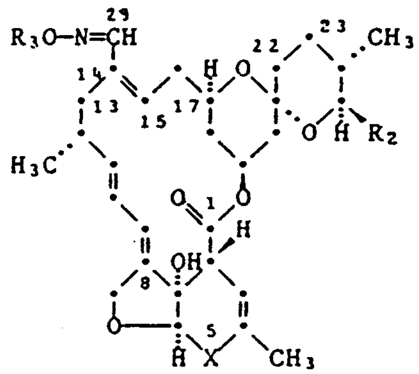

(I)

dans laquelle

X représente un des groupes $-CH(OR_1)-$, $-C(O)-$ ou $C(=N-OR)-$;

$R_1$ représente un hydrogène ou un groupe protégeant OH;

R représente un hydrogène, un groupe protégeant OH, un groupe alkyle, cyclo-alkyle ou acyle; où par groupe protecteur de OH on entend, pour les substituants R et $R_1$, les restes $R_4-C(O)-$ ou $-Si(R_5)(R_6)(R_7)$, où $R_4$ représente un alkyle en $C_1$-$C_{10}$, un halo-alkyle en $C_1$-$C_{10}$, ou un groupe de la série du phényle et du benzyle, non substitué ou substitué par un halogène, un alkyle en $C_1$-$C_3$, un halo-alkyle en $C_1$-$C_3$, un alcoxy en $C_1$-$C_3$, un halo-alcoxy en $C_1$-$C_3$, un cyano et/ou un nitro; $R_5$, $R_6$, $R_7$, indépendamment les uns des autres, représentent un alkyle en $C_1$-$C_4$, un benzyle ou un phényle;

$R_2$ représente un méthyle, un éthyle, un isopropyle, un butyle secondaire ou le groupe

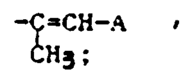

où A représente un méthyle, un éthyle ou un isopropyle, et

$R_3$ représente un hydrogène, un alkyle en $C_1$-$C_6$, un alcényle en $C_2$-$C_6$, un reste de la série du phényle et du benzyle, non substitué ou substitué une fois ou plusieurs fois par un halogène, un alkyle en $C_1$-$C_3$, un alcoxy en $C_1$-$C_3$, un alkylthio en $C_1$-$C_3$, un halo-alkyle en $C_1$-$C_3$, un nitro ou un cyano, ou représente le groupe U

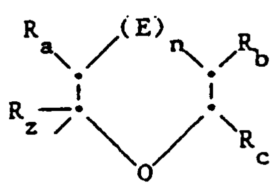

(U)

où n représente un des nombres 0, 1, 2, 3, 4 ou 5, E représente un oxygène ou $-CH(R_d)-$ et $R_z$ représente un hydrogène, $R_a$ représente un hydrogène, un halogène, un alkyle en $C_1$-$C_3$, un alcoxy en $C_1$-$C_3$ ou un 2-tétrahydropyranyle et $R_b$, $R_c$ et $R_d$, indépendamment les uns des autres, représentent un hydrogène, un halogène, un alkyle en $C_1$-$C_3$ ou un alcoxy en $C_1$-$C_3$, où le groupe U peut aussi se présenter sous une forme insaturée,

caractérisé en ce que l'on fait réagir une 29-oxo-milbémycine de formule II

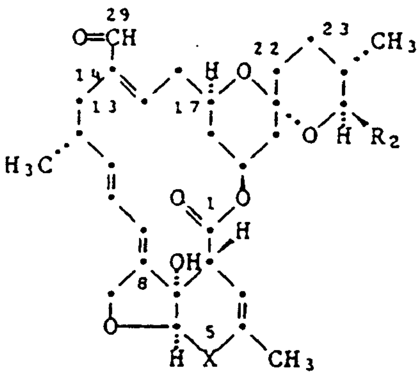

(II)

avec une oxamine primaire de formule III

$H_2N-OR_3$ (III)

ou un de ses sels, et on fait réagir ensuite, si on le désire, le composé résultant de formule I, si $R_3$ représente un hydrogène, avec un halogénure de formule IV

$R_3'$ -Hal (IV)

où $R_2$ représente un méthyle, un éthyle, un isopropyle ou un butyle secondaire, ou le groupe

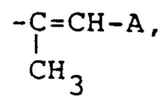

où A représente un méthyle, un éthyle ou un isopropyle et le substituant $R_3$ a les significations données pour la formule I, X représente $-C(OR_1)$, où $R_1$ représente un hydrogène ou un des groupes protecteurs de OH indiqués pour la formule I , et Hal représente un halogène, et $R'_3$ a toutes les significations de $R_3$, à l'exception de l'hydrogène, de telle sorte que l'on part en général de composés de formule II qui soit comportent un groupe 5 hydroxy libre et, si on le désire, on acyle ou silyle ensuite les produits résultants sur l'atome 5-O, ou soit on part d'un composé de formule II déjà silylé ou acylé dans la position 5, et l'on sépare, si on le désire, le groupe protecteur; ou, si le but de la préparation est d'obtenir des composés de formule I, où X représente le groupe -C(O)-, on part de composés de formule II qui comportent un groupe 5-hydroxy libre, et on traite ceux-ci avec un réactif approprié pour une oxydation; ou, si l'on désire des composés de formule I, où X représente le groupe -C( = N-OR)-, où R a la signification donnée pour la formule I, on part d'un composé de formule I, dans lequel X représente -C(O)-, et on fait réagir celui-ci avec de l'hydroxylamine ou un de ses sels, et ensuite, si on le désire, on introduit le substituant R, ou on effectue la réaction avec un composé de formule $NH_2$-OR, ou un de ses sels.

**12.** Procédé selon la revendication 11 pour préparer un composé de formule I

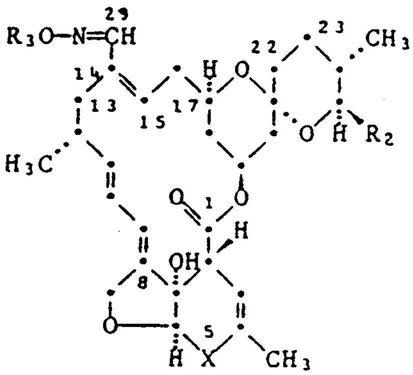

(I)

dans laquelle

X représente un des groupes -CH($OR_1$)-, -C(O)- ou -C(= N-OR)-;

$R_1$ représente un hydrogène ou un groupe protégeant OH;

R représente un hydrogène, un groupe protecteur de OH, un groupe alkyle, cyclo-alkyle; où par groupe protecteur de OH on entend, pour les substituants R et $R_1$, les restes $R_4$-C(O)- ou -Si-($R_5$)($R_6$)($R_7$), où $R_4$ représente un alkyle en $C_1$-$C_{10}$, un halo-alkyle en $C_1$-$C_{10}$, ou un groupe de la série du phényle et du benzyle, non substitué ou substitué par un halogène, un alkyle en $C_1$-$C_3$, un halo-alkyle en $C_1$-$C_3$, un alcoxy en $C_1$-$C_3$, un halo-alcoxy en $C_1$-$C_3$, un cyano et/ou un nitro; $R_5$, $R_6$, $R_7$, indépendamment les uns des autres, représentent un alkyle en $C_1$-$C_4$, un benzyle ou un phényle;

$R_2$ représente un méthyle, un éthyle, un isopropyle, un butyle secondaire ; et

$R_3$ représente un hydrogène, un alkyle en $C_1$-$C_6$, un alcényle en $C_2$-$C_6$, un reste de la série du phényle et du benzyle, non substitué ou substitué une fois ou plusieurs fois par un halogène, un alkyle en $C_1$-$C_3$, un alcoxy en $C_1$-$C_3$, un alkylthio en $C_1$-$C_3$, un halo-alkyle en $C_1$-$C_3$, un nitro ou un cyano, ou représente le groupe U

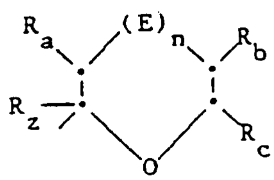

(U)

où n représente un des nombres 0, 1, 2, 3, 4 ou 5, E représente un oxygène ou -CH($R_d$)- et $R_z$ représente un hydrogène, $R_a$ représente un hydrogène, un halogène, un alkyle en $C_1$-$C_3$, un alcoxy en $C_1$-$C_3$ ou un 2-tétrahydropyranyle et $R_b$, $R_c$ et $R_d$, indépendamment les uns des autres, représentent un hydrogène, un halogène, un alkyle en $C_1$-$C_3$ ou un alcoxy en $C_1$-$C_3$, où le groupe U peut aussi se présenter sous une forme insaturée,

caractérisé en ce que l'on fait réagir une 29-oxo-milbémycine de formule II

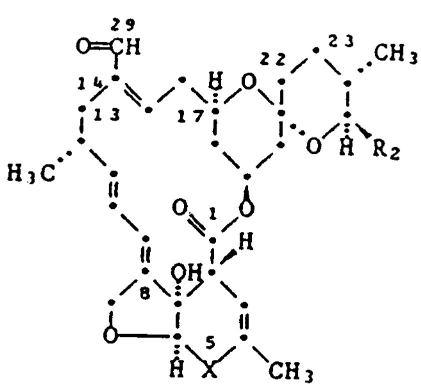

(II)

avec une oxamine primaire de formule III

$H_2N\text{-}OR_3$ (III)

ou un de ses sels, et on fait réagir ensuite, si on le désire, le composé résultant de formule I, si $R_3$ représente un hydrogène, avec un halogénure de formule IV

$R_3'$ -Hal (IV)

où $R_2$ représente un méthyle, un éthyle, un isopropyle ou un butyle secondaire, et le substituant $R_3$ a les significations données pour la formule I, X représente -C(OR$_1$), où $R_1$ représente un hydrogène ou un des groupes protecteurs de OH indiqués pour la formule I, et Hal représente un halogène, et $R'_3$ a toutes les significations de $R_3$, à l'exception de l'hydrogène, de telle sorte que l'on part en général de composés de formule II qui soit comportent un groupe 5 hydroxy libre et, si on le désire, on acyle ou silyle ensuite les produits résultants sur l'atome 5-O, ou soit on part d'un composé de formule II déjà silylé ou acylé dans la position 5, et l'on sépare, si on le désire, le groupe protecteur; ou, si le but de la préparation est d'obtenir des composés de formule I, où X représente le groupe -C(O)-, on part de composés de formule II qui comportent un groupe 5-hydroxy libre, et on traite ceux-ci avec un réactif approprié pour une oxydation; ou, si l'on désire des composés de formule I, où X représente le groupe -C(=N-OR)-, où R a la signification donnée pour la formule I, on part d'un composé de formule I, dans lequel X représente -C(O)-, et on fait réagir celui-ci avec de l'hydroxylamine ou un de ses sels, et ensuite, si on le désire, on introduit le substituant R, ou on effectue la réaction avec un composé de formule $NH_2\text{-}OR$, ou un de ses sels.

**13.** Procédé pour préparer un composé de formule I selon la revendication 1,

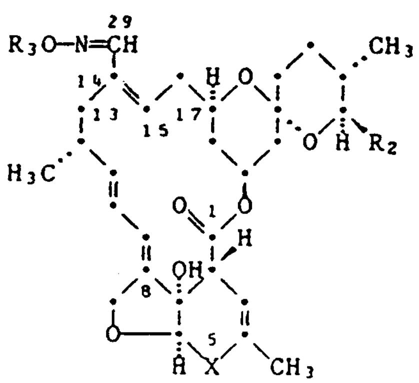

(I)

dans laquelle

- X représente le groupe -CH(OR$_1$)-;
- $R_1$ représente un hydrogène ou un groupe protégeant OH; où par groupe protecteur de OH on entend les restes $R_4$-C(O)- ou -Si($R_5$)($R_6$)($R_7$), où $R_4$ représente un alkyle en $C_1$-$C_{10}$, un halo-alkyle en $C_1$-$C_{10}$, ou un groupe de la série du phényle et du benzyle, non substitué ou substitué par un halogène, un alkyle en $C_1$-$C_3$, un halo-alkyle en $C_1$-$C_3$, un alcoxy en $C_1$-$C_3$, un halo-alcoxy en $C_1$-$C_3$, un cyano et/ou un nitro; $R_5$, $R_6$, $R_7$, indépendamment les uns des autres, représentent un alkyle en $C_1$-$C_4$, un benzyle ou un phényle;
- $R_2$ représente un méthyle, un éthyle, un isopropyle, un butyle secondaire ; et
- $R_3$ représente un hydrogène, un alkyle en $C_1$-$C_6$, un alcényle en $C_2$-$C_6$, un reste de la série du phényle et du benzyle, non substitué ou substitué une fois ou plusieurs fois par un halogène, un alkyle en $C_1$-$C_3$, un alcoxy en $C_1$-$C_3$, un alkylthio en $C_1$-$C_3$, un halo-alkyle en $C_1$-$C_3$, un nitro ou un cyano, ou représente le groupe U

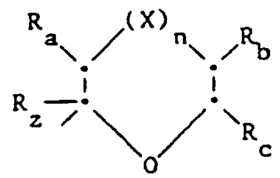

(U)

où n représente un des nombres 0, 1, 2, 3, 4 ou 5, X représente un oxygène ou $-CH(R_d)-$ et $R_z$ représente un hydrogène, $R_a$ représente un hydrogène, un halogène, un alkyle en $C_1$-$C_3$, un alcoxy en $C_1$-$C_3$ ou un 2-tétrahydropyranyle et $R_b$, $R_c$ et $R_d$, indépendamment les uns des autres, représentent un hydrogène, un halogène, un alkyle en $C_1$-$C_3$ ou un alcoxy en $C_1$-$C_3$, où le groupe U peut aussi se présenter sous une forme insaturée,

caractérisé en ce quel'on fait réagir une 29-oxo-milbémycine de formule II

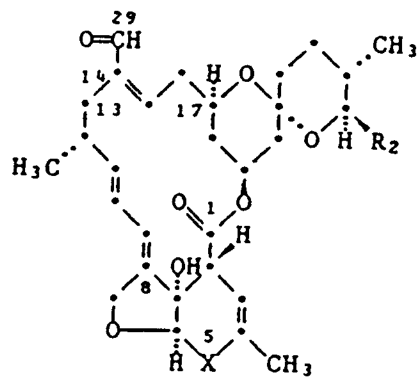

(II)

avec une oxamine primaire de formule III

$H_2N$-$OR_3$ (III)

ou un de ses sels, et on fait réagir ensuite, si on le désire, le composé résultant de formule I, si $R_3$ représente un hydrogène, avec un halogénure de formule IV

$R_3'$ -Hal (IV)

où $R_2$ représente un méthyle, un éthyle, un isopropyle ou un butyle secondaire et les substituants $R_1$ et $R_3$ ont les significations données pour la formule I, Hal représente un halogène, et $R'_3$ a toutes les significations de $R_3$, à l'exception de l'hydrogène, de telle sorte que l'on part de composés de formule II qui soit comportent un groupe 5 hydroxy libre et, si on le désire, on acyle ou silyle ensuite les produits résultants sur l'atome 5-O, ou soit on part d'un composé de formule II déjà silylé ou acylé dans la position 5, et l'on sépare, si on le désire, le groupe protecteur .

**14.** Procédé pour lutter contre des parasites des plantes ou contre des insectes nuisibles , caractérisé en ce que l'on applique sur les parasites, les insectes nuisibles ou leurs biotopes, une quantité parasiticide ou insecticide efficace d'un composé de formule I

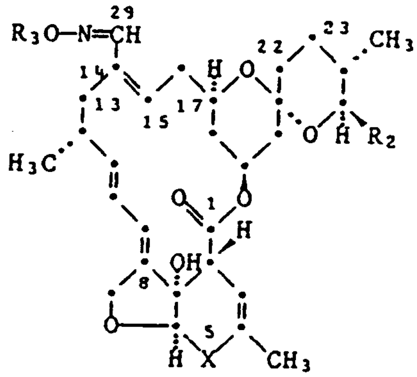

(I)

dans laquelle

- X représente un des groupes $-CH(OR_1)-$, -C(O)- ou -C( = N-OR)-;
- $R_1$ représente un hydrogène ou un groupe protégeant OH;
- R représente un hydrogène, un groupe protecteur de OH, un groupe alkyle, cyclo-alkyle; où par groupe protecteur de OH on entend, pour les substituants R et $R_1$, les restes $R_4$-C(O)- ou -Si-$(R_5)(R_6)(R_7)$, où $R_4$ représente un alkyle en $C_1$-$C_{10}$, un halo-alkyle en $C_1$-$C_{10}$, ou un groupe de la série du phényle et du benzyle, non substitué ou substitué par un halogène, un alkyle en $C_1$-$C_3$, un halo-alkyle en $C_1$-$C_3$, un alcoxy en $C_1$-$C_3$, un halo-alcoxy en $C_1$-$C_3$, un cyano et/ou un nitro; $R_5$, $R_6$, $R_7$, indépendamment les uns des autres, représentent un alkyle en $C_1$-$C_4$, un benzyle ou un phényle;
- $R_2$ représente un méthyle, un éthyle, un isopropyle, un butyle secondaire ou le groupe

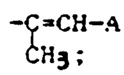 ,

où A représente un méthyle, un éthyle ou un isopropyle, et

- $R_3$ représente un hydrogène, un alkyle en $C_1$-$C_6$, un alcényle en $C_2$-$C_6$, un reste de la série du phényle et du benzyle, non substitué ou substitué une fois ou plusieurs fois par un halogène, un alkyle en $C_1$-$C_3$, un alcoxy en $C_1$-$C_3$, un alkylthio en $C_1$-$C_3$, un halo-alkyle en $C_1$-$C_3$, un nitro ou un cyano, ou représente le groupe U

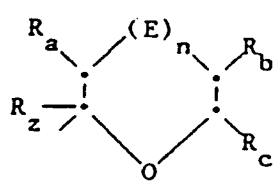

(U)

où n représente un des nombres 0, 1, 2, 3, 4 ou 5, E représente un oxygène ou $-CH(R_d)-$ et $R_z$ représente un hydrogène, $R_a$ représente un hydrogène, un halogène, un alkyle en $C_1$-$C_3$, un alcoxy en $C_1$-$C_3$ ou un 2-tétrahydropyranyle et $R_b$, $R_c$ et $R_d$, indépendamment les uns des autres, représentent un hydrogène, un halogène, un alkyle en $C_1$-$C_3$ ou un alcoxy en $C_1$-$C_3$, où le groupe U peut aussi se présenter sous une forme insaturée.

**15.** Procédé pour lutter contre des parasites des plantes ou contre des insectes nuisibles, selon la revendication 14, caractérisé en ce que l'on applique sur les parasites, les insectes nuisibles ou leurs biotopes, une quantité parasiticide ou insecticide efficace d'un composé de formule I

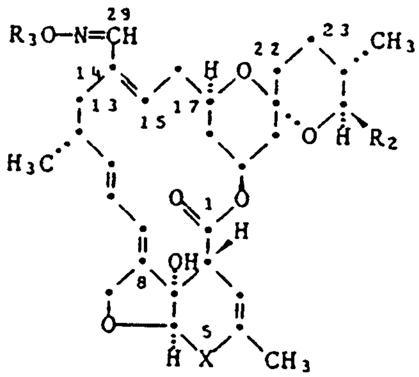

(I)

dans laquelle

X représente un des groupes $-CH(OR_1)-$, -C(O)- ou -C( = N-OR)-;

$R_1$ représente un hydrogène ou un groupe protégeant OH;

R représente un hydrogène, un groupe protecteur de OH, un groupe alkyle, cyclo-alkyle; où par groupe protecteur de OH on entend, pour les substituants R et $R_1$, les restes $R_4$-C(O)- ou $-Si-(R_5)(R_6)(R_7)$, où $R_4$ représente un alkyle en $C_1$-$C_{10}$, un halo-alkyle en $C_1$-$C_{10}$, ou un groupe de la série du phényle et du benzyle, non substitué ou substitué par un halogène, un alkyle en $C_1$-$C_3$, un halo-alkyle en $C_1$-$C_3$, un alcoxy en $C_1$-$C_3$, un halo-alcoxy en $C_1$-$C_3$, un cyano et/ou un nitro; $R_5$, $R_6$, $R_7$, indépendamment les uns des autres, représentent un alkyle en $C_1$-$C_4$, un benzyle ou un phényle;

$R_2$ représente un méthyle, un éthyle, un isopropyle, un butyle secondaire ; et

$R_3$ représente un hydrogène, un alkyle en $C_1$-$C_6$, un alcényle en $C_2$-$C_6$, un reste de la série du phényle et du benzyle, non substitué ou substitué une fois ou plusieurs fois par un halogène, un alkyle en $C_1$-$C_3$, un alcoxy en $C_1$-$C_3$ , un alkylthio en $C_1$-$C_3$, un halo-alkyle en $C_1$-$C_3$, un nitro ou un cyano, ou représente le groupe U

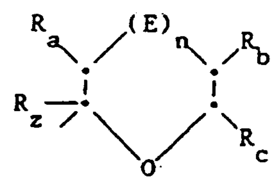

(U)

où n représente un des nombres 0, 1, 2, 3, 4 ou 5, E représente un oxygène ou $-CH(R_d)-$ et $R_z$ représente un hydrogène, $R_a$ représente un hydrogène, un halogène, un alkyle en $C_1$-$C_3$, un alcoxy en $C_1$-$C_3$ ou un 2-tétrahydropyranyle et $R_b$, $R_c$ et $R_d$, indépendamment les uns des autres, représentent un hydrogène, un halogène, un alkyle en $C_1$-$C_3$ ou un alcoxy en $C_1$-$C_3$, où le groupe U peut aussi se présenter sous une forme insaturée.

**16.** Procédé pour lutter contre des parasites des plantes ou contre des insectes nuisibles, selon la revendication 14, caractérisé en ce que l'on applique sur les parasites, les insectes nuisibles ou leurs biotopes, une quantité parasiticide ou insecticide efficace d'un composé de formule I

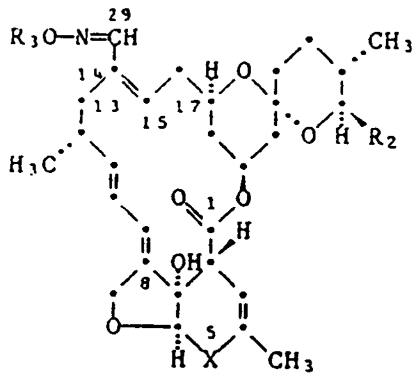

(I)

dans laquelle

X représente le groupe -CH($OR_1$)-;

$R_1$ représente un hydrogène ou un groupe protecteur de OH; où par groupe protecteur de OH on entend les restes $R_4$-C(O)- ou -Si($R_5$)($R_6$)($R_7$), où $R_4$ représente un alkyle en $C_1$-$C_{10}$, un halo-alkyle en $C_1$-$C_{10}$, ou un groupe de la série du phényle et du benzyle, non substitué ou substitué par un halogène, un alkyle en $C_1$-$C_3$, un halo-alkyle en $C_1$-$C_3$, un alcoxy en $C_1$-$C_3$, un halo-alcoxy en $C_1$-$C_3$, un cyano et/ou un nitro; $R_5$, $R_6$, $R_7$, indépendamment les uns des autres, représentent un alkyle en $C_1$-$C_4$, un benzyle ou un phényle;

$R_2$ représente un méthyle, un éthyle, un isopropyle, un butyle secondaire ; et

$R_3$ représente un hydrogène un alkyle en $C_1$-$C_6$, un alcényle en $C_2$-$C_6$, un reste de la série du phényle et du benzyle, non substitué ou substitué une fois ou plusieurs fois par un halogène, un alkyle en $C_1$-$C_3$, un alcoxy en $C_1$-$C_3$, un alkylthio en $C_1$-$C_3$, un halo-alkyle en $C_1$-$C_3$, un nitro ou un cyano, ou représente le groupe U

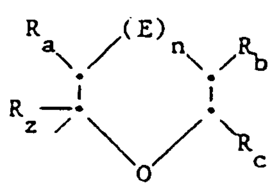

(U)

où n représente un des nombres 0, 1, 2, 3, 4 ou 5, E représente un oxygène ou -CH($R_d$)- et $R_z$ représente un hydrogène, $R_a$ représente un hydrogène, un halogène, un alkyle en $C_1$-$C_3$, un alcoxy en $C_1$-$C_3$ ou un 2-tétrahydropyranyle et $R_b$, $R_c$ et $R_d$, indépendamment les uns des autres, représentent un hydrogène, un halogène, un alkyle en $C_1$-$C_3$ ou un alcoxy en $C_1$-$C_3$, où le groupe U peut aussi se présenter sous une forme insaturée.

**17.** Procédé selon la revendication 14, caractérisé en ce que, comme parasites, il s'agit de nématodes.

**18.** Utilisation d'un composé de formule I selon la revendication 1, pour préparer un moyen de lutte contre des ecto et endoparasites sur les animaux utiles, ou contre les insectes nuisibles à tous les stades de leur développement.